(19) Europäisches Patentamt
European Patent Office
Office européen des brevets

(11) EP 3 617 186 A1

(12) EUROPEAN PATENT APPLICATION
published in accordance with Art. 153(4) EPC

(43) Date of publication:
**04.03.2020 Bulletin 2020/10**

(21) Application number: **18790399.2**

(22) Date of filing: **19.04.2018**

(51) Int Cl.:
***C07C 233/11*** *(2006.01)* ***C07C 311/16*** *(2006.01)*
***A61K 31/18*** *(2006.01)* ***A61K 31/166*** *(2006.01)*
***A61P 29/00*** *(2006.01)*

(86) International application number:
**PCT/CN2018/083792**

(87) International publication number:
**WO 2018/196677 (01.11.2018 Gazette 2018/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.04.2017 CN 201710294651**

(71) Applicant: **Sichuan Kelun-Biotech Biopharmaceutical Co., Ltd.**
**Chengdu, Sichuan 611138 (CN)**

(72) Inventors:
- **ZHU, Jiawang**
  **Chengdu**
  **Sichuan 611138 (CN)**
- **SONG, Zhiquan**
  **Chengdu**
  **Sichuan 611138 (CN)**
- **YANG, Long**
  **Chengdu**
  **Sichuan 611138 (CN)**
- **LI, Rui**
  **Chengdu**
  **Sichuan 611138 (CN)**
- **ZHANG, Shuai**
  **Chengdu**
  **Sichuan 611138 (CN)**
- **ZHOU, Lin**
  **Chengdu**
  **Sichuan 611138 (CN)**
- **ZHAO, Mingliang**
  **Chengdu**
  **Sichuan 611138 (CN)**
- **TANG, Zujian**
  **Chengdu**
  **Sichuan 611138 (CN)**
- **ZHONG, Wei**
  **Chengdu**
  **Sichuan 611138 (CN)**
- **ZENG, Hong**
  **Chengdu**
  **Sichuan 611138 (CN)**
- **SONG, Hongmei**
  **Chengdu**
  **Sichuan 611138 (CN)**
- **ZHOU, Xin**
  **Chengdu**
  **Sichuan 611138 (CN)**
- **TAN, Yuting**
  **Chengdu**
  **Sichuan 611138 (CN)**
- **HU, Xiao**
  **Chengdu**
  **Sichuan 611138 (CN)**
- **WANG, Lichun**
  **Chengdu**
  **Sichuan 611138 (CN)**
- **WANG, Jingyi**
  **Chengdu**
  **Sichuan 611138 (CN)**

(74) Representative: **Mewburn Ellis LLP**
**City Tower**
**40 Basinghall Street**
**London EC2V 5DE (GB)**

# (54) FLUOROALLYLAMINE DERIVATIVE AND USE THEREOF

(57) The present invention relates to a fluoroallylamine derivative and use thereof. In particular, the present invention relates to a compound as shown in Formula I, a prodrug, an isomer, an isotope-labeled compound, a solvate or a pharmaceutically acceptable salt thereof, which has VAP-1/SSAO inhibitory activity, and can be used for treating a disease associated with VAP-1/SSAO overactivity.

EP 3 617 186 A1

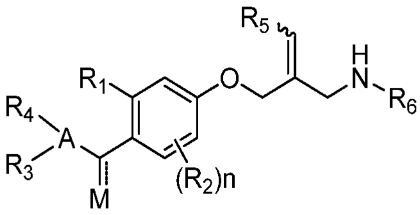

Formula I

**Description**

Technical field

The invention relates to the medical field, particularly, relates to a fluroallylamine derivative and use thereof as an inhibitor of vascular adhesion protein-1 (VAP-1)/semicarbazide-sensitive amine oxidase (SSAO).

Background art

Vascular adhesion protein-1 (VAP-1) is a glycoprotein dimmer having a relative molecular weight (Mw) ranging from 170,000 to 180,000, and a multifunctional protein that is widely present in mammal animals. The vascular adhesion protein-1 is an endothelial adhesion molecule and also has an activity of semicarbazide-sensitive amine oxidase (SSAO). The protein is widely distributed in tissues, primarily derived from endothelia cells, plain muscle cells and adipose cells, and it is present in vivo in the following two most common forms: a tissue bonding form and a soluble form. And the form that circulates in human and animal blood plasma is the soluble form, which is a cleavage product of the membrane-bound vascular adhesion protein-1/semicarbazide-sensitive amine oxidase.

It is found that vascular adhesion protein-1/semicarbazide-sensitive amine oxidase is induced to express during the formation of fats (Fontana, E., et. al., Biochem. J., 2001, 356, 769; Moldes, M., et. al., J. Biol. Chem., 1999, 274, 9515); vascular adhesion protein-1/semicarbazide-sensitive amine oxidase also play an important role in leukocyte exudation, adhesion cascade reactions, glycometabolism regulation, vascular injuries, etc.; in inflammatory diseases, tissue injury fibrosis, glycometabolism-related diseases, tumors, strokes and many other clinical diseases, both the expressions and the functions of the vascular adhesion protein-1/semicarbamide-sensitive amine oxidase play important roles; in skins of patients suffering from atopic eczema or psoriasis, positive cells having a high level of vascular adhesion protein-1/semicarbazide-sensitive amine oxidase are found. Generally, vascular adhesion protein-1/semicarbazide-sensitive amine oxidase is an effective biological target for diabetes, pneumonia, hepatitis, pulmonary fibrosis, liver fibrosis, fatty liver, stroke and other inflammatory or fibrosis-related diseases.

Some small organic molecules are found to have inhibitory activity against vascular adhesion protein-1/semicarbazide-sensitive amine oxidase, for example, hydrazines, phenyl-substituted methylhydrazines, hydrazinol, hydrazine-substituted dihydroindenes, aromatic cycloalkylamine, allylamine, propynylamine, oxazolidone, haloalkylamines, 1,3,4-oxazolidone, oxadiazine, sulfamide, thiazole derivatives, and other organic compounds (Dunkel, P. et. al., Curr. Med. Chem., 2008, 15, 1827; Tetra. Letters, 1977, 36, 3155). Also, it was reported that compounds of the propynylamine family can be used to inhibit vascular adhesion protein-1/semicarbazide-sensitive amine oxidase (O'Connell, K. M., et. al., Biochemistry, 2004, 43, 10965). These compounds, for example semicarbazides, exhibit the inhibiting function depending on the hydrazine functional group to form a covalent imine bond with TPQ cofactors.

Summary of the invention

Through intensive research, the inventor unexpectedly developed a compound having effective inhibitory activity against vascular adhesion protein-1/semicarbazide-sensitive amine oxidase, which can be used in preventing or treating a disease associated with the overactivity of vascular adhesion protein-1/semicarbazide-sensitive amine oxidase. In particular, the invention provides a fluoroallylamine derivative represented by Formula I, which shows outstanding inhibitory activity against vascular adhesion protein-1/semicarbazide-sensitive amine oxidase, good selectivity over monoamine oxidase and diamine oxidase, and improved metabolic stability in vivo.

A first aspect of the invention provides a compound of Formula I, or a pharmaceutically acceptable salt, an ester, a solvate, an isomer, or an isotope-labeled compound thereof; any crystal form or racemate thereof; a metabolite thereof; or a mixture thereof:

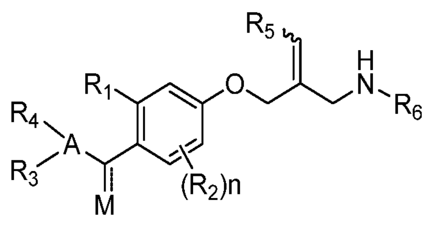

Formula I

Wherein:

$R_1$ is selected from the group consisting of hydrogen, halogen, $C_{1-6}$ alkyl, 3-10-membered cycloalkyl-$CH_2NHC(O)$-, 3-8-membred aliphatic heterocyclyl-$CH_2NHC(O)$-, 6-20-membered aryl-$CH_2NHC(O)$-, 5-20-membered heteroaryl-$CH_2NHC(O)$-, 5-20-membered fused heteroaryl-$CH_2NHC(O)$-, benzo-fused 3-10-membered cycloalkyl-NHC(O)-, 3-8-membered aliphatic heterocyclyl-NHC(O)-, 6-20-membered aryl-NHC(O)-, 5-20-membered heteroaryl-NHC(O)-, and 5-20-membered fused heteroaryl-NHC(O)-; and wherein the $C_{1-6}$ alkyl, 3-10-membered cycloalkyl-$CH_2NHC(O)$-, 3-8-membered aliphatic heterocyclyl-$CH_2NHC(O)$-, 6-20-membered aryl-$CH_2NHC(O)$-, 5-20-membered heteroaryl-$CH_2NHC(O)$-, 5-20-membered fused heteroaryl-$CH_2NHC(O)$-, benzo-fused 3-10-membered cycloalkyl-NHC(O)-, 3-8-membered aliphatic heterocyclyl-NHC(O)-, 6-20-membered aryl-NHC(O)-, 5-20-membered heteroaryl-NHC(O)-, and 5-20-membered fused heteroaryl-NHC(O)- are unsubstituted or substituted independently by one or more (for example 1, 2, 3 or 4) substituents selected from the group consisting of halogen, hydroxyl, -NRR', $C_{1-3}$ alkyl, $C_{1-3}$ alkoxyl, and cyano;

each of $R_2$ is independently selected from the group consisting of hydrogen, cyano, nitro, hydroxyl, halogen, $C_{1-6}$ alkyl, 3-10-membered cycloalkyl, 3-8-membered aliphatic heterocyclyl, halogenated $C_{1-6}$ alkyl, $C_{1-6}$ alkoxyl, and halogenated $C_{1-6}$ alkoxyl;

n=1, 2 or 3;

$R_3$ and $R_4$ are such that

(a) $R_3$ is selected from the group consisting of 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl phenyl, 3-8-membered aliphatic heterocyclyl, 6-20-membered aryl, 5-20-membered heteroaryl, and 5-20-membered fused heteroaryl; and wherein the 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl phenyl, 3-8-membered aliphatic heterocyclyl, 6-20-membered aryl, 5-20-membered heteroaryl, and 5-20-membered fused heteroaryl are unsubstituted or substituted independently by one or more (for example 1, 2, 3 or 4) substituents selected from the group consisting of halogen, hydroxyl, -NRR', $C_{1-3}$ alkyl, $C_{1-3}$ alkoxyl, cyano, 3-10-membered cycloalkyl, 3-8-membered aliphatic heterocyclyl, 6-20-membered aryl, 5-20-membered heteroaryl, 5-20-membered fused heteroaryl, halogenated $C_{1-3}$ alkyl, halogenated $C_{1-3}$ alkoxyl, $C_{1-3}$ alkoxy-$C_{1-3}$ alkyl, $C_{1-3}$ alkoxy-$C_{1-3}$ alkoxyl, and C1-3 alkoxy-$C_{1-3}$ alkoxy-$C_{1-3}$ alkyl; $R_4$ is selected from hydrogen, $C_{1-6}$ alkyl, and halogenated $C_{1-6}$ alkyl;

(b) $R_3$ and $R_1$ are linked together to form a ring X, wherein the ring X is a 5-8-membered aliphatic ring or a 5-8-membered aliphatic heterocycle; and wherein the 5-8-membered aliphatic ring or 5-8-membered aliphatic heterocycle is unsubstituted or each substituted independently by one or more (for example 1, 2, 3 or 4) substituents selected from the group consisting of O=, halogen, cyano, -NRR', nitro, hydroxyl, $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl; or the aforementioned 5-8-membered aliphatic ring or 5-8-membered aliphatic heterocycle is fused with a 3-8-membred aliphatic ring to form a spiro structure; $R_4$ is selected from the group consisting of hydrogen, $C_{1-6}$ alkyl, 3-8-membered cycloalkyl, 3-8-membered aliphatic heterocyclyl, 6-10-membered aryl, 5-10-membered heteroaryl, and 5-10-membered fused heteroaryl; and wherein the $C_{1-6}$ alkyl, 3-8-membered cycloalkyl, 3-8-membered aliphatic heterocyclyl, 6-10-membered aryl, 5-10-membered heteroaryl or 5-10-membered fused heteroaryl is optionally substituted by one or more (for example 1, 2, 3 or 4) substituents selected from the group consisting of halogen, -NRR', cyano, hydroxyl, $C_{1-4}$ alkyl, $C_{1-4}$ alkoxyl, 3-8-membered cycloalkyl, 3-8-membered aliphatic heterocyclyl, 6-10-membered aryl, 5-10-membered heteroaryl, and 5-10-membered fused heteroaryl; wherein the $C_{1-4}$ alkyl, 3-8-membered cycloalkyl, 3-8-membered aliphatic heterocyclyl, 6-10-membered aryl, 5-10-membered heteroaryl or 5-10-membered fused heteroaryl is optionally substituted by one or more (for example, 1, 2, 3 or 4) substituents selected from halogen, -NRR', $C_{1-3}$ alkyl, and $C_{1-3}$ alkoxyl;

(c) $R_3$ and $R_4$ are linked together to form a ring Y, wherein the ring Y is a 5-8-membered aliphatic ring or a 5-8-membered aliphatic heterocycle; and wherein the 5-8-membered aliphatic ring or 5-8-membered aliphatic heterocycle is unsubstituted or each substituted independently by one or more (for example 1, 2, 3 or 4) substituents selected from the group consisting of halogen, cyano, -NRR', nitro, hydroxyl, $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl; wherein each of $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, or 5-20-membered fused heteroaryl is unsubstituted or substituted independently by one or more (for example, 1, 2, 3 or 4) substituents selected from the group consisting of halogen, cyano, -NRR', $C_{1-6}$ alkyl, $C_{1-6}$ alkoxyl, 6-20-membered aryl, 5-20-membered heteroaryl, and 5-20-membered fused heteroaryl; or the aforementioned 5-8-membered aliphatic ring or 5-8-membered aliphatic heterocycle is fused with a 6-20-membered aromatic ring or a 5-20-membered aromatic heterocycle to form a fused ring system, wherein the fused ring system is unsubstituted or substituted independently by one or more (for example 1, 2, 3 or 4) substituents selected from the group consisting of halogen, cyano, -NRR', $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl; or

(d) $R_3$ is linked together with M to form a ring Z, wherein the ring Z is a 3-10-membered aliphatic heterocycle, a 6-20-membered aromatic ring or a 5-20-membered aromatic heterocycle; and wherein the 3-10-membered aliphatic heterocycle, 6-20-membered aromatic ring or 5-20-membered aromatic heterocycle is unsubstituted or each substituted independently by one or more (for example 1, 2, 3 or 4) substituents selected from the group consisting of halogen, cyano, -NRR', nitro, hydroxyl, =O, $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroary; or the aforementioned 3-10-membered aliphatic heterocycle, 6-20-membered aromatic ring or 5-20-membered aromatic heterocycle is fused with a 6-20-membered aromatic ring or a 5-20-membered aromatic heterocycle to form a fused ring system, wherein the fused ring system is unsubstituted or each substituted independently by one or more (for example 1, 2, 3 or 4) substituents selected from the group consisting of halogen, cyano, -NRR', $C_{1-6}$ alkyl, halogenated $C_{1-6}$ alkyl, halogenated $C_{1-6}$ alkoxyl, 6-20-membered aryl, 5-20-membered heteroaryl, and 5-20-membered fused heteroaryl; $R_4$ is selected from hydrogen, $C_{1-6}$ alkyl, and $C_{1-6}$ alkoxyl, or $R_4$ is absent or forms a covalent bond;

$R_5$ is halogen;

$R_6$ is selected from hydrogen, $C_{1-6}$ alkyl, and -COOR; wherein $C_{1-6}$ alkyl is unsubstituted or substituted by one or more (for example 1, 2, 3 or 4) substituents selected from halogen, hydroxyl, amino, and cyano;

atom A is selected from C, N, O and S;

M is selected from C, N, O, $H_2$ and =NR;

R and R' are independently selected from the group consisting of hydrogen, halogen, cyano, hydroxyl, $C_{1-3}$ alkyl, $C_{1-3}$ alkoxyl, 3-8-membered cycloalkyl, 3-8-membered aliphatic heterocyclyl, 6-10-membered aryl, 5-10-membered heteroaryl, and 5-10-membered fused heteroaryl;

In some preferred embodiments according to the invention, $R_1$ is not a hydrogen, and

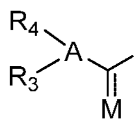

as a whole is independently a hydrogen.

In some preferred embodiments according to the invention, the compound is a mixture of the cis-configuration and the trans-configuration in any ratio.

In some preferred embodiments according to the invention, the compound is in cis (Z)-configuration.

In some preferred embodiments according to the invention, the compound is in trans (E)-configuration.

In some preferred embodiments according to the invention, the compound has a structure as represented by Formula II:

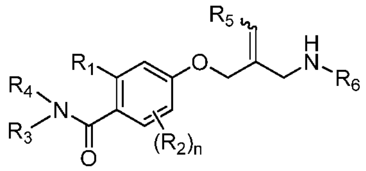

Formula II

wherein, in the compound of Formula II, $R_3$ is selected from the group consisting of 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl-phenyl, 3-8-membered aliphatic heterocyclyl, 6-20-membered aryl, 5-20-membered heteroaryl, and 5-20-membered fused heteroaryl; and wherein the 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl-phenyl, 3-8-membered aliphatic heterocyclyl, 6-20-membered aryl, 5-20-membered heteroaryl, and 5-20-membered fused heteroaryl are unsubstituted or substituted independently by one or more (for example 1, 2, 3 or 4) substituents selected from the group consisting of halogen, hydroxyl, -NRR', $C_{1-3}$ alkyl, $C_{1-3}$ alkoxyl, cyano, 3-10-membered cycloalkyl, 3-8-membered aliphatic heterocyclyl, 6-20-membered aryl, 5-20-membered heteroaryl, 5-20-membered fused heteroaryl, halogenated $C_{1-3}$ alkyl, halogenated $C_{1-3}$ alkoxyl, $C_{1-3}$ alkoxyl-$C_{1-3}$ alkyl, $C_{1-3}$ alkoxyl-$C_{1-3}$ alkoxyl, and $C_{1-3}$ alkoxyl-$C_{1-3}$ alkoxyl-$C_{1-3}$ alkyl; $R_4$ is selected from hydrogen, $C_{1-6}$ alkyl, and halogenated $C_{1-6}$ alkyl; $R_1$, $R_2$, $R_5$, $R_6$, R, R' and n are as defined in Formula I.

In some preferred embodiments according to the invention, in the compound of Formula II, $R_3$ is selected from the group consisting of 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl-phenyl, 3-8-membered aliphatic heterocyclyl, 6-20-membered aryl, 5-20-membered heteroaryl, and 5-20-membered fused heteroaryl; and wherein the 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl-phenyl, 3-8-membered aliphatic heterocyclyl, 6-20-membered aryl, 5-20-membered heteroaryl, and 5-20-membered fused heteroaryl are unsubstituted or substituted independently by one or more (for example 1 or 2) substituents selected from the group consisting of halogen, hydroxyl, -NRR', $C_{1-3}$ alkyl, $C_{1-3}$ alkoxyl, cyano, 3-10-membered cycloalkyl, 3-8-membered aliphatic heterocyclyl, 6-20-membered aryl, 5-20-membered heteroaryl, 5-20-membered fused heteroaryl, halogenated $C_{1-3}$ alkyl, halogenated $C_{1-3}$ alkoxyl, $C_{1-3}$ alkoxy-$C_{1-3}$ alkyl, $C_{1-3}$ alkoxy-$C_{1-3}$ alkoxyl, and $C_{1-3}$ alkoxy-$C_{1-3}$ alkoxy-$C_{1-3}$ alkyl; $R_4$ is selected from H and methyl; $R_1$ is selected from H, halogen, and $C_{1-3}$ alkyl; $R_2$ is selected from the group consisting of hydrogen, cyano, nitro, halogen, $C_{1-3}$ alkyl, and $C_{1-3}$ alkoxyl; and n=1; $R_5$, $R_6$, R and R' are as defined in Formula I.

In some preferred embodiments according to the invention, in the compound of the Formula II, $R_3$ is selected from the group consisting of phenyl, quinazolinyl, quinoxalinyl, naphthyl, benzo[1,4]dioxanyl group, benzoimidazolyl, indolyl, [1,3,4]oxadiazolyl, [1,3,4]oxadiazol-phenyl, imidazol-phenyl, phenyl-cyclohexyl, benzocyclohexyl, tetrahydropyranyl, thienyl, benzothiazolyl, indazolyl, tetrahydropyran-$CH_2$-, cyclopropyl-$CH_2$-, pyridyl-$CH_2$-, and quinolyl; wherein the phenyl, quinazolinyl, naphthyl, benzo[1,4]dioxanyl group, benzoimidazolyl, indolyl, imidazol-phenyl, phenyl-cyclohexyl, benzocyclohexyl, tetrahydropyranyl, thienyl, benzothiazolyl, indazolyl, tetrahydropyran-$CH_2$-, cyclopropyl-$CH_2$-, pyridyl-$CH_2$-, and quinolyl are unsubstituted or substituted independently by one or more (for example 1 or 2) substituents selected from the group consisting of methoxyl-methyl, isopropyl, trifluoromethoxyl, methyl, halogen, methoxyl-ethoxyl-methyl, trifluoromethyl, phenyl, -$N(CH_3)_2$, difluoromethoxyl, pyrrolinyl, and morpholinyl; $R_4$ is H or methyl.

In some preferred embodiments according to the invention, in the compound of Formula II, $R_1$ is selected from H, halogen, and $C_{1-3}$ alkyl. In some preferred embodiments according to the invention, in the compound of Formula II, $R_2$ is selected from H, halogen, and $C_{1-3}$ alkyl.

In some preferred embodiments according to the invention, in the compound of Formula II, n=1.

In some preferred embodiments according to the invention, in the compound of Formula II, $R_5$ is F.

In some preferred embodiments according to the invention, in the compound of Formula II, $R_6$ is H.

In some preferred embodiments according to the invention, in the compound of Formula II, $R_3$ is selected from the group consisting of 6-15-membered aryl, benzo-fused 3-8-membered cycloalkyl, benzo-fused 3-8-membered aliphatic heterocyclyl, 3-8-membered aliphatic heterocyclyl, 5-10-membered heteroaryl-$CH_2$, 5-10-membered fused heteroaryl-$CH_2$, 3-8-membered cycloalkyl-$CH_2$, 3-8-membered aliphatic heterocyclyl-$CH_2$, 6-10-membered aryl-$CH_2$, 5-10-membered heteroaryl, and 5-10-membered fused heteroaryl; and wherein the 6-15-membered aryl, benzo-fused 3-8-membered cycloalkyl, benzo-fused 3-8-membered aliphatic heterocyclyl, 3-8-membered aliphatic heterocyclyl, 5-10-membered heteroaryl-$CH_2$, 5-10-membered fused heteroaryl-$CH_2$, 3-8-membered cycloalkyl-$CH_2$, 3-8-membered aliphatic heterocyclyl-$CH_2$, 6-10-membered aryl-$CH_2$, 5-10-membered heteroaryl, and 5-10-membered fused heteroaryl are unsubstituted or substituted independently by one or more (for example 1 or 2) substituents selected from the group consisting of halogen, $C_{1-3}$ alkyl, $C_{1-3}$ alkoxyl-$C_{1-3}$ alkoxyl, and NRR', wherein R and R' are independently selected from H and $C_{1-3}$ alkyl; $R_4$ is H or methyl;

$R_1$ is selected from H and halogen; $R_2$ is selected from the group consisting of H, halogen, $C_{1-3}$ alkyl, and $C_{1-3}$ alkoxyl, and n=1;

$R_5$ and $R_6$ are as defined in Formula I.

In some preferred embodiments according to the invention, in the compound of Formula II, $R_3$ is selected from the group consisting of phenyl, naphthyl, benzocyclohexyl, tetrahydropyranyl, pyridyl-$CH_2$-, and benzo[1,4]dioxanyl group, wherein the phenyl, naphthyl, benzocyclohexyl, tetrahydropyranyl, pyridyl-$CH_2$-, and benzo[1,4]dioxanyl group are unsubstituted or substituted independently by one or more (for example1 or 2) substituents selected from the group consisting of methyl, halogen, -$N(CH_3)_2$, and methoxy-methoxyl; $R_4$ is H or methyl.

In some preferred embodiments according to the invention, $R_3$ is selected from the group consisting of 3-7-membered cycloalkyl-$CH_2$, 3-7-membered aliphatic heterocyclyl-$CH_2$, 6-10-membered aryl-$CH_2$, 5-6-membered heteroaryl-$CH_2$-, benzo-fused 3-7-membered cycloalkyl, 3-7-membered aliphatic heterocyclyl, 6-10-membered aryl, 5-10-membered heteroaryl, and 5-10-membered fused heteroaryl; $R_4$ is H;

$R_1$ is H; $R_2$ is H or halogen, and n=1; $R_5$ is F; $R_6$ is H.

In some preferred embodiments according to the invention, in the compound of Formula II, $R_3$ is selected from 6-10-membered aryl and 3-7-membered aliphatic heterocyclyl; $R_4$ is H.

In some preferred embodiments according to the invention, in the compound of Formula II, $R_3$ is selected from phenyl and tetrahydropyranyl; $R_4$ is H.

In some embodiments according to the invention, the compound is a mixture of the cis-configuration and the trans-configuration in any ratio.

In some preferred embodiments according to the invention, the compound of Formula II is in cis (Z)-configuration.

In some preferred embodiments according to the invention, the compound of Formula II is in trans (E)-configuration.

In some preferred embodiments according to the invention, the compound has a structure as represented by Formula III:

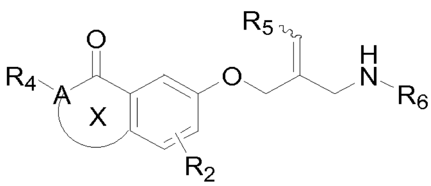

Formula III

wherein, the ring X is a 5-8-membered aliphatic ring or a 5-8-membered aliphatic heterocyclic ring; and wherein each of 5-8-membered aliphatic ring or the 5-8-membered aliphatic heterocyclic ring is unsubstituted or substituted independently by one or more (for example 1, 2, 3 or 4) substituents selected from the group consisting of =O, halogen, cyano, -NRR', $C_{1-4}$ alkyl, $C_{1-4}$ alkoxyl, halogenated $C_{1-4}$ alkyl, halogenated $C_{1-4}$ alkoxyl, 6-10-membered aryl, 5-10-membered heteroaryl, and 5-10-membered fused heteroaryl; or the aforementioned 5-8-membered aliphatic ring or the 5-8-membered aliphatic heterocyclic ring is fused with a 3-8-membered aliphatic ring to form a spiro structure;

$R_4$ is selected from the group consisting of hydrogen, $C_{1-6}$ alkyl, 3-8-membered cycloalkyl, 3-8-membered aliphatic heterocyclyl, 6-10-membered aryl, 5-10-membered heteroaryl and 5-10-membered fused heteroaryl; and wherein the $C_{1-6}$ alkyl, 3-8-membered cycloalkyl, 3-8-membered aliphatic heterocyclyl, 6-10-membered aryl, 5-10-membered heteroaryl or 5-10-membered fused heteroaryl is optionally substituted by one or more (for example 1, 2, 3 or 4) substituents selected from the group consisting of halogen, cyano, -NRR', hydroxyl, $C_{1-4}$ alkyl, $C_{1-4}$ alkoxyl, 3-8-

membered cycloalkyl, 3-8-membered aliphatic heterocyclyl, 6-10-membered aryl, 5-10-membered heteroaryl, and 5-10-membered fused heteroaryl;

$R_2$, $R_5$, $R_6$, R, R' and A are as defined in Formula I.

In some preferred embodiments according to the invention, in the compound of Formula III, A is selected from N and C.

In some preferred embodiments according to the invention, in the compound of Formula III, $R_2$ is selected from hydrogen and halogen. In some preferred embodiments according to the invention, in the compound of Formula III, $R_4$ is selected from hydrogen and $C_{1-3}$ alkyl.

In some preferred embodiments according to the invention, in the compound of Formula III, $R_5$ is halogen.

In some preferred embodiments according to the invention, in the compound of Formula III, $R_6$ is H.

In some preferred embodiments according to the invention, in the compound of Formula III, the ring X is a 5-7-membered aliphatic ring or a 5-7-membered aliphatic heterocyclic ring; and wherein the 5-7-membered aliphatic ring or the 5-7-membered aliphatic heterocyclic ring is unsubstituted or substituted by one or more (for example 1, 2, 3 or 4) substituents selected from the group consisting of =O, halogen, $C_{1-4}$ alkyl, 6-10-membered aryl, 5-10-membered heteroaryl, and 5-10-membered fused heteroaryl; or the aforementioned 5-7-membered aliphatic ring or the 5-7-membered aliphatic heterocyclic ring is fused with a 3-6-membered aliphatic ring to form a spiro structure; A is selected from N and C; $R_2$ is selected from F, Cl, and H; $R_4$ is selected from H, $CH_3$, and $CH_2CH_3$; $R_5$ is F; $R_6$ is H.

In some preferred embodiments according to the invention, in the compound of Formula III, the ring X is a 5-6-membered aliphatic ring or a 5-6-membered aliphatic heterocyclic ring; and wherein the 5-6-membered aliphatic ring or the 5-6-membered aliphatic heterocyclic ring is unsubstituted or substituted by one or more (for example 1, 2 or 3) substituents selected from the group consisting of O=, halogen, and $C_{1-3}$ alkyl; A is selected from N and C; $R_2$ is selected from F and H; $R_4$ is selected from H, $CH_3$, and $CH_2CH_3$; $R_5$ is F; $R_6$ is H.

In some preferred embodiments according to the invention, in the compound of Formula III, the ring X is a 5-membered aliphatic ring or a 6-membered nitrogen-containing aliphatic heterocyclic ring.

In some preferred embodiments according to the invention, in the compound of Formula III, the 6-membered nitrogen-containing aliphatic heterocyclic ring is a piperidine ring. In some embodiments according to the invention, the compound is a mixture of the cis-configuration and the trans-configuration in any ratio.

In some preferred embodiments according to the invention, the compound of Formula III is in cis (Z)-configuration.

In some preferred embodiments according to the invention, the compound of Formula III is in trans (E)-configuration.

In some preferred embodiments according to the invention, the compound has a structure as represented by the Formula IV:

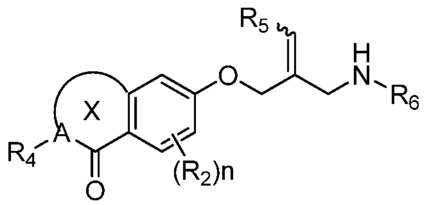

Formula IV

wherein, the ring X is a 5-8-membered aliphatic ring or a 5-8-membered aliphatic heterocyclic ring; and wherein the 5-8-membered aliphatic ring or the 5-8-membered aliphatic heterocyclic ring is unsubstituted or each substituted independently by one or more (for example 1, 2, 3 or 4) substituents selected from the group consisting of =O, halogen, cyano, -NRR', $C_{1-4}$ alkyl, $C_{1-4}$ alkoxyl, halogenated $C_{1-4}$ alkyl, halogenated $C_{1-4}$ alkoxyl, 6-10-membered aryl, 5-10-membered heteroaryl, and 5-10-membered fused heteroaryl; or the aforementioned 5-8-membered aliphatic ring or the 5-8-membered aliphatic heterocyclic ring is fused with a 3-8-membered aliphatic ring to form a spiro structure;

$R_4$ is selected from the group consisting of hydrogen, $C_{1-6}$ alkyl, 3-8-membered cycloalkyl, 3-8-membered aliphatic heterocyclyl, 6-10-membered aryl, 5-10-membered heteroaryl, and 5-10-membered fused heteroaryl; and wherein the $C_{1-6}$ alkyl, 3-8-membered cycloalkyl, 3-8-membered aliphatic heterocyclyl, 6-10-membered aryl, 5-10-membered heteroaryl, and 5-10-membered fused heteroaryl are optionally substituted by one or more (for example 1, 2, 3 or 4) substituents selected from the group consisting of halogen, cyano, hydroxyl, $C_{1-4}$ alkyl, $C_{1-4}$ alkoxyl, 3-8-membered

cycloalkyl, 3-8-membered aliphatic heterocyclyl, 6-10-membered aryl, 5-10-membered heteroaryl, and 5-10-membered fused heteroaryl; wherein the $C_{1-4}$ alkyl, 3-8-membered cycloalkyl, 3-8-membered aliphatic heterocyclyl, 6-10-membered aryl, 5-10-membered heteroaryl or 5-10-membered fused heteroaryl is optionally substituted by one or more (for example 1, 2 or 3) substituents selected from halogen, -NRR', and $C_{1-3}$ alkyl;
$R_2$, $R_5$, $R_6$, R, R', A and n are as defined in Formula I.

In some preferred embodiments according to the invention, in the compound of Formula IV, the ring X is selected from a 5-7-membered aliphatic ring or a 5-7-membered nitrogen- or oxygen-containing aliphatic heterocyclic ring (for example pyrrolidine ring, piperidine ring or tetrahydropyran ring); and wherein the 5-7-membered aliphatic ring or the 5-7-membered nitrogen- or oxygen-containing aliphatic heterocyclic ring is unsubstituted or each substituted independently by one or more (for example 1, 2, 3 or 4) substituents selected from halogen and $C_{1-4}$ alkyl (for example methyl); or the aforementioned 5-7-membered aliphatic ring or the 5-7-membered nitrogen- or oxygen-containing aliphatic heterocyclic ring is fused with a cyclopropyl to form a spiro structure.

In some preferred embodiments according to the invention, in the compound of Formula IV, $R_4$ is selected from the group consisting of hydrogen, phenyl, diaza-naphthyl, halogenated benzyl, tetrahydropyranyl-$CH_2$-, $C_{1-3}$ alkyl (for example methyl, and ethyl), cyclopentyl, benzyl, and tetrahydropyrayl.

In some preferred embodiments according to the invention, in the compound of Formula IV, $R_2$ is selected from hydrogen and halogen.

In some preferred embodiments according to the invention, in the compound of Formula IV, $R_5$ is selected from halogen.

In some preferred embodiments according to the invention, in the compound of Formula IV, $R_6$ is H. In some preferred embodiments according to the invention, in the compound of Formula IV, A is selected from N and C.

In some preferred embodiments according to the invention, in the compound of Formula IV, the ring X is a 5-7-membered aliphatic ring or a 5-7-membered aliphatic heterocyclic ring; and wherein the 5-7-membered aliphatic ring or the 5-7-membered aliphatic heterocyclic ring is unsubstituted or substituted by one or more(for example 1, 2, 3 or 4) substituents selected from the group consisting of =O, halogen, $C_{1-4}$ alkyl, 6-10-membered aryl, 5-10-membered heteroaryl, and 5-10-membered fused heteroaryl; or the aforementioned 5-7-membered aliphatic ring or the 5-7-membered aliphatic heterocyclic ring is fused with a 3-6-membered aliphatic ring to form a spiro structure; A is selected from N and C; each of $R_2$ is independently selected from F, Cl, and H, and n=1 or 2; $R_4$ is selected from the group consisting of H, $C_{1-4}$ alkyl, benzyl, and halogenated benzyl; $R_5$ is F; $R_6$ is H.

In some preferred embodiments according to the invention, in the compound of Formula IV, the ring X is a 5-6-membered aliphatic ring or a 5-6-membered aliphatic heterocyclic ring; and wherein the 5-6-membered aliphatic ring or the 5-6-membered aliphatic heterocyclic ring is unsubstituted or substituted by one or more (for example 1, 2, 3 or 4) substituents selected from =O, halogen, and $C_{1-4}$ alkyl; A is selected from N and C; each of $R_2$ independently is selected from F, Cl, and H, and n=1; $R_4$ is selected from H, $C_{1-4}$ alkyl, benzyl and chloro benzyl; $R_5$ is F; $R_6$ is H'.

In some preferred embodiments according to the invention, in the compound of Formula IV, the ring X is selected from pyrrolidine ring and piperidine ring, the pyrrolidine ring or piperidine ring is unsubstituted or substituted by one or more methyl.

In some preferred embodiments according to the invention, in the compound of Formula IV, $R_4$ is selected from H, halogenated benzyl, and methyl.

In some preferred embodiments according to the invention, in the compound of Formula IV, the ring X is a 6-membered aliphatic heterocyclic ring (for example a 6-membered nitrogen-containing aliphatic heterocyclic ring, such as piperidine ring); and wherein the 6-membered aliphatic heterocyclic ring is unsubstituted or substituted by one or more (for example 1, 2 or 3) substituents selected from =O, halogen, and $C_{1-3}$ alkyl; A is N; each of $R_2$ is independently is selected from F and H, and n=1; $R_4$ is selected from H, $CH_3$, and $CH_2CH_3$; $R_5$ is F; $R_6$ is H.

In some embodiments according to the invention, the compound is a mixture of the cis-configuration and the trans-configuration in any ratio.

In some preferred embodiments according to the invention, the compound of Formula IV is in cis (Z)-configuration.

In some preferred embodiments according to the invention, the compound of Formula IV is in trans (E)-configuration.

In some preferred embodiments according to the invention, the compound has a structure as represented by the formula V:

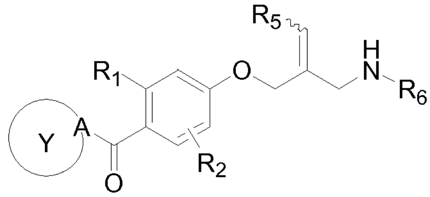

Formula V

wherein, the ring Y is a 5-8-membered aliphatic heterocyclic ring or a 5-8-membered aliphatic ring; and wherein the 5-8-membered aliphatic heterocyclic ring or the 5-8-membered aliphatic ring is unsubstituted or each substituted independently by one or more (for example 1, 2, 3 or 4) substituents selected from the group consisting of halogen, cyano, -NRR', nitro, hydroxyl, $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl, wherein the $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl are unsubstituted or each substituted independently by one or more (for example 1, 2, 3 or 4) substituents selected from the group consisting of halogen, cyano, -NRR', $C_{1-6}$ alkyl, 6-20-membered aryl, 5-20-membered heteroaryl, and 5-20-membered fused heteroaryl; or the aforementioned 5-8-membered aliphatic heterocyclic ring or the 5-8-membered aliphatic ring is fused with a 6-20-membered aromatic ring or a 5-20-membered aromatic heterocycle to form a fused ring system, wherein the fused ring system is unsubstituted or each substituted independently by one or more (for example 1, 2, 3 or 4) substituents selected from the group consisting of halogen, cyano, -NRR', $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl; $R_1$, $R_2$, $R_5$, $R_6$, R and R' are as defined in Formula I; A is selected from N and C.

In some preferred embodiments according to the invention, in the compound of formula V, the ring Y is a 5-7-membered aliphatic heterocyclic ring; and wherein the 5-7-membered aliphatic heterocyclic ring is unsubstituted or substituted by one or more (for example 1, 2, 3 or 4) substituents selected from the group consisting of halogen, cyano, -NRR', 6-15-membered aryl, and $C_{1-4}$ alkyl, wherein the 6-15-membered aryl and $C_{1-4}$ alkyl are unsubstituted or substituted independently by one or more (for example 1, 2, 3 or 4) substituents selected from the group consisting of halogen, $C_{1-4}$ alkyl, halogenated $C_{1-4}$ alkyl, and 6-10-membered aryl; or the aforementioned 5-7-membered aliphatic heterocyclic ring is fused with a 6-10-membered aromatic ring or a 5-10-membered aromatic heterocycle to form a fused ring system, and wherein the fused ring system is unsubstituted or each substituted independently by one or more (for example 1, 2, 3 or 4) substituents selected from the group consisting of halogen, $C_{1-4}$ alkyl, halogenated $C_{1-4}$ alkyl, and 6-10-membered aryl; $R_1$, $R_2$, $R_5$, $R_6$, R and R' are as defined in Formula I; A is N.

In some preferred embodiments according to the invention, in the compound of formula V, $R_1$ is H.

In some preferred embodiments according to the invention, in the compound of formula V, $R_2$ is selected from halogen.

In some preferred embodiments according to the invention, in the compound of formula V, $R_5$ is selected from halogen.

In some preferred embodiments according to the invention, in the compound of formula V, $R_6$ is H.

In some preferred embodiments according to the invention, in the compound of formula V, A is N.

In some preferred embodiments according to the invention, in the compound of formula V, the ring Y is a 5-7-membered nitrogen-containing aliphatic heterocyclic ring; and wherein the 5-7-membered nitrogen-containing aliphatic heterocyclic ring is unsubstituted or substituted by one or more substituents selected from phenyl and diphenylmethyl; or the aforementioned 5-7-membered nitrogen-containing aliphatic heterocyclic ring is fused with a benzene ring or a thiophene ring to form a fused ring system, the fused ring system is unsubstituted or substituted independently by one or more substituents selected from halogen, phenyl, and trifluoromethyl.

In some preferred embodiments according to the invention, in the compound of formula V, the ring Y is a 5-7-membered aliphatic heterocyclic ring; and wherein the 5-7-membered aliphatic heterocyclic ring is unsubstituted or substituted by one or more (for example 1, 2, 3 or 4) substituents selected from 6-10-membered aryl; A is N, $R_1$ is H; $R_2$ is H or F; $R_5$ is F; $R_6$ is H.

In some preferred embodiments according to the invention, in the compound of formula IV, the ring Y is a 5-7-membered aliphatic heterocyclic ring containing one nitrogen atom; and wherein the 5-7-membered aliphatic heterocyclic ring containing one nitrogen atom is unsubstituted or substituted by phenyl; or the aforementioned 5-7-membered aliphatic heterocyclic ring containing one nitrogen atom is fused with a benzene ring to form a fused ring system, the fused ring system is unsubstituted or substituted by one or more substituents selected from halogen, phenyl, and trifluoromethyl.

In some embodiments according to the invention, the compound is a mixture of the cis-configuration and the

trans-configuration in any ratio.

In some preferred embodiments according to the invention, the compound of formula V is in cis (Z)-configuration.

In some preferred embodiments according to the invention, the compound of formula V is in trans (E)-configuration.

In some preferred embodiments according to the invention, the compound has a structure represented by the formula VI:

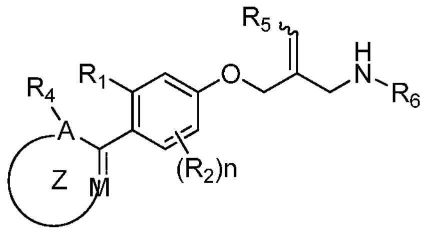

Formula VI

wherein, the ring Z is a 3-10-membered aliphatic heterocycle, a 6-20-membered aromatic ring or a 5-20-membered aromatic heterocycle; and wherein each of 3-10-membered aliphatic heterocycle, 6-20-membered aromatic ring or 5-20-membered aromatic heterocycle is unsubstituted or substituted independently by one or more (for example 1, 2, 3 or 4) substituents selected from the group consisting of halogen, cyano, -NRR', nitro, hydroxyl, =O, $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl; or the aforementioned 3-10-membered aliphatic heterocycle, 6-20-membered aromatic ring or 5-20-membered aromatic heterocycle is fused with a 6-20-membered aromatic ring or a 5-20-membered aromatic heterocycle to form a fused ring system, wherein the fused ring system is unsubstituted or each substituted independently by one or more (for example 1, 2, 3 or 4) subsituents selected from the group consisting of halogen, cyano, -NRR', $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl;

$R_4$ is selected from hydrogen, $C_{1-6}$ alkyl, and $C_{1-6}$ alkoxyl, or $R_4$ is absent or forms a covalent bond;

$R_1$, $R_2$, $R_5$, $R_6$, R, R', A, M and n are as defined in Formula I.

In some preferred embodiments according to the invention, in the compound of formula VI, $R_1$ is selected from H or halogen.

In some preferred embodiments according to the invention, in the compound of formula VI, $R_2$ is selected from H or halogen.

In some preferred embodiments according to the invention, in the compound of formula VI, $R_5$ is selected from halogen.

In some preferred embodiments according to the invention, in the compound of formula VI, $R_6$ is selected from H or $C_{1-6}$ alkyl.

In some preferred embodiments according to the invention, in the compound of formula VI, A is selected from S or N.

In some preferred embodiments according to the invention, in the compound of formula VI, M is selected from C or N.

In some preferred embodiments according to the invention, in the compound of formula VI, n=1 or 2.

In some preferred embodiments according to the invention, in the compound of formula VI, the ring Z is a 5-7-membered aliphatic heterocyclic ring, a 6-10-membered aromatic ring or a 5-10-membered aromatic heterocycle; and wherein each of 5-7-membered aliphatic heterocyclic ring, 6-10-membered aromatic ring or 5-10-membered aromatic heterocycle is unsubstituted or substituted independently by one or more (for example 1, 2, 3 or 4) subsituents selected from the group consisting of halogen, cyano, -NRR', nitro, hydroxyl, $C_{1-3}$ alkyl, $C_{1-3}$ alkoxyl, halogenated $C_{1-3}$ alkyl, and halogenated $C_{1-6}$ alkoxyl; or the aforementioned 5-7-membered aliphatic heterocyclic ring, 6-10-membered aromatic ring or 5-10-membered aromatic heterocycle is fused with a 6-10-membered aromatic ring or a 5-10-membered aromatic heterocycle to form a fused ring system, wherein the fused ring system is unsubstituted or substituted independently by one or more (for example 1, 2, 3 or 4) subsituents selected from the group consisting of halogen, cyano, -NRR', $C_{1-3}$ alkyl, $C_{1-3}$ alkoxyl, halogenated $C_{1-3}$ alkyl, halogenated $C_{1-3}$ alkoxyl, 6-10-membered aryl, 5-10-membered heteroaryl,

and 5-10-membered fused heteroaryl;

$R_4$ is selected from hydrogen and $C_{1-6}$ alkyl, or $R_4$ is absent or forms a covalent bond;

$R_1$ is selected from hydrogen and halogen; each of $R_2$ is independently selected from hydrogen, halogen, and cyano, and n=1 or 2; $R_5$ is F or Cl; $R_6$ is selected from hydrogen and $C_{1-6}$ alkyl; A is selected from N and S; M is N;

R and R' are as defined in Formula I.

In some preferred embodiments according to the invention, in the compound of formula VI, the ring Z is a 5-7-membered aliphatic heterocyclic ring, a 6-10-membered aromatic ring or a 5-10-membered aromatic heterocycle; and wherein each of 5-7-membered aliphatic heterocyclic ring, 6-10-membered aromatic ring or 5-10-membered aromatic heterocycle is unsubstituted or substituted independently by one or more (for example 1, 2 or 3) subsituents selected from the group consisting of halogen, cyano, -NRR', nitro, hydroxyl, $C_{1-3}$ alkyl, $C_{1-3}$ alkoxyl, halogenated $C_{1-3}$ alkyl, and halogenated $C_{1-6}$ alkoxyl; wherein R and R' are independently selected from hydrogen and $C_{1-3}$ alkyl; or the aforementioned 5-7-membered aliphatic heterocyclic ring, 6-10-membered aromatic ring or 5-10-membered aromatic heterocycle is fused with a 6-10-membered aromatic ring or a 5-10-membered aromatic heterocycle to form a fused ring system, wherein the fused ring system is unsubstituted or substituted independently by one or more (for example 1, 2, 3 or 4) subsituents selected from the group consisting of halogen, cyano, -NRR', $C_{1-3}$ alkyl, $C_{1-3}$ alkoxyl, halogenated $C_{1-3}$ alkyl, and halogenated $C_{1-3}$ alkoxyl; wherein R and R' are independently selected from hydrogen and $C_{1-3}$ alkyl;

$R_4$ is selected from hydrogen and $C_{1-6}$ alkyl, or $R_4$ is absent or forms a covalent bond;

$R_1$ is selected from H and halogen; each of $R_2$ independently is H or F, and n=1 or 2;

$R_5$ is F or Cl;

$R_6$ is selected from H and $C_{1-6}$ alkyl;

A is N;

M is N.

In some preferred embodiments according to the invention, in the compound of formula VI, the ring Z is a 5-7-membered aromatic heterocycle, wherein the 5-7-membered aromatic heterocycle contains one or more heteroatoms selected from N or S; optionally, wherein the 5-7-membered aromatic heterocycle is substituted by $C_{1-3}$ alkyl. In some preferred embodiments according to the invention, the ring Z is selected from the group consisting of pyrimidine ring, imidazole ring, thiazole ring, and [1,2,4]triazine ring. In some preferred embodiments according to the invention, the aforementioned 5-7-membered aromatic heterocycle is fused with a 6-10-membered aromatic ring (for example a benzene ring) or a 5-10-membered nitrogen-containing aromatic heterocycle (for example an imidazole ring) to form a fused ring system, optionally, the fused ring system is substituted by halogen.

In some preferred embodiments according to the invention, in the compound of formula VI, the ring Z is a pyrimidine ring, an imidazole ring or a thiazole ring, which is optionally substituted by methyl group; or wherein the pyrimidine ring, imidazole ring or thiazole ring is fused with a benzene ring to from a fused ring system, the fused ring system is optionally substituted by halogen.

In some preferred embodiments according to the invention, in the compound of formula VI, the ring Z is a pyrimidine ring or an imidazole ring; or wherein the pyrimidine ring or the imidazole ring is fused with a benzene ring to form a fused ring system.

In some embodiments according to the invention, the compound is a mixture of the cis-configuration and the trans-configuration in any ratio.

In some preferred embodiments according to the invention, the compound of formula VI is in cis (Z)-configuration.

In some preferred embodiments according to the invention, the compound of formula VI is in trans (E)-configuration.

In the above formulas I to VI according to the invention, all the groups defined in the preferred embodiments can be suitably selected and combined, so as to obtain different formula scopes or specific embodiments. These scopes and embodiments each belong to the invention.

In some preferred embodiments according to the invention, the compound is selected from the group consisting of:

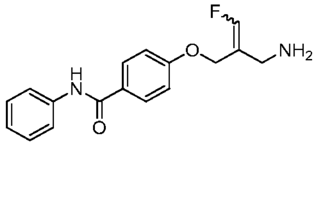

1,

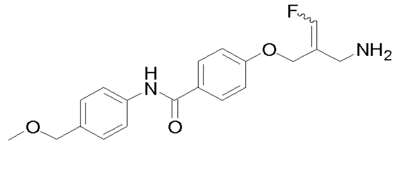

2,

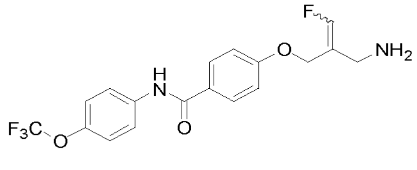

3,

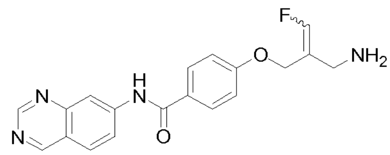

4,

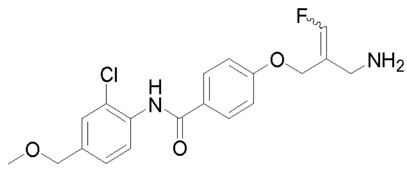

5,

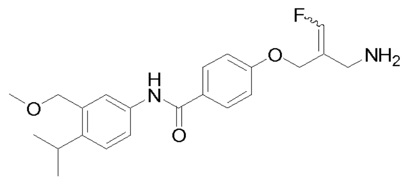

6,

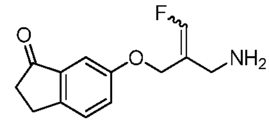

7,

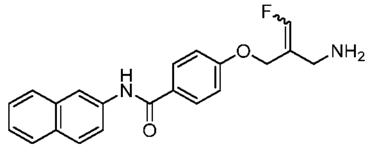

8,

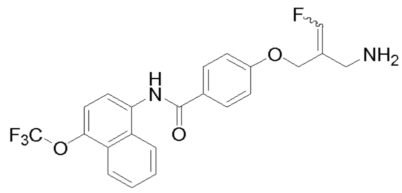

9,

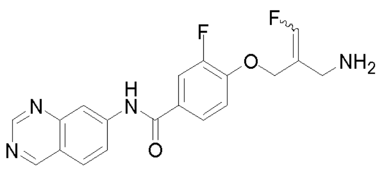

10,

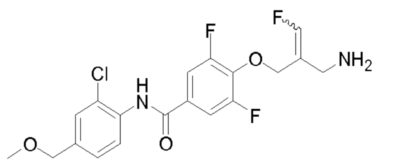

11,

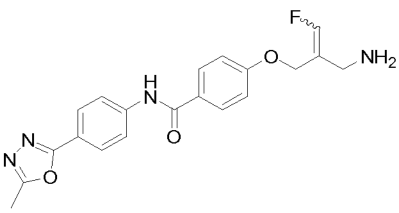

12,

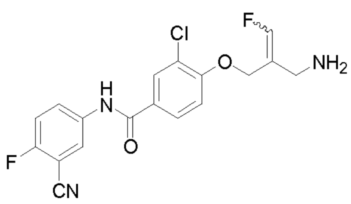

13,

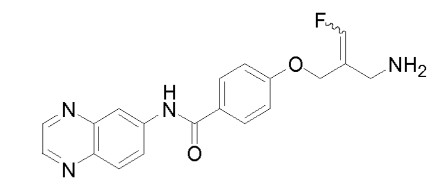

14,

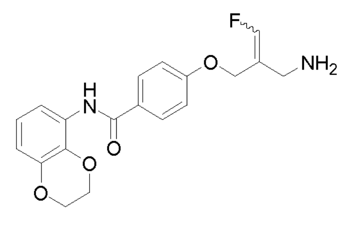

15,

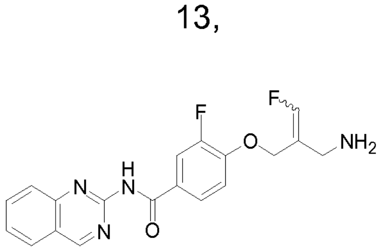

16,

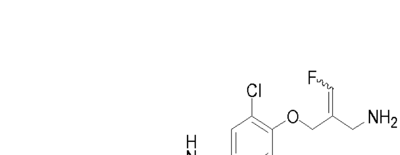

17,

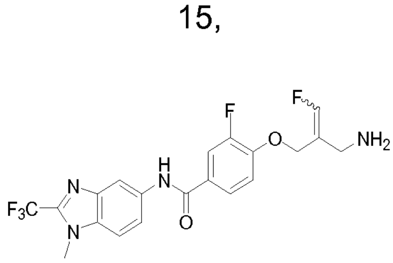

18,

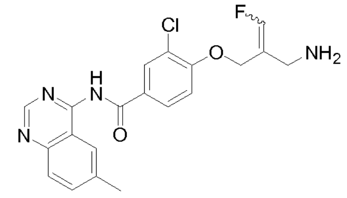

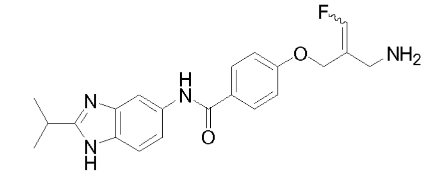

20,

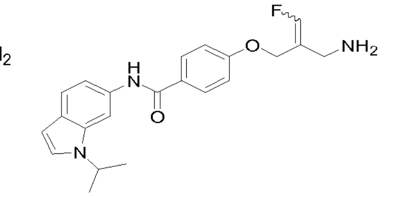

19,

21,

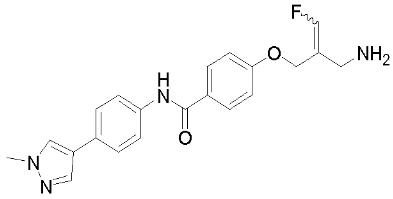

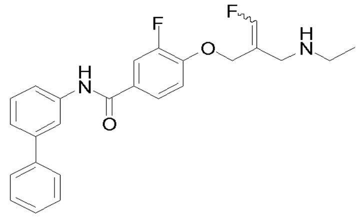

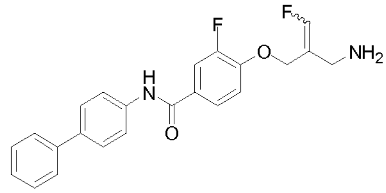

22,

24,

23,

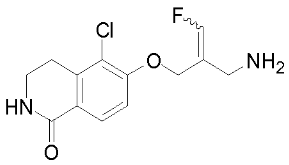

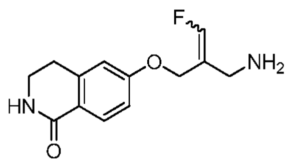

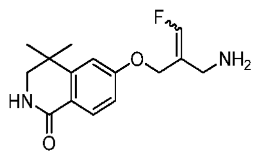

25,

26,

27,

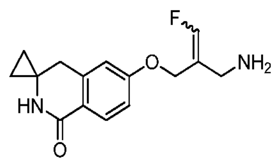

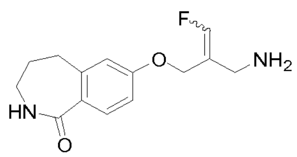

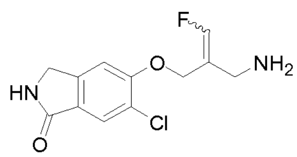

29,

30,

28,

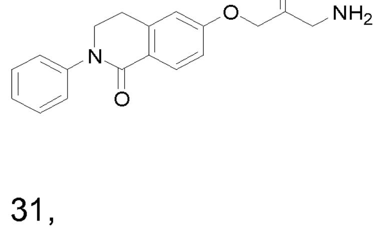

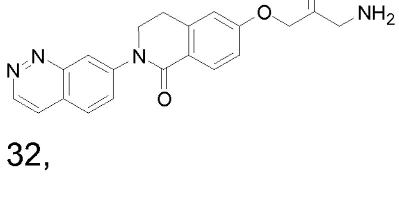

32,

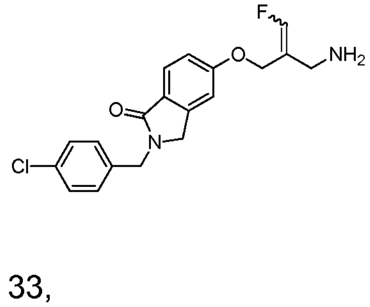

31,

33,

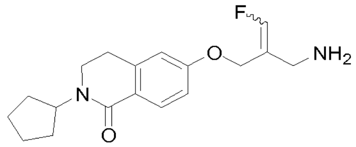

34,

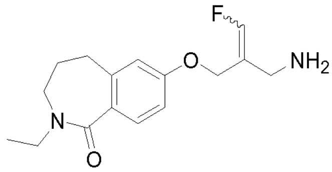

35,

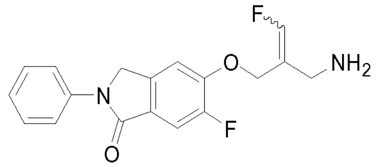

36,

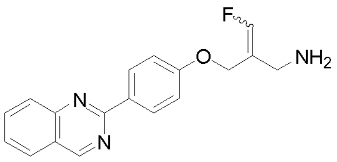

37,

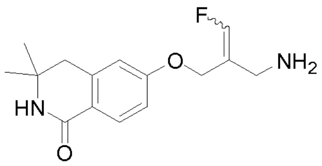

38,

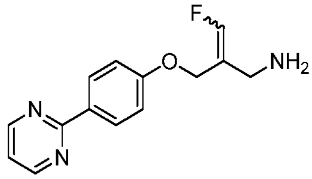

39,

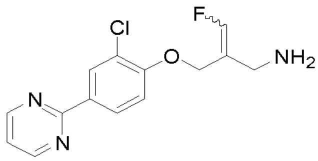

40,

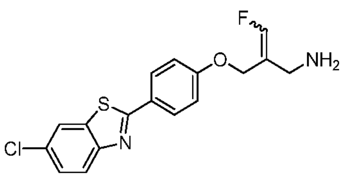

41,

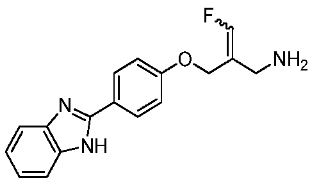

42,

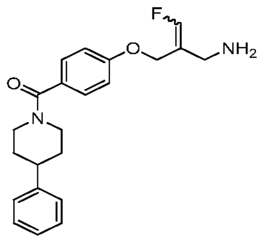

44,

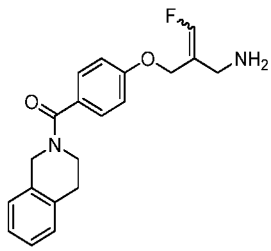

45,

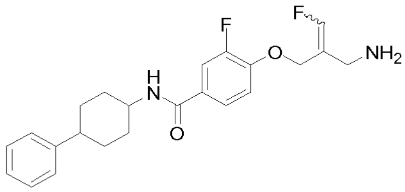

43,

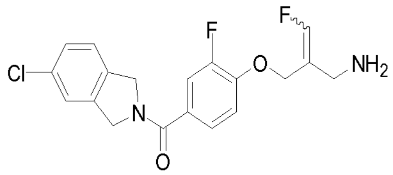

46,

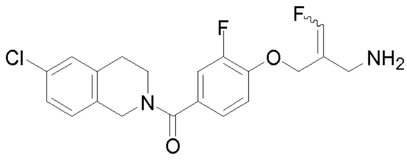

47,

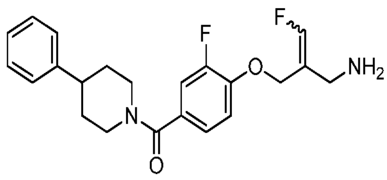

48,

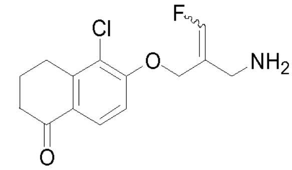

49,

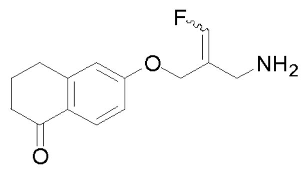

50,

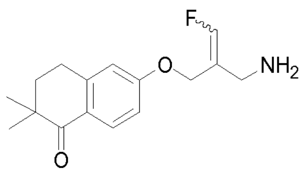

51,

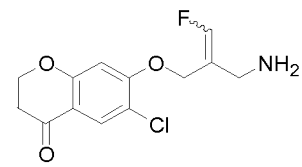

52,

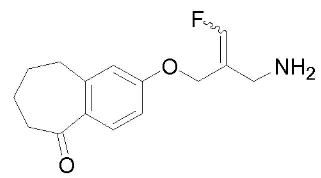

53,

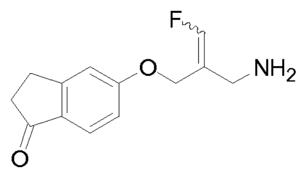

54,

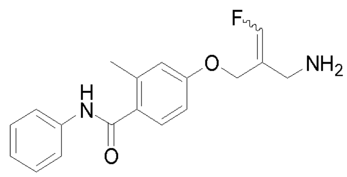

55,

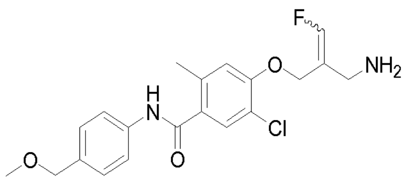

56,

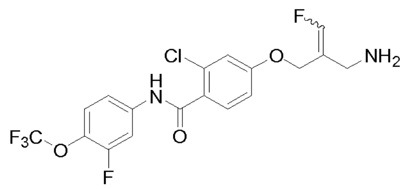

57,

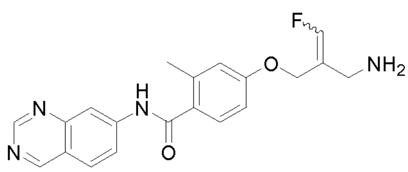

58,

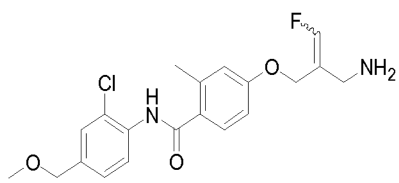

59,

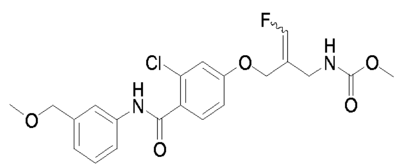

60,

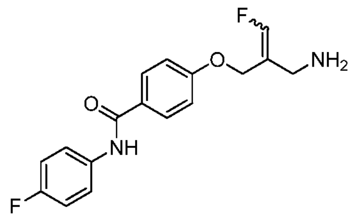

61,

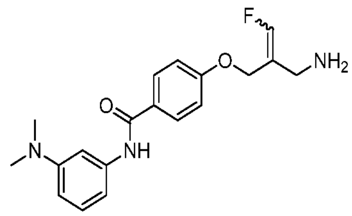

62,

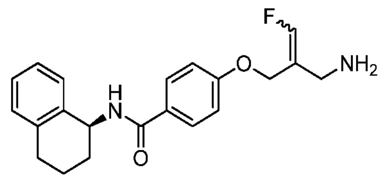

63,

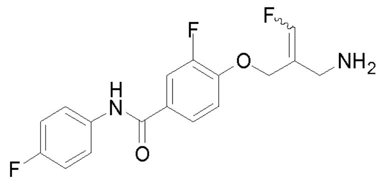

64,

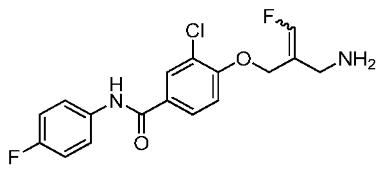

65,

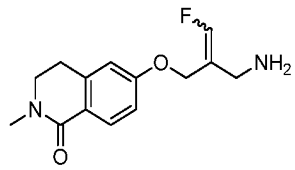

66,

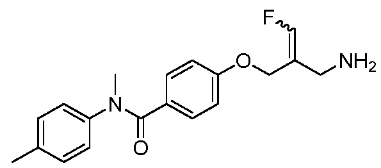

67,

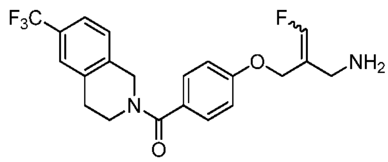

68,

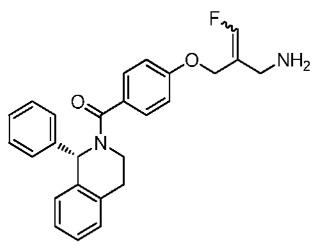

69,

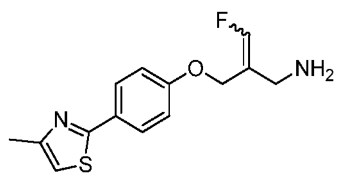

70,

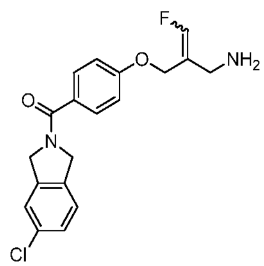

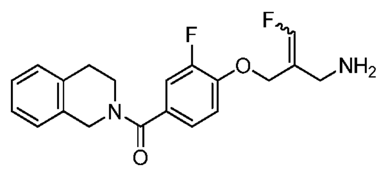

72,

71,

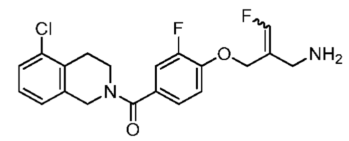

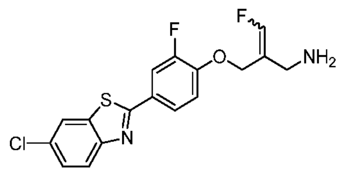

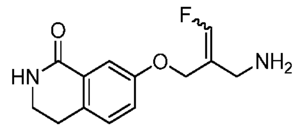

73,

74,

75,

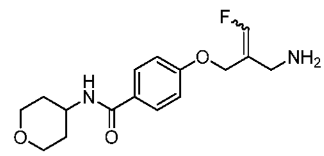

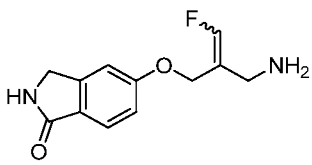

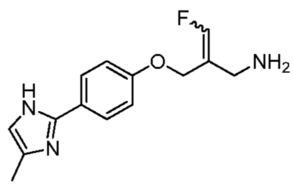

76,

77,

78,

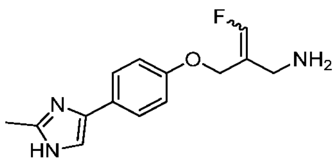

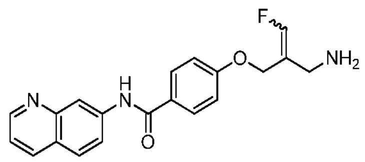

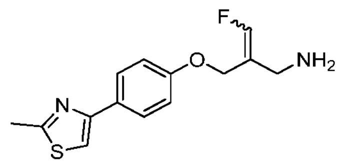

79,

80,

81,

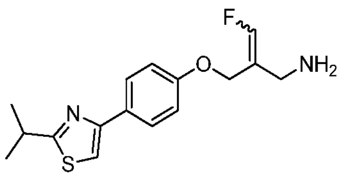

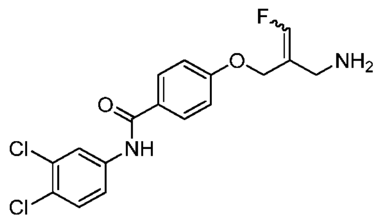

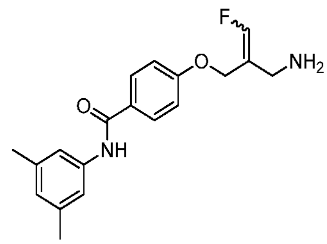

82,

83,

84,

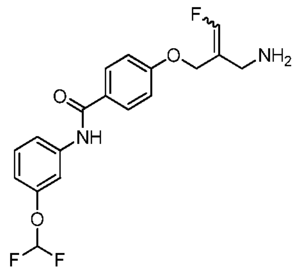

85,

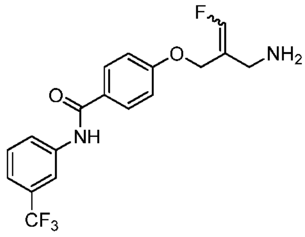

86,

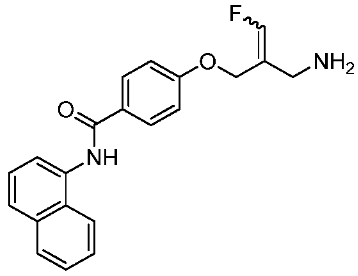

87,

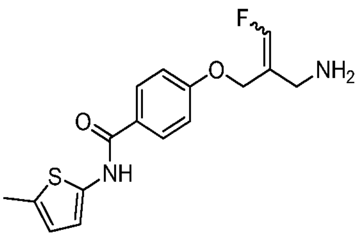

88,

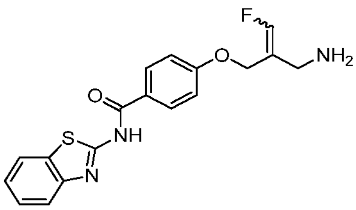

89,

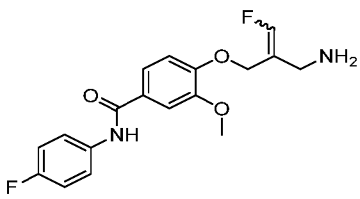

90,

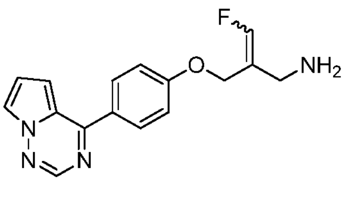

91,

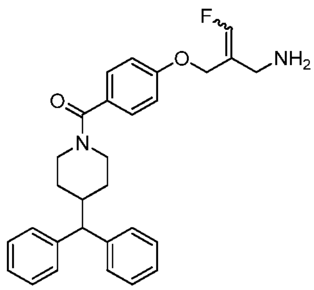

92,

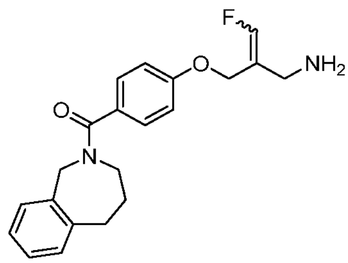

93,

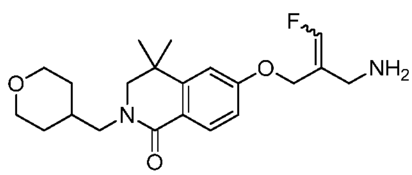

94,

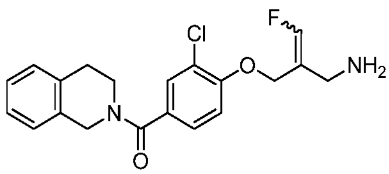

95,

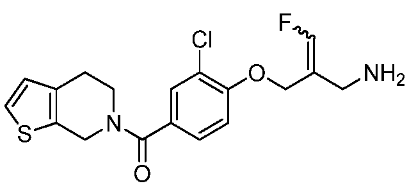

96,

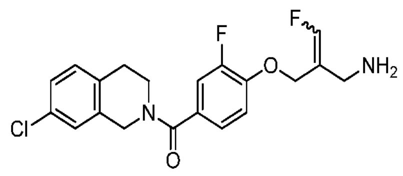

97,

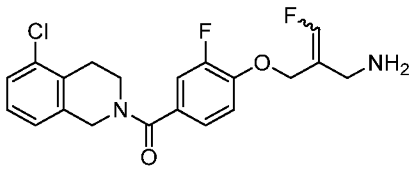

98,

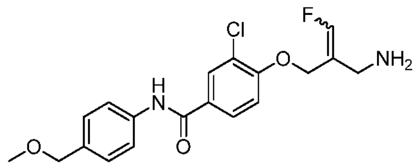

99,

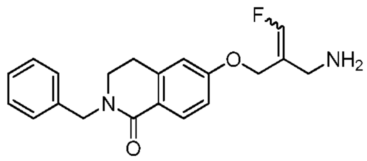

100,

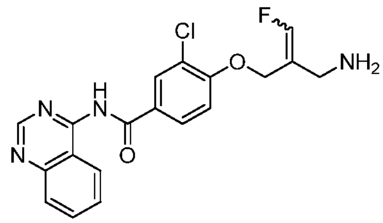

101,

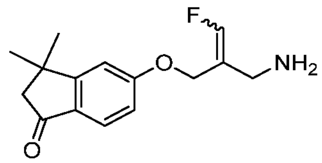

102,

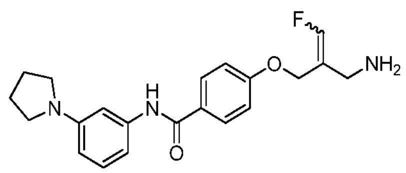

103,

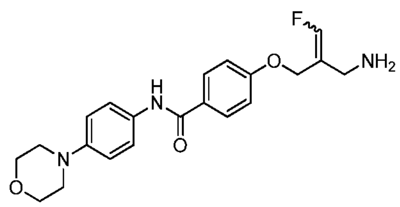

104,

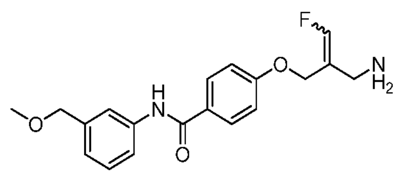

105,

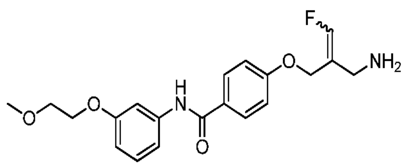

106,

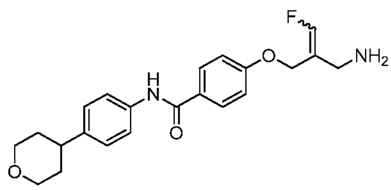

107,

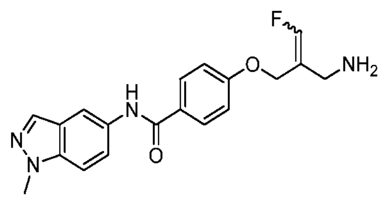

108,

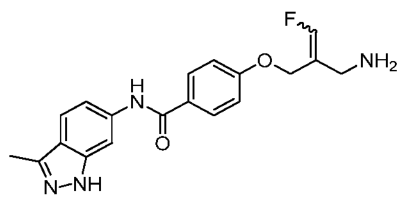

109,

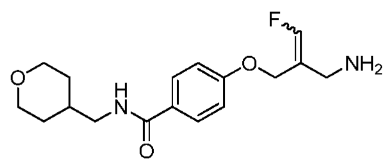

110,

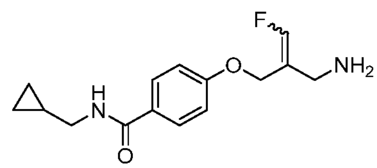

111,

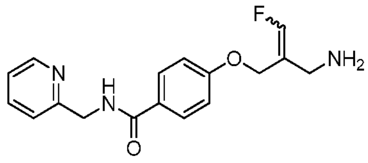

112,

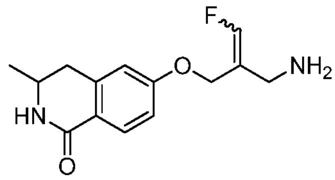

113,

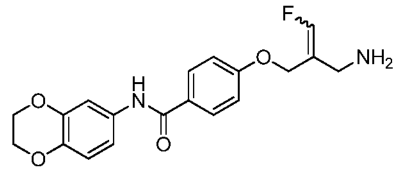

and 114.

In some preferred embodiments according to the invention, the pharmaceutically acceptable salt of the compound is a hydrochloride or a trifluoroacetate, and preferably, the pharmaceutically acceptable salt is a hydrochloride.

In some preferred embodiments according to the invention, the pharmaceutically acceptable salt is

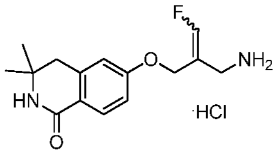

HC38

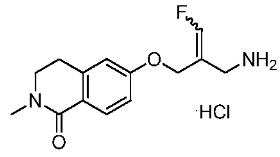

or HC66.

In some preferred embodiments according to the invention, In some preferred embodiments according to the invention, the pharmaceutically acceptable salt is

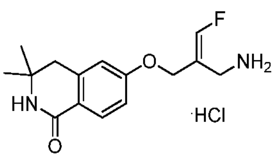

HC38-E

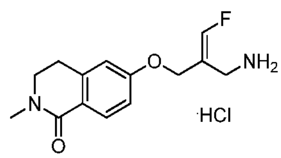

or HC66-E.

The compound of the invention refers to the compound covered by Formulas I to VI, or a pharmaceutically acceptable salt, an ester, a hydrate, a solvate, an isomer, or an isotope-labeled compound thereof; any crystal form or racemate thereof; a metabolite thereof; or a mixture thereof.

A second aspect of the invention provides a pharmaceutical composition, comprising a compound according to the first aspect of the invention, or a pharmaceutically acceptable salt, an ester, a hydrate, a solvate, an isomer, or an isotope-labeled compound thereof; any crystal form or racemate thereof; a metabolite thereof; or a mixture thereof, and one or more pharmaceutical excipients.

The pharmaceutical excipient in the text refers to a vehicle and an additive intended for manufacturing drug dosage forms and prescription dispensing, and refers to, except for active ingredients, any materials that have been subjected to reasonable evaluations in the safety and contained in pharmaceutical formulations. The pharmaceutical excipient may be used for shaping, acting as carriers and increasing stabilities, and may have the important functions in solubilizing, improving solubilization and sustained or controlled releasing, and is an important ingredient that can influence the quality, safety and efficiency of corresponding drugs. According to the sources, the excipients may be classified into natural, semi-synthetical, and synthetical. According to the functions and uses, the excipients may be classified into solvents, propellants, solubilizing agents, cosolvents, emulsifiers, colorants, adhesives, disintegrants, fillers, lubricants, wetting agents, osmolytes, stabilizers, fluidizers, odorants, preservatives, suspending agents, coating materials, aromatics, anti-adhesives, antioxidants, chelating agents, penetration enhancers, pH regulators, buffers, plasticizers, surfactants, foamers, defoamers, thickeners, inclusion agent, moisturizers, absorbents, diluents, flocculants and anti-flocculants, filter aids, release blockers, etc.; according to the administration route, the excipients may be classified into oral, injection, mucosa, transdermal or local administrations, inhalation administration via nose or mouth

and ocular administration. Specific pharmaceutical excipients include water, lactose, glucose, fructose, sucrose, sorbitol, mannitol, polyethylene glycol, propylene glycol, starch, rubber, gelatin, alginate, calcium silicate, calcium phosphate, cellulose, water syrup, methyl cellulose, polyvinylpyrrolidone, alkyl para-hydroxybenzosorbate, talc, magnesium stearate, stearic acid, glycerin, sesame oil, olive oil, soybean oil, etc.

The pharmaceutical composition may be administrated in any forms, as long as achieving the effects of preventing, relieving, avoiding or healing symptoms in human or animal patients. For example, it can be prepared into various suitable dosages according to its administration route.

When being administrated orally, the pharmaceutical composition can be prepared into any orally acceptable formulation forms, including, but being not limited to, tablets, capsules, granules, pills, syrups, orally-administrated solutions, orally-administrated suspensions and orally-administrated emulsions. Carriers used in tablets generally include lactose and corn starch, and additionally, lubricants, such as magnesium stearate, also can be added. Diluents as used in the capsules generally include lactone and dry core starch. Orally administrated suspensions are usually used by mixing active ingredients with suitable emulsifiers and suspensions. Optionally, some sweeteners, aromatics or colorants can be added to the above orally administrated formulations.

When being transdermally or locally administrated, the pharmaceutical composition can be prepared into an appropriate ointment, lotion or liniment form, in which the active ingredient is suspended or dissolved in one or in a variety of carriers. Carriers used in the ointment preparations include, but are not limited to, mineral oil, liquid vaseline, white vaseline, propylene glycol, polyethylene oxide, polypropylene oxide, emulsified wax and water; lotion or liniment can be used. Carriers used in the lotion or liniment form include, but are not limited to, mineral oil, dehydrated sorbitol monostearate, tween 60, hexadecanol wax, hexadecylene aromatic alcohol, 2-octyl dodecanol, benzyl alcohol and water.

The pharmaceutical composition may be also administrated in an injection form, including an injection liquid, sterile powder for injection and a concentrated solution for injection. In the pharmaceutical composition, useful carriers and solvents include water, a Ringer's solution and isotonic sodium chloride solutions. Additionally, sterilized nonvolatile oil can also be used as a solvent or a suspension medium, for example monoglyceride or diglyceride.

The dose and administration scheme of the pharmaceutical composition can be easily determine by one of ordinary skilled person in the clinical art. The composition or compound according to the invention is generally administered twice every day to once every three days, preferably once every day, and the total administration dosage is 0.01 - 1000 mg/time. Generally, the treatment dose varies depending on issues in interest, such as age, sexuality and general health status of patients to be treated; treatment frequency and desired effects; degree of tissue damage; symptom duration; and other variables that can be adjusted by various physicians. The desired dose can be administrated once or by multiple times, so as to obtain desirable effects. Also, the pharmaceutical composition of the invention can be provided in a form of unit dose.

The invention is further provided with a process of preparing the compound of the first aspect, wherein $R_6$ in Formula I is hydrogen, the process consists in a route selected from:

Route I:

Subjecting compound SM-1 and compound INT-1 to a nucleophilic substitution reaction to afford compound INT-2; removing the protection of compound INT-2 to afford a target product;

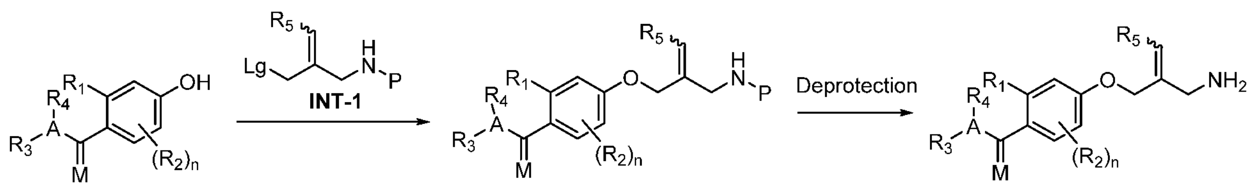


SM-1 INT-2

Route II:

Subjecting compound SM-1 and compound INT-3 to a Mitsunobu reaction to afford compound INT-2; removing the protection of compound INT-2 to afford a target product;

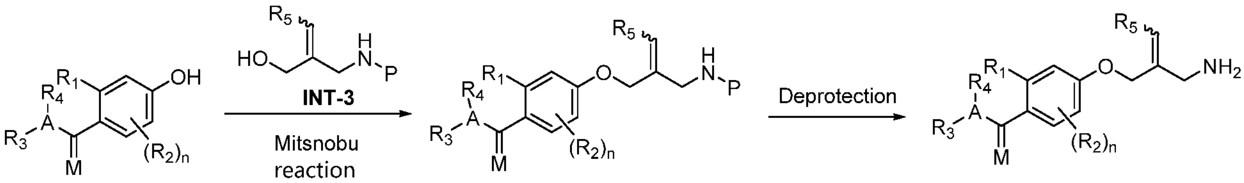


SM-1 INT-2

Route III:

Subjecting compound SM-2 and compound INT-1 to a nucleophilic substitution reaction to afford compound INT-4; subjecting compound INT-4 to a coupling reaction to afford compound INT-2; removing the protection of compound INT-2 to afford a target product;

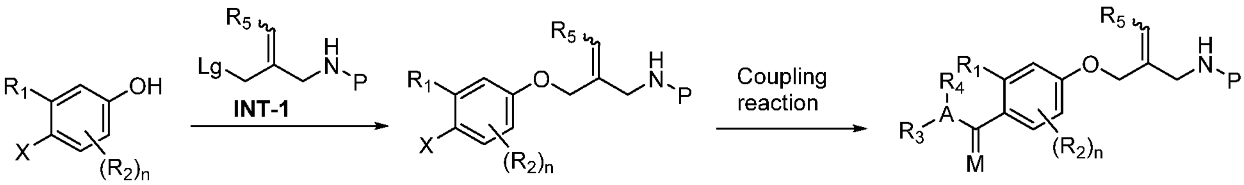


SM-2 INT-4 INT-2

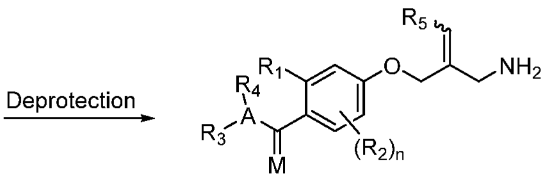


Route IV:

Subjecting compound SM-2 and compound INT-3 to a Mitsunobu reaction to afford compound INT-4; subjecting compound INT-4 to a coupling reaction to afford compound INT-2; removing the protection of compound INT-2 to afford a target compound;

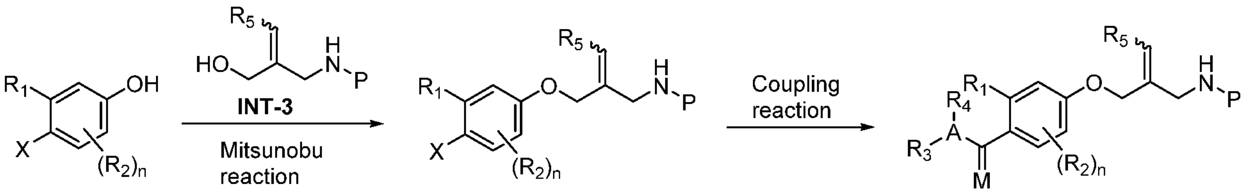


SM-2 INT-4 INT-2

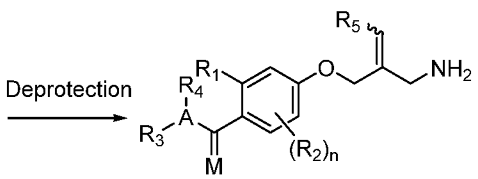


Route V:

Subjecting compound SM-3 and compound INT-1 to a nucleophilic substitution reaction to afford compound INT-5; removing the protection of compound INT-5 to afford a target product;

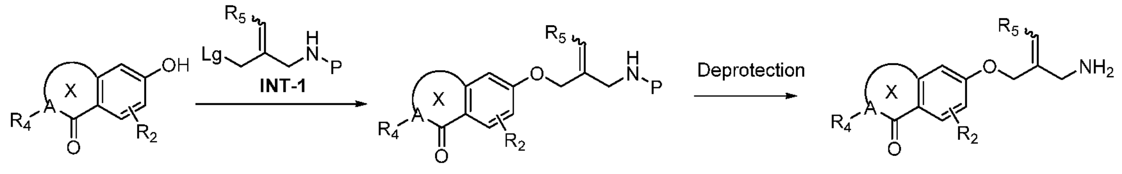


SM-3 INT-5

Scheme VI:

Subjecting compound SM-3 and compound INT-3 to a Mitsunobu reaction to afford compound INT-5; removing the protection of compound INT-2 to afford a target product;

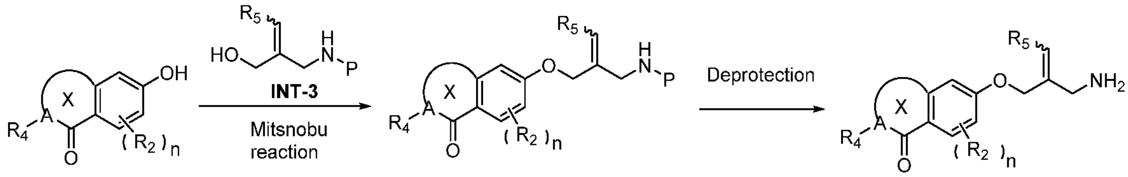


SM-3 INT-5

wherein, Lg represents a leaving group, such as halogen, -OTs, etc.; P represents an amino protective group, such as Boc, Cbz, Fmoc, benzyl, etc.; the other atoms and groups are as previously defined.

The intermediates INT-1 and INT-3 are commercially available or can be prepared according to the processes in the specific examples of the invention.

The invention is further provides the use of the compounds, or a pharmaceutically acceptable salt, an ester, a hydrate, a solvate, an isomer, or an isotope-labeled compound thereof; any crystal form or racemate thereof; a metabolite thereof; or a mixture thereof in the manufacture of VAP-1/SSAO inhibitors.

The invention is further provides the use of the compounds, or a pharmaceutically acceptable salt, an ester, a hydrate, a solvate, an isomer, or an isotope-labeled compound thereof; any crystal form or racemate thereof; a metabolite thereof; or a mixture thereof, or the pharmaceutical composition of the invention in the manufacture of of a medicament for the treatment of a disease or disorder associated with the overactivity of VAP-1/SSAO.

The invention is further provides a method for treating a disease or disorder associated with the overactivity of VAP-1/SSAO, comprising administrating a patient in need of such treatment with a therapeutically effective amount of the above defined compound, or a pharmaceutically acceptable salt, an ester, a hydrate, a solvate, an isomer, or an isotope-labeled compound thereof; any crystal form or racemate thereof; a metabolite thereof; or a mixture thereof, or the pharmaceutical composition of the invention.

The "effective amount" as used in the invention refers to an amount that is sufficient to obtain or at least partially obtain desirable effects. For example, the "therapeutically effective amount" refers to an amount that is sufficient to heal or at least partially prevent diseases of patients suffered therefrom or its complications thereof. Measurement of such an effective amount is entirely within the abilities of a person skilled in the art. For example, an effective amount for treatment uses depends on severity of diseases to be treated, overall states of patient's immune systems, patient's common conditions, such as age, weight and sexuality, drug administration means, and other treatments that perform simultaneously.

In the invention, the diseases or disorders associated with the overactivity of VAP-1/SSAO are selected from the group consisting of an inflammatory disease (for example a liver-related inflammatory disease, such as hepatitis, hepatomegaly, hepatic fibrosis, cirrhosis or ascites; a respiratory tract-related inflammatory disease, such as tracheitis, pneumonia, pulmonary fibrosis, asthma, acute lung injury, acute respiratory distress syndrome, bronchitis or chronic obstructive pulmonary disease; an eye-related inflammatory disease, for example Le uveitis; other inflammation, for example synovitis or peritonitis), organ and/or tissue transplantation rejection, an autoimmune disease (for example rheumatoid arthritis or multiple sclerosis (for example chronic multiple sclerosis)), a skin disease (for example eczema or psoriasis), a diabetes mellitu (for example type I or type II diabetes) and stroke.

In the invention, the subject is selected from any animals, preferably mammals such as cattle, equine, sheep,

pigs, canines, felines, rodents, and primates. Among them, the particularly preferred subject is human.

The invention is further provides a method for inhibiting the activity of VAP-1/SSAO in a cell, comprising the steps of administrating cells in need with an effective amount of the compound as above defined, or a pharmaceutically acceptable salt, an ester, a hydrate, a solvate, an isomer, or an isotope-labeled compound thereof; any crystal form or racemate thereof; a metabolite thereof; or a mixture thereof.

In some embodiments according to the invention, the method is applied in vivo, and for example, said cell is in vivo cell of a subject (for example mammals such as cattle, equine, sheep, pigs, canines, felines, rodents, or primates (for example human); or, the method is applied in vitro, and for example, said cell is in vitro cell (for example cell lines or a cell derived from the subject).

The invention is further provides a kit for reducing or inhibiting the activity of VAP-1/SSAO in a cell, and said kit comprises the compound of the invention, or a pharmaceutically acceptable salt, an ester, a hydrate, a solvate, an isomer, or an isotope-labeled compound thereof; any crystal form or racemate thereof; a metabolite thereof; or a mixture thereof, and optionally an instruction for use.

In the invention, unless being otherwise specified, scientific and technical terms used in the text have the meaning that are commonly understood by a person skilled in the art. For better understanding of the invention, definitions and explanations for related terms are provided as follows:

In the invention, the "hydrogen" and the hydrogen in each group include protium (H), deuterium (D), and tritium (T).

In the invention, the "halogen" includes fluorine, chlorine, bromine and iodine.

In the invention, the "$C_{1-6}$ alkyl" refers to a linear or branched alkyl having 1 to 6 carbon atoms, such as $C_{1-4}$ alkyl, $C_{1-3}$ alkyl, $C_{1-2}$ alkyl, $C_1$ alkyl, $C_2$ alkyl, $C_3$ alkyl, $C_4$ alkyl, $C_5$ alkyl or $C_6$ alkyl. Specific examples include, but are not limited to, methyl, ethyl, propyl, iso-propyl, n-butyl, iso-butyl, sec-butyl, tert-butyl, n-pentyl, and n-hexyl.

In the invention, the "halogenated $C_{1-6}$ alkyl" refers to a group formed by substituting the above defined $C_{1-6}$ alkyl with one or more above defined halogen, for example, halogenated $C_{1-4}$ alkyl, halogenated $C_{1-2}$ alkyl, fluoro $C_1$-$C_4$ alkyl, and fluoro $C_1$-$C_2$ alkyl. Specific examples include, but are not limited to, fluoromethyl, difluoromethyl, trifluoromethyl, trifluoroethyl, and pentafluoroethyl.

In the invention, the "$C_{1-6}$ alkoxyl" refers to a group formed in the form of $C_{1-6}$ alkyl-O-, wherein the "$C_{1-6}$ alkyl" is as previously defined, for example, $C_1$-$C_4$ alkoxyl, and $C_1$-$C_2$ alkoxyl. Specific examples include, but are not limited to, methoxyl, ethoxyl, propoxy, isopropoxy, butoxy, 2-butoxy, isobutoxy, sec-butoxy, tert-butoxy, pentoxy, and hexyloxy.

In the invention, the "halogenated $C_{1-6}$ alkoxyl" refers to a group formed by substituting the above defined $C_{1-6}$ alkoxyl with one or more above defined halogen, for example, halogenated $C_{1-4}$ alkoxyl, halogenated $C_{1-2}$ alkoxyl, fluoro $C_1$-$C_4$ alkoxyl, and fluoro $C_1$-$C_2$ alkoxyl. Specific examples include, but are not limited to, trifluoromethoxyl, and difluoromethoxyl.

In the invention, the "3-10-membered cycloalkyl" refers to a saturated or partially saturated monocyclic hydrocarbon radical containing 3 to 10 carbon atoms, for example 3-6-membered cycloalkyl. Specific examples include, but are not limited to, cyclopropyl, cyclobutyl, cyclopentyl, cyclohexyl, cyclohexenyl, cyclohexadienyl, cycloheptanyl, cycloheptatrienyl, and cyclooctyl.

In the invention, the "3-8-membered aliphatic heterocyclyl" refers to a saturated or partially saturated non-aromatic cyclic group containing 3 to 8 ring members wherein at least one ring member is a heteroatom selected from N, O and S, and preferably, the number of said heteroatom is 1, 2, 3 or 4, for example, 3-6-membered aliphatic heterocyclyl, 3-8-membered nitrogen-containing aliphatic heterocyclyl, 3-8-membered oxygen-containing aliphatic heterocyclyl, 3-8-membered sulfur-containing aliphatic heterocyclyl, 3-6-membered nitrogen-containing aliphatic heterocyclyl, 3-6-membered oxygen-containing aliphatic heterocyclyl, and 3-6-membered sulfur-containing aliphatic heterocyclyl. Specific examples include, but are not limited to, oxiranyl, oxocyclobutyl, pyrrolidinyl, tetrahydrofuryl, piperidyl, piperazinyl, tetrahydropyranyl, and homopiperazinyl.

In the invention, the "6-20-membered aryl" refers to a aromatic monocyclic or polycyclic hydrocarbon radical containing 6-20 ring members, for example 6-10-membered aryl. Specific examples include, but are not limited to, phenyl, naphthyl, anthryl, and phenanthryl.

In the invention, the "5-20-membered heteroaryl" refers to an aromatic monocyclic group in which at least one member is a heteroatom selected from N, O and S, and preferably, the number of the heteroatom is 1, 2, 3 or 4, for example, 5-10-membered heteroaryl, 5-6-membered heteroaryl. For example, 5-20-membered oxygen-containing heteroaryl, 5-20-membered sulfur-containing heteroaryl, 5-20-membered nitrogen-containing heteroaryl, 5-10-membered oxygen-containing heteroaryl, 5-10-membered sulfur-containing heteroaryl, 5-10-membered nitrogen-containing heteroaryl, 5-6-membered oxygen-containing heteroaryl, 5-6-membered sulfur-containing heteroaryl, and 5-6-membered nitrogen-containing heteroaryl. Specific examples include, but are not limited to, furyl, thienyl, pyrrolyl, thiazolyl, isothiazolyl, thiadiazolyl, oxazolyl, iso-oxazolyl, imdazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridone group, 4-pyridone group, pyrimidinyl, 2*H*-1,2-oxazinyl, 4*H*-1,2-oxazinyl, 6*H*-1,2-oxazinyl, 4*H*-1,3-oxazinyl, 6*H*-1,3-oxazinyl, 4*H*-1,4-oxazinyl, pyridazinyl, pyrazinyl, 1,2,3-triazinyl, 1,3,5-triazinyl, 1,2,4,5-tetraazinyl, azacycloheptatrienyl, and 1,3-diazacycloheptatrienyl.

In the invention, the "3-8-membered aliphatic ring" refers to a saturated or partially saturated non-aromatic carbocyclic ring, for example, including 5-6-membered aliphatic ring, 5-membered aliphatic ring, 6-membered aliphatic ring, 7-membered aliphatic ring, and 8-membered aliphatic ring.

In the invention, the "3-10-membered aliphatic heterocycle" refers to a saturated or partially saturated non-aromatic aliphatic ring in which at least one ring member is a heteroatom selected from N, O and S, and preferably, the number of said heteroatom is 1, 2, 3 or 4, for example, 3-10-membered nitrogen-containing aliphatic heterocycle, 3-10-membered oxygen-containing aliphatic heterocycle, 3-10-membered sulfur-containing aliphatic heterocycle, 5-8-membered nitrogen-containing aliphatic heterocycle, 5-8-membered oxygen-containing aliphatic heterocycle, 5-8-membered sulfur-containing aliphatic heterocycle, 5-6-membered nitrogen-containing aliphatic heterocycle, 5-6-membered oxygen-containing aliphatic heterocycle, 5-6-membered sulfur-containing aliphatic heterocycle. For example, 5-6-membered aliphatic heterocycle, 5-membered aliphatic heterocycle, 6-membered aliphatic heterocycle, 7-membered aliphatic heterocycle, and 8-membered aliphatic heterocycle.

In the invention, the "6-20-membered aromatic ring" refers to aromatic rings having 6-20 carbon atoms, for example, 6-20-membered aromatic rings, and 6-10-membered aromatic rings. Specific examples include, but are not limited to, benzene ring, naphthalene ring, anthracene ring and phenanthrene ring.

In the invention, the "5-20-membered aromatic heterocycle" refers to an aromatic ring in which at least one ring member is a heteroatom selected from N, O and S, and preferably, the number of the heteroatom is 1, 2, 3 or 4, for example, aromatic heterocycle containing 1 or 2 N atoms, aromatic heterocycle only containing one O atom, aromatic heterocycle only containing one S atom, etc. For example, 5-20-membered aromatic heterocycle, 5-14-membered aromatic heterocycle, 5-10-membered aromatic heterocycle, and 5-6-membered aromatic heterocycle, etc. For example, 5-20-membered nitrogen-containing aromatic heterocycle, 5-20-membered oxygen-containing aromatic heterocycle, 5-20-membered sulfur-containing aromatic heterocycle, 5-14-membered nitrogen-containing aromatic heterocycle, 5-14-membered oxygen-containing aromatic heterocycle, 5-14-membered sulfur-containing aromatic heterocycle, 5-10-membered nitrogen-containing aromatic heterocycle, 5-10-membered oxygen-containing aromatic heterocycle, 5-10-membered sulfur-containing aromatic heterocycle, 5-6-membered nitrogen-containing aromatic heterocycle, 5-6-membered oxygen-containing aromatic heterocycle, and 5-6-membered sulfur-containing aromatic heterocycle. Specific examples include, but are not limited to, imidazole ring, thiazole ring, and pyrimidine ring.

In the invention, the "fused ring system (fused ring)" refers to a polycyclic structures formed by two or more than two (for example 3, 4 or 5) carbocycles or heterocycles sharing common ring edges, wherein the carbocycles include aliphatic rings and aromatic rings, and the heterocycles include aromatic heterocycles and aliphatic heterocycles. The fused ring system includes, but are not limited to, fused ring system formed by aliphatic ring and aliphatic ring, fused ring system formed by aliphatic ring and aliphatic heterocycle, fused ring system formed by aliphatic ring and aromatic ring, fused ring system formed by aliphatic ring and aromatic heterocycle, fused ring system formed by aliphatic heterocycle and aliphatic heterocycle, fused ring system formed by aliphatic heterocycle and aromatic heterocycle, fused ring system formed by aliphatic heterocycle and aromatic ring, fused ring system formed by aromatic heterocycle and aromatic heterocycle, and fused ring system formed by aromatic heterocycle and aromatic ring; for example, fused ring system formed by a 5-7-membered aliphatic heterocycle, a 6-10-membered aromatic ring or a 5-10-membered aromatic heterocycle and a 6-10-membered aromatic ring or a 5-10-membered aromatic heterocycle, or fused ring system formed by a 5-8-membered aliphatic heterocycle or a 5-8-membered aliphatic ring and a 6-20-membered aromatic ring or a 5-20-membered aromatic heterocycle; for example, benzofuran, benzoisofuran, benzothiophene, indole, isoindole, benzoxazole, benzoimidazole, indazole, benzotriazole, quinoline, 2-quinolone, 4-quinolone, 1-isoquinolone, iso-quinoline, acridine, phenanthridine, benzopyridazine, phthalazine, quinazoline, quinoxaline, phenozine, pteridine, purine, naphthalene, phenazine, phenothiazine, benzoimidazole ring, benzothiazole ring, quinazoline ring, or pyrrolidone [2,1-f][1,2,4] triazine ring.

In the invention, the "fused heteroaryl" refers to an aromatic fused ring group containing one or more (for example 1, 2, 3 or 4) heteroatoms (for example O, N, and S), which may be formed by sharing common ring edge between aryl and heteroaryl, or heteroaryl and heteroaryl.

In the invention, the "metabolite" refers to a compound converted in vivo from the compound of the invention, or a pharmaceutically acceptable salt, an ester, a solvate, a hydrate, an isomers, an isotope-labeled compound or any crystal form or racemate thereof after being administered to the subject in need.

In the invention, the "pharmaceutically acceptable salt" refers to a salt retains the biological efficiency and properties of the parent compound, for example, the salt may be prepared by the following process: protonating the proton-withdrawing moiety of the compound and/or de-protonating the proton-donating moiety of the compound. It should be noted that the protonation of the proton-withdrawing moiety can result in the formation of a cationic matter, wherein the charge is balanced due to the presence of physiological anions, and the de-protonation of the proton-donating moiety can result in the formation of a anionic matter, wherein the charge is balanced due to the presence of physiological cations. Specific examples include, but are not limited to, a sodium salt, a potassium salt, a calcium salt, an ammonia salt, an aluminum salt, a propionate, a tyrate, a formate, a salt formed with cysteine, a hydriodate, a nicotinate, an

oxalate, a hydrochloride or a trifluoroacetates

In the compounds of Formulas I to VI according to the invention, atoms in the groups include isotopes thereof. For example, H or hydrogen incudes $^1H$, $^2H$, and $^3H$.

In the invention, the "formulation" or "dosage form" should include solid and liquid formulations of the compound of the invention. A person skilled in the art would understand that, according to desired doses and pharmacokinetic parameters, the ingredient of the compound of the invention may be present in different formulations.

In the embodiments of the invention, the compound or salt thereof may be present in a solvate form, the solvent is selected from organic solvents (for example ethanol or propanol).

In the embodiments of the invention, the compound or salt thereof may be present in a hydrate form.

In the embodiments of the invention, if the compound contains a chiral carbon, the invention includes isomers formed by any stereo configurations based on the chiral carbon, for example including racemates or any single mirror-image isomer. Further, the invention includes all other possible stereo isomers. In other words, the compound of the invention includes all of its enantiomers, diastereoisomers, cis/trans isomers, and racemates, etc.

Beneficial effects of the invention

Through intensive research, the inventor surprisingly finds a compound as represented by Formula I, having VAP-1/SSAO inhibitory activity, can be used for the treatment of disease associated with the overactivity of VAP-1/SSAO. The compound has outstanding inhibitory activity against vascular adhesion protein-1/semicarbazide-sensitive amine oxidase, and good selectivity over monoamine oxidase and diamine oxidase, as well as improved metabolic stability in vivo.

Specific modes for carrying out the invention

The embodiments of the invention will be described in detail below by reference to the following examples. However, a person skilled in the art would understand that the following examples are only intened for illustrating the invention, rather than limiting the scope of the invention. As for the examples in which conditions are not indicated, they are carried out under conventional conditions or conditions proposed by the manufacturers. All reagents or apparatus without indicated manufactures are conventional products that are commercially available.

$^1H$ NMR spectroscopy is recorded by a Bruker apparatus (400 MHz) at ambient temperature with TMS as the internal standard. Chemical shift ($\delta$) is given in ppm, and the coupling constant (J) is given in Hz. The splitting of peaks in $^1H$ NMR spectra are abbreviated as follows: s (singlet), d(doublet), t (triplet), q (quartlet), m (multiple), br (broad). Deuterated solvent used is deuterated methanol ($CD_3OD$), deuterated chloroform ($CDCl_3$) or hexa-deuterated dimethyl sulfoxide (DMSO-*d*6).

LC-MS is measured by an Aglient 1200 liquid chromatograph in combination with an Aglient 6120 Quadrupole-type mass spectrometer at 214nm and 254nm.

A preparative liquid chromatography method is conducted by using SHIMADZU CBM-20A- and Aglient 1260-type preparative liquid chromatographs with a C18 OBD 19×150 mm 5 μM preparative column, at 214 nm, wherein water is mobile phase A, and acetonitrile (adding 0.5‰ formic acid) is mobile phase B, the linear gradient elution is as the following table:

| Time (min) | A% | B% |
|---|---|---|
| 0 | 90 | 10 |
| 15 | 40 | 60 |
| 30 | 10 | 90 |

MS is measured by an Agilent (ESI) mass spectrometer, (manufacturer: Agilent).

The preparative high-performance liquid chromatography is performed in a SHMADZU LC-8A preparative liquid chromatograph (YMC, ODS, 250×20 mml chromatographic column).

A thin layer chromatography (TLC) is performed on an aluminum plate (20×20 cm) manufactured by Merck, and the silica gel used in thin layer chromatography separation and purification is GF 254 (0.4-0.5 nm) manufactured in Yantai.

Reaction is monitored by thin layer chromatography (TLC) or LCMS, and the developing agent system includes dichloromethane and methanol system, n-hexane and ethyl acetate system, and petroleum ether and ethyl acetate system, wherein the volume ratios of the solvents may be adjusted according to the polarity of the compounds being

tested, and triethylamine may be added for the adjustment.

Unless being otherwise specified, all of the solvents applied in the reactions are either commercially available anhydrous solvents or HPLC-grade solvents, without further purification.

Microwave reactions are performed in a BiotageInitiator + (400 W, RT-300 °C) microwave reactor.

In the examples, unless being particularly specified, the reaction is conducted at room temperature (20 °C-30 °C).

The reagents applied in the invention are purchased from Acros Organics, Aldrich Chemical Company, Topbiochem. LTD, and other companies.

Unless being particularly specified, all of the abbreviations used in conventional synthesis methods as well as the examples and the intermediate synthesis examples have the following meanings:

| | |
|---|---|
| DMF: N,N-dimethyl formamide; | DMA: N,N-dimethyl acetamide; |
| DMSO: dimethyl sulfoxide; | NMP: N-methylpyrrolidone; |
| DIBAL-H: diisobutyl aluminum hydride; | DIEA: N, N-diisopropylethylamine; |
| THF: tetrahydrofuran; | Boc: tert-butoxycarbonyl; |
| NBS: N-bromosuccinimide; | Cbz-Cl: benzyl chloroformate; |
| m-CPBA: meta-chloroperbenzoic acid; | TFA: trifluoroacetic acid; |
| $Et_2O$: diethylether; | EtOH: ethanol; |
| Dioxane: 1,4-dioxane; | TLC: thin layer chromatography; |
| HATU: O-(7-azabenzotriazole-1-yl)-N,N,N',N'-tetramethylurea hexafluorophosphate; | |
| Me: methyl; | DCM: dichloromethane; |
| EA: ethyl acetate; | DDQ: 2,3-dicholor-5,6-dicyano-1,4- benzoquinone; |
| Xphos: 2-dicyclohexylphosphino-2',4',6'-triisopropyl biphenyl; | |
| PE: petroleum ether; | MTBE: methyl tert-butyl ether; |
| ACN: acetonitrile | |

Preparation of tert-butyl (2-bromethyl-3-fluoroallyl)carbamate (Int-1)

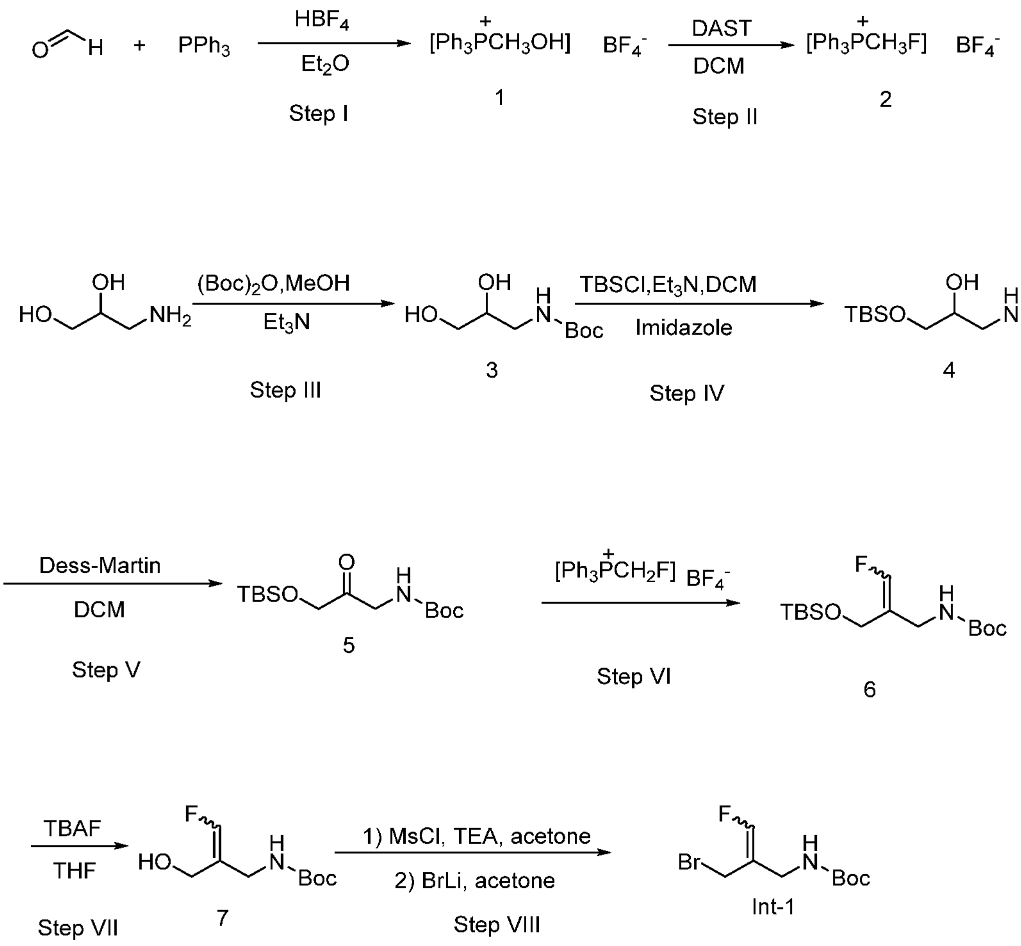


Step I: Preparation of hydroxymethyl triphenylphosphonium tetrafluoroborate (1)

Paraformaldehyde (30 g, 1.00 mol) and triphenylphosphine (250 g, 0.95 mol) were placed in a reaction flask and added with ether (2 L). To the reaction flask with stirring, aqueous fluoroboric acid solution (525 g, 2.38 mol) was slowly added. The mixture was reacted at room temperature for 5 days.The reaction solution was concentrated by removing the ether, and then filtered to provide solids. The solids were washed with ether (500 mL), water (500 mL) and ether (500 mL) in order. The solids were dried in a drying oven (50 °C) overnight to afford the product hydroxymethyl triphenylphosphonium tetrafluoroborate (240 g, 67%).

Step II: Preparation of fluoromethyl triphenylphosphonium tetrafluoroborate (2)

The compound 1 (150 g, 0.396 mol) was dissolved in dichloromethane (1.5 L) and cooled to 0 °C. To the reaction system, DAST (191 g, 1.187 mol) was slowly dripped. Then the reaction solution was raised to room temperature and reacted overnight. The reaction solution was quenched by pouring into ice water. The resulting mixture was extracted with dichloromethane. The obtained organic phases were combined, washed once by water (500 mL) and saturated saline solution (500 ml) respectively, dried over anhydrous sodium sulfate, and concentrated. The obtained crude product was dissolved with dichloromethane (350 mL) under heating, and then was slowly dripped into petroleum ether while stirring. During the process, solid precipitations were accompanied. The solids were filtered and dried in drying oven (50 °C) overnight to give the product fluoromethyl triphenylphosphonium tetrafluorofluoroborate (110 g, 73%).

Step III: Preparation of tert-butyl (2,3-dihydroxypropyl)carbamate (3)

3-amino-1,2-propanediol (10 g, 110 mmol) was added to anhydrous methanol (200 mL) and then triethylamine (23 mL, 170 mL) and $(Boc)_2O$ (26.4 g, 120 mmol) were added while stirring, and the mixture was stirred overnight at room temperature. The reaction solution was concentrated and extracted twice with water and ethyl acetate. The organic phase was dried and concentrated to afford a yellow oily liquid (12.7 g, 4%).

Step IV: Preparation of tert-butyl (3-tert-butyldimethylsiloxy-2-hydroxypropyl)carbamate(4)

The compound 3 (12.7 g, 66.5 mmol) was dissolved in dichloromethane (100 mL) and added with triethylamine (11.6 mL, 113.0 mmol) and imidazole (410 mg, 6.0 mmol), and in ice bath, TBSCI (11 g, 72.0 mmol) was added thereto while stirring. The resulting mixture was slowly warmed to room temperature and reacted overnight. The reaction solution was extracted twice by adding dichloromethane and a saline solution, the obtained organic phase was dried and concentrated to afford a pale yellow oily liquid. The liquid was purified by column chromatograph to afford the compound 4 (15 g, 75%)

Step V: Preparation of tert-butyl (3-tert-butyldimethylsiloxy-2-oxo-propyl)carbamate (5)

The compound 4 (120 g, 400 mmol) was dissolved in dichloromethane (1 L) and added with Des-Martin oxidant (250 g, 600 mmol) and $NaHCO_3$ (100 g, 1190 mmol) in an ice bath, and the mixture was raised to room temperature and reacted overnight. The reaction solution was subjected to suction filtration, and the mother liquor, by adding saturated $Na_2SO_3$ and dichloromethane, was extracted four times, to afford the organic phase. The organic phase was dried, concentrated, and subjected to column chromatography separation, to give a colorless oily liquid (50 g, 42%).

Step VI: Preparation of tert-butyl (2-(tert-butyldimethylsiloxymethyl)-3-fluoroallyl)carbamate (6)

The compound 2 (158 g, 0.416 mol) was dissolved in THF (200 mL), and then cooled to -20 °C. Under the protection of nitrogen gas, NaHMDS (208 mL, 0.417 mol, 2N in THF) was slowly dripped into the reaction solution in 30 min. The reaction solution was reacted for 30 min at -20 °C, then the compound 5 (42 g, 0.139 mol) was dissolved in THF (100 mL) and slowly dripped into the reaction solution. After the ice bath was removed, the reaction solution was subjected to natural warmup to room temperature and reacted for 2 hours. After the reaction was complete, the reaction solution was quenched by pouring into ice water. The resulting mixture was concentrated at low temperature to remove the organic solvents, and then extracted with ethyl acetate. The organic phases were combined, washed successively with water and saturated saline solution once, dried over anhydrous sodium sulfate, and concentrated. The crude product was purified by silica column chromatograph (petroleum:ethyl acetate=97:3) to give the product tert-butyl (2-(tert-butyldimethylsiloxymethyl)-3-fluoroallyl)carbamate (18g,41%).

Step VII: Preparation of tert-butyl (3-fluoro-2-hydroxymethylallyl)carbamate (7)

The compound 6 (18.0 g, 56.4 mmol) was dissolved in THF (200 mL) and added with TBAF (21.4 g, 67.7 mmol), and the mixture was reacted at room temperature for 3 hours. After the reaction was complete, water was added to quench the reaction, and the reaction solution was extracted with ethyl acetate. The organic phases were combined, then washed once with saturated saline solution, dried over magnesium sulfate, and concentrated. The crude product was directly used in the next step.

Step VIII: Preparation of tert-butyl (2-bromomethyl-3-fluoroallyl)carbamate (Int-1)

The compound 7 (56.4 mmol) and triethylamine (19.3 g, 169.3 mmol) were dissolved in acetone (200 ml). The mixture was cooled to 0 °C, then methane sulfonyl chloride (7.7 g, 67.7 mmol) was slowly dripped thereto (large quantity of solids were precipitated). After the addition, the resulting mixture was reacted for 2 hours at room temperature. After the completion of the reaction, filtration was conducted, and the filtrate was added with lithium bromide (24.5 g, 282.0 mmol) and reacted overnight at room temperature. When the reaction was complete, the solids were filtered. The filtrate was concentrated, and separated by adding water and ethyl acetate, the obtained aqueous phase was extracted, and the organic phases were combined. The organic phase, after being washed with water and statured saline solution, was dried with anhydrous sodium sulfate then concentrated. The crude product was purified by silica column chromatograph (petroleum ether: ethyl acetate=97: 3) to give the target product tert-butyl (2-bromomethyl-3-fluoroallyl)carbamate (6.0 g, 40%).

Example 1

Preparation of 4-((2-(aminomethyl)-3-fluoroallyl)oxy)-N-(naphthyl)benzoamide trifluoroacetate (TM8)

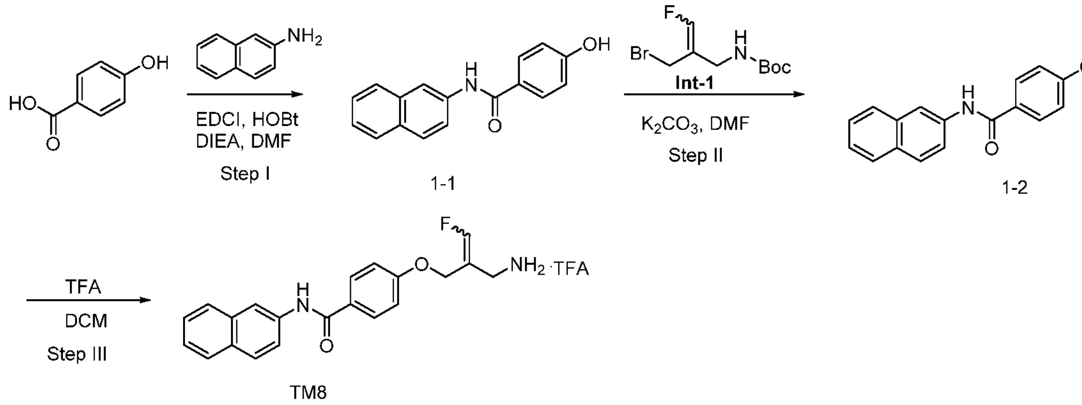


Step I: Preparation of N-naphthyl-4-hydroxybenzoamide (1-1)

Para-hydroxybenzoic acid (200 mg, 1.45 mmol) was dissolved in DMF (10 mL), and in an ice bath, HOBT (294 mg, 2.17 mmol), DIEA (374 mg, 2.90 mmol) and EDCI (417 mg, 2.17 mmol) were added in order and stirred for 30 min, and then, 2-naphthylamine (248 mg, 1.74 mmol) was added thereto. The mixture was raised to room temperature and stirred overnight. The reaction solution was quenched with water and extracted with ethyl acetate. The organic phases were combined. The organic phase was washed twice with saturated saline solution and dried over anhydrous magnesium sulfate. After filtration and concentration, the crude product was purified by column chromatograph to give white solids (240 mg, 65%).
MS m/z(ESI): 264 $[M+H]^+$

Step II: Preparation of tert-butyl (2-(4-(naphthyl-2-carbamoyl)phenoxy)methyl-3-fluoroallyl)amino formate (1-2)

The compound 1-1 (240 mg, 0.91 mmol) was dissolved in DMF (10 mL) and added with the Int-1 (293 mg, 1.09 mmol) and potassium carbonate (450 mg, 3.27 mmol), the mixture was stirred overnight at room temperature. Then water was added to quench the reaction. The reaction solution was extracted with ethyl acetate, the organic phases were combined. Then the organic phase was washed with saturated saline solution three times, dried over magnesium sulfate, fitered, concentrated, and purified by preparative TLC to give the target product (206 mg, 50 %).
MS m/z(ESI): 451 $[M+H]^+$

Step III: Preparation of N-naphthyl-4-(2-aminomethyl-3-fluoroallyloxy)-benzamide trifluoroacetate (TM8)

The compound 1-2 (206 mg, 0.5mmol) was dissolved in dichloromethane (6 mL) and then added with TFA (2 mL), the mixture was stirred at room temperature for 30 min. After LC-MS monitored the completion of the reaction, the reaction mixture was concentrated under vacuum and then lyophilized to afford the target product TM8 (60 mg, 39%).
MS m/z (ESI): 351 $[M+H]^+$
$^1$HNMR (400 MHz, DMSO-$d_6$) δ: 10.31 (s, 1H), 8.44 (s, 1H), 8.07-7.99 (m, 4H), 7.89-7.82 (m, 4H), 7.49-7.42 (m, 2H), 7.18-7.14 (m, 2H), 4.82 (s, 2H), 4.70 (d, $J$ = 2.8 Hz, 2H), 3.66 (s, 2H), 3.58 (d, $J$ = 2.0 Hz, 2H).

According to general empirical analyses, it was deduced that the chemical shifts 4.82, 3.58 may be the characteristic peaks of the Z-configuration, and the chemical shifts 4.70, 3.66 may be the characteristics peaks of the E-configuration.

Example 2: Preparation of N-methyl-N-p-methylphenyl-4-((2-aminomethyl-3-fluoroallyl)oxy)-benzamide trifluoroacetate (TM67)

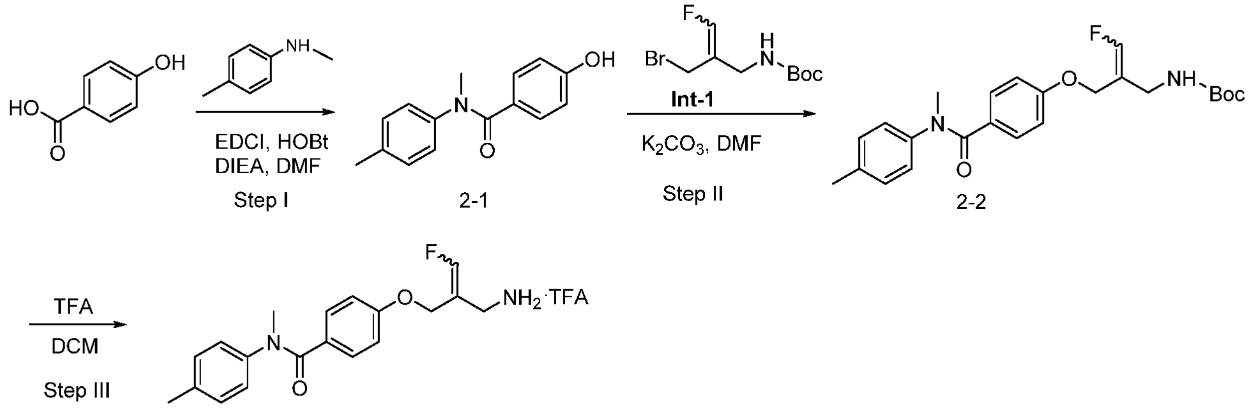


TM67

Except for that N-methyl-p-methylaniline in Step I of this example replaced the 2-naphthylamine in Step I of Example 1, the title compound was prepared according to a similar method to that described in Example 1, with the total yield of 19%.
MS m/z (ESI): 329 [M+H]+
$^1$HNMR (400 MHz, DMSO-$d_6$) δ: 7.97 (s, 2H), 7.38-7.18 (m, 3H), 7.09-7.01 (m, 4H), 6.84-6.82 (m, 2H), 4.64 (d, *J* = 2.0 Hz, 2H), 4.52 (d, *J* = 3.6 Hz, 2H), 3.57 (d, *J* = 7.6 Hz, 2H), 3.51 (s, 2H), 3.32 (s, 3H), 2.23 (s, 3H).

According to general empirical analyses, it was deduced that the chemical shifts 4.64, 3.51 may be the characteristic peaks of the Z-configuration, and the chemical shifts 4.52, 3.57 may be the characteristics peaks of the E-configuration.

Example 3: Preparation of N-(4-fluorophenyl)-4-((2-aminomethyl-3-fluoroallyl)oxy)-benzamide trifluoroacetate (TM61)

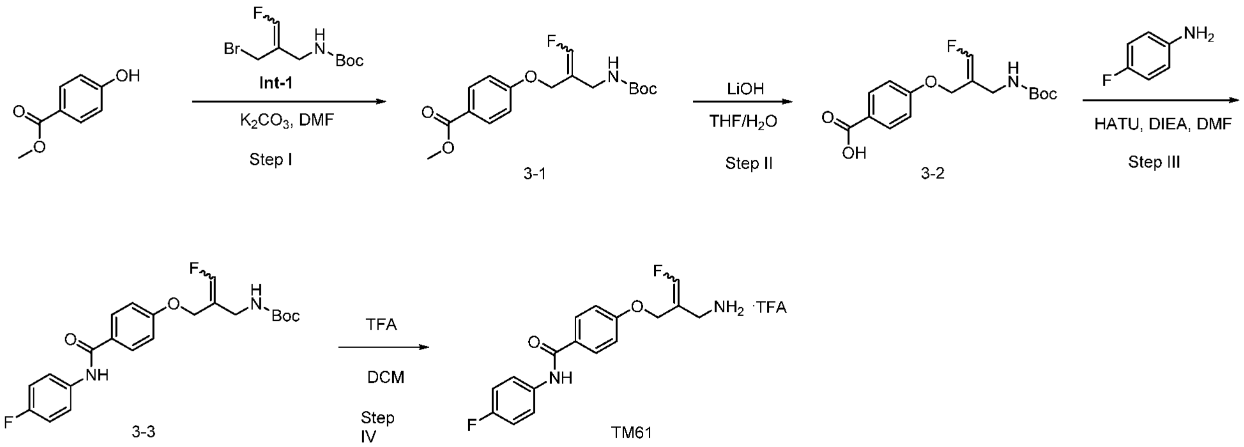


Step I: Preparation of methyl 4-((2-(tert-butoxycarbonylaminomethyl)-3-fluoroallyl)oxy)benzoate (3-1)

Except for that methyl hydroxybenzoate in this step replaced the intermediate 1-1 in Step II of Example 1, the title compound was prepared according to a similar method to that described in Step II of Example 1. The resultant crude product was directly applied in the next step.
MS m/z (ESI): 340 [M+H]+

Step II: Preparation 4-(2-(tert-butoxycarbonylaminomethyl)-3-fluoroallyloxy)benzoic acid (3-2)

The compound 3-1 (2.71 g, 7.99 mmol) was dissolved in tetrahydrofuran (18 mL) and water (9 mL) and added with lithium hydroxide (2.02 g, 48.08 mmol), the mixture was reacted overnight at 45 °C. The reaction solution was adjusted with diluted hydrochloric acid aqueous solution to pH = 2-3, then extracted twice with ethyl acetate, washed with saturated saline solution and dried over anhydrous sodium sulfate, followed by concentration under vacuum, to

afford the product (1.78 g, 69%).
MS m/z (ESI): 326 $[M+H]^+$

Step III: Preparation tert-butyl (2-(4-(4-fluorophenylaminoformyl)phenoxy)methyl-3-fluoroallyl)carbamate (3-3)

The compound 3-2 (150 mg, 0.44 mmol) was dissolved in DMF (4 mL) and added with DIEA (114 mg, 0.88 mmol) and HATU (252 mg, 0.66 mmol), the mixture was stirred for half an hour in ice bath under the protection of nitrogen gas. Then para-fluoroaniline (49 mg, 0.44 mmol) was added at the same temperature. After 15 minute, the ice bath was removed, and the mixture was allowed to react for 2 hours at room temperature. After the completion of the reaction, the mixture was quenched by adding saturated sodium bicarbonate, and the resulting mixture was extracted twice with ethyl acetate. The organic phase was collected and washed twice with saturated saline solution, dried over anhydrous sodium sulfate and concentrated in vacuum, then purified by preparative TLC to afford the product (168 mg, 87%).
MS m/z (ESI): 419 $[M+H]^+$

Step IV: Preparation of N-(4-fluorophenyl)-4-((2-aminomethyl-3-fluoroallyl)oxy)-benzamide trifluoroacetate (TM61)

Except for that the intermediate 3-3 in this step replaced the intermediate 1-2 in Step III of Example 1, the title compound was prepared according to a similar method to that described in Step III of Example 1, with the yield of about 100%.
MS m/z (ESI): 319 $[M+H]^+$
$^1$HNMR (400 MHz, $CD_3OD$) $\delta$: 7.97-7.94 (m, 2H), 7.68-7.65 (m, 2H), 7.35-7.02 (m, 5H), 4.89 (d, *J* = 2.4 Hz, 2H), 4.69 (d, *J* = 3.2 Hz, 2H), 3.83 (d, *J* = 2.0 Hz, 2H), 3.71 (d, *J* = 2.4 Hz, 2H,).
According to general empirical analyses, it was deduced that the chemical shifts 4.89, 3.71 may be the characteristic peaks of the Z-configuration, and the chemical shifts 4.69, 3.83 may be the characteristics peaks of the E-configuration.

Example 4: Preparation of (4-(2-aminomethyl-3-fluoroallyloxy)phenyl)-(6-trifluoromethyl-3,4-dihydroisoquinol-2(1H)-yl)-methanone trifluoroacetate (TM68)

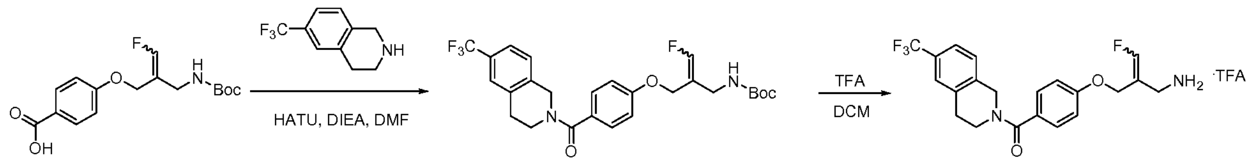


3-2 Step I 4-1 Step II TM68

Except for that 1,2,3,4-tetrahydro-6-trifluoromethylisoquinoline in the first step of the example replaced the para-fluoroaniline in Step III of Example 3, the title compound was prepared according to processes similar to those described in Steps III and IV of Example 3, with the total yield of 16%.
MS m/z (ESI): 409 $[M+H]^+$
$^1$HNMR (400 MHz, $CD_3OD$) $\delta$: 7.76-7.47 (m, 4H), 7.40-6.92 (m, 4H), 5.06-4.67 (m, 2H), 4.87 (d, *J* = 2.4 Hz, 2H), 4.67 (d, J= 3.2 Hz, 2H), 3.83 (d, *J* = 1.6 Hz, 2H), 3.83-3.70 (m, 2H), 3.70 (d, *J* = 2.4 Hz, 2H), 3.01 (s, 2H).
According to general empirical analyses, it was deduced that the chemical shifts 4.87, 3.70 may be the characteristic peaks of the Z-configuration, and the chemical shifts 4.67, 3.83 may be the characteristics peaks of the E-configuration.

Example 5: Preparation of N-(3-dimethylaminophenyl)-4-(2-aminomethyl-3-fluoroallyloxy)-benzamide trifluoroacetate (TM62)

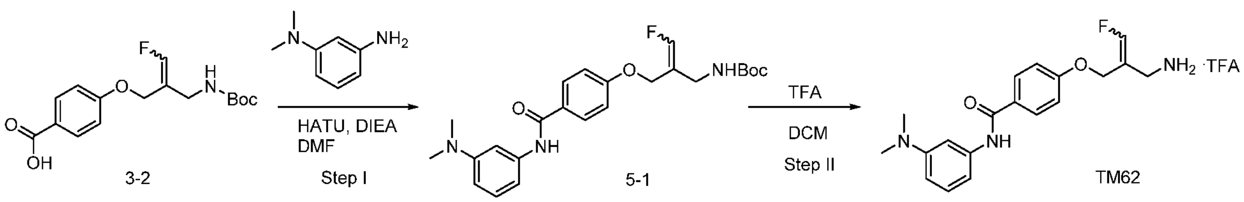


Except for that N,N-dimethyl-m-phenylenediamine in Step I of the example replaced the para-fluoroaniline in Step III of Example 3, the title compound was prepared according to processes similar to those described in Steps III and IV of Example 3, with the total yield of 49%.
MS m/z (ESI): 344 $[M+H]^+$
$^1$HNMR (400 MHz, $CD_3OD$) $\delta$: 8.04-7.96 (m, 3H), 7.54-7.45 (m, 2H), 7.36-7.00 (m, 4H), 4.90 (d, $J$ = 2.4 Hz, 2H), 4.70 (d, $J$ = 3.2 Hz, 2H), 3.84 (d, $J$ = 1.6 Hz, 2H), 3.71 (d, $J$ = 1.6 Hz, 2H), 3.23 (s, 6H).

According to general empirical analyses, it was deduced that the chemical shifts 4.90, 3.71 may be the characteristic peaks of the Z-configuration, and the chemical shifts 4.70, 3.84 may be the characteristics peaks of the E-configuration.

Example 6: Preparation of (S)-(4-(2-aminomethyl)-3-fluoroallyloxy)phenyl)-(1-phenyl-3,4-dihydroisoquinol-2(1H)-yl)-methanone trifluoroacetate (TM69)

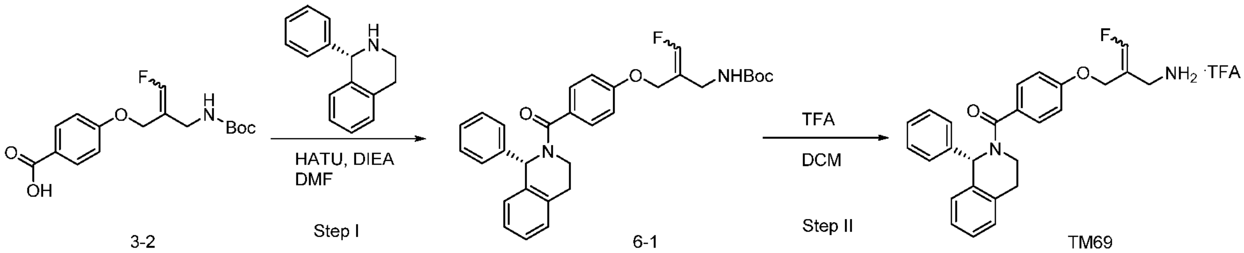


Except for that (S)-1-phenyl-1,2,3,4-tetrahydroisoquinoline in Step I of the example replaced the para-fluoroaniline in Step III of Example 3, the title compound was prepared according to processes similar to those described in Steps III and IV of Example 3, with the total yield of 52%.
MS m/z (ESI): 417 $[M+H]^+$
$^1$HNMR (400 MHz, $CD_3OD$) $\delta$: 7.38 (dd, $J$ = 8.8 Hz, 2.4 Hz, 2H), 7.33-7.20 (m, 8H), 7.13-7.00 (m, 4H), 4.86 (d, $J$ = 2.8 Hz, 2H), 4.65 (d, $J$ = 3.2 Hz, 2H), 3.82 (d, $J$ = 1.2 Hz, 2H), 3.75 (br, 1H), 3.69 (d, $J$ = 2.4 Hz, 2H), 3.40 (m, 1H), 3.08-3.00 (m, 1H), 2.85-2.76 (m, 1H).

According to general empirical analyses, it was deduced that the chemical shifts 4.86, 3.69 may be the characteristic peaks of the Z-configuration, and the chemical shifts 4.65, 3.82 may be the characteristics peaks of the E-configuration.

Example 7: Preparation of 2-(4-(4-methylthiazolyl-2-phenoxy)methyl)-3-fluoroallylamine trifluoroacetate (TM70)

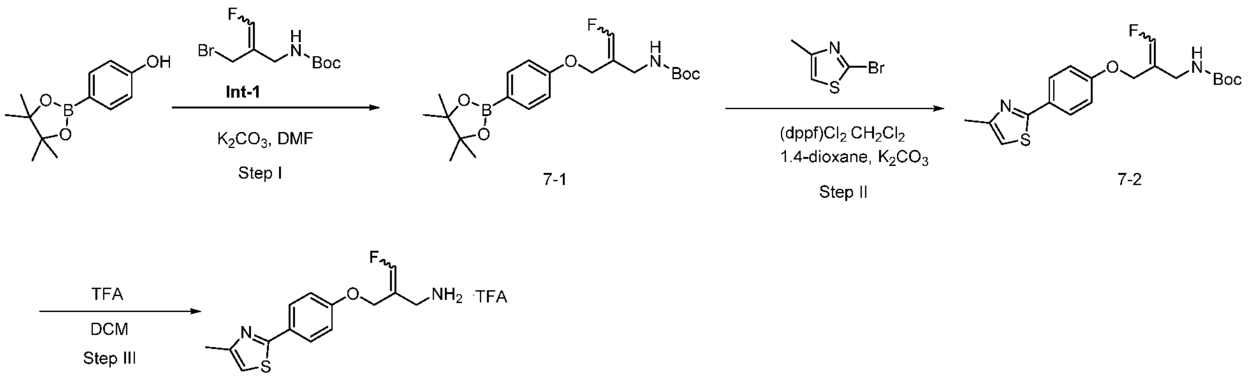


TM70

Step I: Preparation of tert-butyl (3-fluoro-2-(4-(4,4,5,5-tetramethyl-1,3,2-dioxaborolan-2-yl) phenoxymethyl)allyl)carbamate (7-1)

Except for that 4-hydroxyphenylboronic acid pinacol ester in this step replaced the intermediate 1-1 in Step II of Example 1, the title compound was prepared according to a similar method to that described in Step II of Example 1, with the yield of 83%.
MS m/z(ESI): 408 $[M+H]^+$

Step II: Preparation of tert-butyl (3-fluoro-2-(4-(4-methylthiazolyl-2-phenoxy)methyl)allyl)carbamate (7-2)

The compound 7-1 (100 mg, 0.25 mmol) was dissolved in 1,4-dioxane (3 mL), and added with 2-bromo-4-methylthiazole (52 mg, 0.30 mmol), [1,1'-bis(diphenylphosphine)ferrocene]palladium dichloride dichloromethane complex (20 mg, 0.03 mmol) and 1mmol/L potassium carbonate aqueous solution (1.3 mL), and the mixture was reacted overnight at 90 °C under the protection of nitrogen gas. After the completion of the reaction, the mixture was quenched by adding water. The aqueous phase was extracted twice by ethyl acetate, the organic phase was collected. The organic phase was washed with saturated saline solution, dried over anhydrous sodium sulfate and concentrated in vacuum. The crude product was purified by TLC to afford the target product (62 mg, 67%).
MS m/z (ESI): 379 $[M+H]^+$

Step III: Preparation of 2-(4-(4-methylthiazolyl-2-phenoxy)methyl)-3-fluoroallylamine trifluoroacetate (TM70)

Except for that the intermediate 7-2 in this step replaced the intermediate 1-2 in Step III of Example 1, the title compound was prepared through a similar method to that described in Step III of Example 1, with the yield of about 100%.
MS m/z (ESI): 279 $[M+H]^+$
$^1$HNMR (400 MHz, $CD_3OD$) δ: 7.89 (dd, *J* = 8.8 Hz, 2.4 Hz, 2H), 7.24 (d, *J* = 81.2 Hz, 1H), 7.12 (d, *J* = 80.8 Hz, 1H), 7.14-7.11 (m, 3H), 4.88 (d, *J* = 2.4 Hz, 2H), 4.68 (d, *J* = 3.2 Hz, 2H), 3.84 (d, *J* = 2.6 Hz, 2H), 3.70 (d, *J* = 2.0 Hz, 2H), 2.47 (s, 3H).
According to general empirical analyses, it was deduced that the chemical shifts 7.12, 4.88, 3.70 may be the characteristic peaks of the Z-configuration, and the chemical shifts 7.24, 4.68, 3.84 may be the characteristics peaks of the E-configuration.

Example 8: Preparation of (4-(2-aminomethyl-3-fluoroallyloxy)phenyl)-(5-chloroisoindolin-2-yl)-methanone trifluoroacetate (TM71)

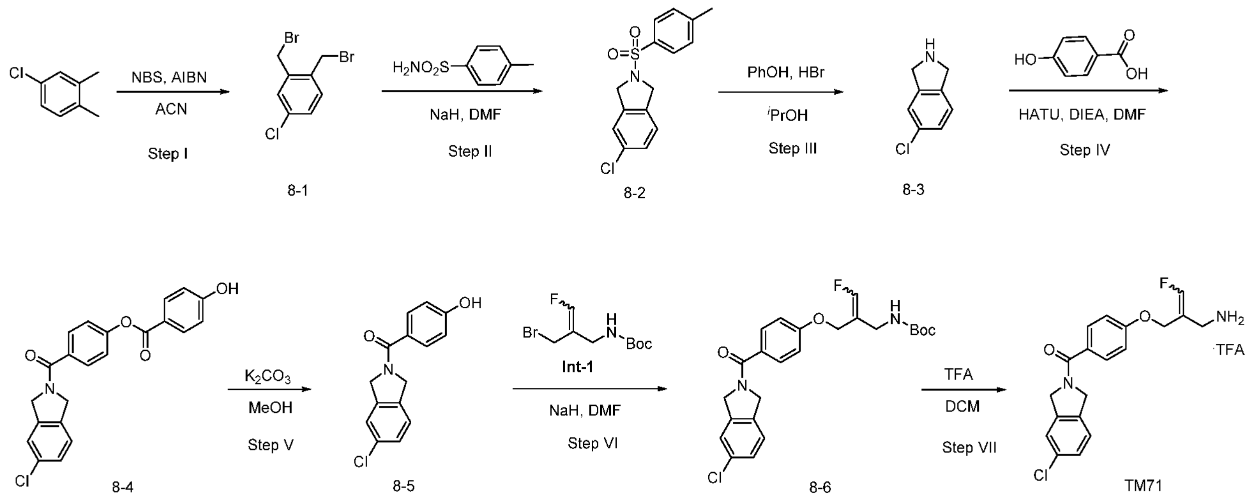


Step I: Preparation of 1,2-di(bromomethyl)-4-chlorobenzene (8-1)

The compound 4-chloro-1,2-dimethylbenzene (0.2 mL, 1.5 mmol) was dissolved in acetonitrile (26 mL) and then added with NBS (0.55 g, 3.0 mmol) and AIBN (0.14 g, 0.8 mmol), and the mixture was reacted at 75 °C for 2 hours. The reaction was quenched by adding water, and the mixture was extracted twice with ethyl acetate. The organic phase was washed with saturated saline solution, and dried over anhydrous sodium sulfate and concentrated in vacuum. The crude product was subjected to column chromatograph to afford the target product (0.20 g, 47%).

Step II: Preparation of 5-chloro-2-p-tosylisoindoline (8-2)

Sodium hydride (1.60 g, 40.00 mmol) was dissolved in DMF (20 mL) and then dripped with a solution of 4-methylbenzene sulfonamide(2.64 g, 15.40 mmol) in DMF (10 mL), the mixture was reacted at 90 °C for 2 hours. A solution of the compound 8-1 (2.00 g, 6.76 mmol) in DMF (10 mL) was dripped, the resulting mixture was reacted for 4 hours at 90 °C. The reaction solution was poured into ice water, the mixture was subjected to stirring and suction filtration. The filter cake was washed with diluted hydrochloric acid and dissolved with ethyl acetate, then washed with 5% sodium carbonate solution. The organic phase was collected and washed with saturated saline solution, and dried over anhydrous sodium sulfate and concentrated in vacuum to afford the target product (1.40 g, 68%).
MS m/z (ESI): 308 $[M+H]^+$

Step III: Preparation of 5-chloroisoindoline (8-3)

The compound 8-2 (1 g, 3.26 mmol), phenol (1 g, 10.60 mmol), hydrobromic acid (8 mL) and isopropanol (1.4 mL) were reacted at 126 °C for 4 hours. The reaction was quenched by adding water, the mixture was extracted three times with ethyl acetate. The aqueous phase was regulated to pH = 8-9 by diluted sodium hydroxide aqueous solution, and extracted twice with ethyl acetate. The combined organic phase was washed with saturated saline solution, dried over anhydrous sodium sulfate and concentrated in vacuum. The crude product was added with HCl/1,4-dioxane solution, then subjected to stirring, and followed by concentrated in vacuum to afford the target product (489 mg, 98%).
MS m/z (ESI): 155[M+H]+

Step IV: Preparation of 4-(5-chloroisoindoline-2-carbonyl)phenyl-4-hydroxybenzoate (8-4)

Para-hydroxy benzoic acid (270 mg, 1.95 mmol) was dissolved in DMF (6 mL), and in ice bath successively added with DIEA (503 mg, 3.90 mmol) and HATU (1.11 g, 2.93 mmol), the mixture was reacted for 1 hour under the protection of nitrogen gas. Then followed by an addition of the solution of the compound 8-3 (300 mg, 1.95 mmol) in DMF (2 mL), the mixture was reacted for 15 min in ice bath under the protection of nitrogen gas, then warmed to room temperature and reacted overnight. After the completion of the reaction, the mixture was quenched with sodium bicarbonate aqueous solution, the aqueous phase was extracted with ethyl acetate. The organic phase was combined, washed with water, dried and concentrated to afford the crude product (400 mg).
MS m/z (ESI): 394 $[M+H]^+$

Step V: Preparation of (5-chloroisoindolin-2-yl)(4-hydroxyphenyl)methanone (8-5)

The compound 8-4 (300 mg, 0.76 mmol) was dissolved in methanol (8 mL) and added with potassium carbonate solids (300 mg, 2.17 mmol) and water (2 mL), the mixture was reacted at room temperature for 4 hours. After the completion of the reaction, the reaction solution was diluted with water and regulated with diluted hydrochloric acid to pH=3-4. The aqueous solution was extracted twice with dichloromethane. The organic phase was washed successively with water and saturated saline solution, dried over anhydrous sodium sulfate and concentrated, then purified with preparative TLC, to afford the target product (94 mg, 45%).
MS m/z (ESI): 274 $[M+H]^+$

Step VI: Preparation of tert-butyl (2-(4-(5-chloroisoindolin-2-carbonyl)phenoxymethyl)-3-fluoroallyl)carbamate (8-6)

Except for that the intermediate 8-5 in this step replaced the intermediate 1-1 in step II in Example 1, the title compound was prepared according to a similar method to that described in Step II of Example 1, with the yield of 90%.
MS m/z (ESI): 461 $[M+H]^+$

Step VII: Preparation of (4-(2-aminomethyl-3-fluoroallyloxy)phenyl)-(5-chloroisoindolin-2-yl)-methanone trifluoroacetate (TM71)

Except for that the intermediate 8-6 in this step replaced the intermediate 1-2 in step III in Example 1, the title compound was prepared according to a similar method to that described in Step III of Example 1, with the yield of 72%.
MS m/z (ESI): 361 $[M+H]^+$
$^1$HNMR (400 MHz, DMSO-$d_6$) δ: 7.79 (br, 2H), 7.62 (d, *J* = 8.0 Hz, 2H), 7.50-7.24 (m, 4H), 7.11-7.06 (m, 2H), 4.85-4.78 (m, 4H), 4.80 (s, 2H)), 4.65 (d, *J* = 3.2 Hz, 2H), 3.64 (s, 2H), 3.55 (s, 2H).

According to general empirical analyses, it was deduced that the chemical shifts 4.80, 3.55 may be the characteristic peaks of the Z-configuration, and the chemical shifts 4.65, 3.64 may be the characteristics peaks of the E-configuration.

Example 9: Preparation of N-phenyl-4-((2-aminomethyl-3-fluoroallyl)oxy)-benzamide trifluoroacetate (TM1) and resolution of the isomers thereof

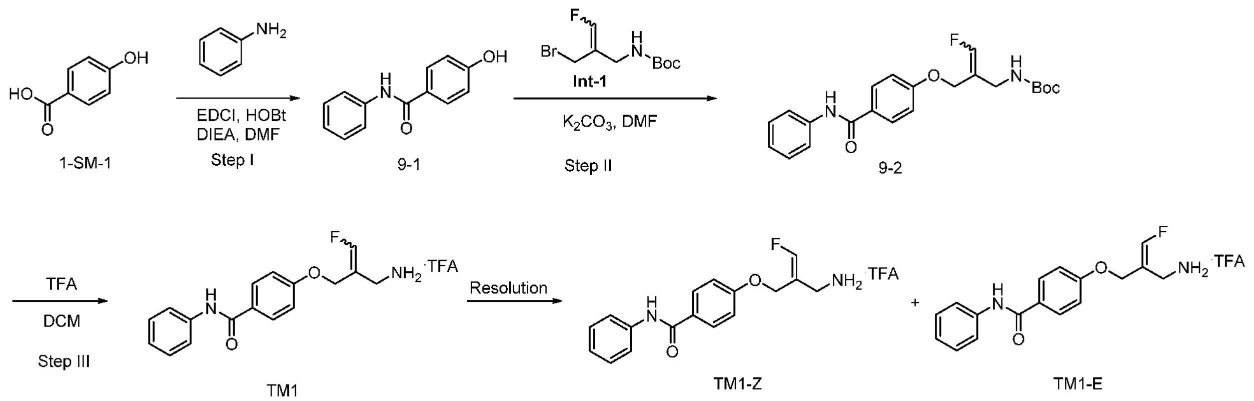


Except for that in Step I of the example, aniline replaced the 2-naphthylamine in Step I of Example 1, the title compound was prepared according to a similar method to that described in Example 1, with the total yield of 35%.

Method for resolution of N-phenyl-4-((2-aminomethyl-3-fluoroallyl)oxy)-benzamide trifluoroacetate isomers
Apparatus: Agilent 1260, Sunfire C18; Mobile phase: A: 0.1% TFA, B: ACN;
Elution gradient: 0-8 min: 20%-30% B, flow rate: 16 mL/min; 8.1-10 min: 95% B, flow rate: 20 mL/min; 10.1-12 min: 20% B, flow rate: 20 mL/min.

The eluent liquid having a retention time from 5.6 to 6.7 min was collected and lyophilized to give the TM1-E, and the eluent liquid having a retention time from 7.0 to 8.0 min was collected and lyophilized to give the TM1-Z.

(Z)-N-phenyl-4-((2-aminomethyl-3-fluoroallyl)oxy)-benzamide trifluoroacetate (TM1-Z)

MS m/z (ESI): 301 $[M+H]^+$
$^1$HNMR (400 MHz, $CD_3OD$) δ: 7.97 (d, *J* = 8.8 Hz, 2H), 7.66 (dd, *J* = 7.6 Hz, 2H), 7.37-7.33 (m, 2H), 7.16-7.12 (m, 3H), 7.12 (d, *J* = 80.4 Hz, 1H), 4.90 (d, *J* = 2.0 Hz, 2H), 3.71 (d, *J* = 2.4 Hz, 2H).

(E)-N-phenyl-4-((2-aminomethyl-3-fluoroallyl)oxy)-benzamide trifluoroacetate (TM1-E).

MS m/z (ESI): 301$[M+H]^+$
$^1$HNMR (400 MHz, $CD_3OD$) δ: 7.97 (d, *J* = 9.2 Hz, 2H), 7.66 (dd, *J* = 7.6 Hz, 2H), 7.37-7.33 (m, 2H), 7.33-7.11 (m, 4H), 4.70 (d, *J* = 2.8 Hz, 2H), 3.84 (d, *J* = 2.0 Hz, 2H).

Example 10: Preparation of (S)-N-(1,2,3,4-tetrahydro-1-naphthyl)-4-((2-aminomethyl-3-fluoroallyl)oxy)-benzamide trifluoroacetate (TM63) and resolution of the isomers thereof

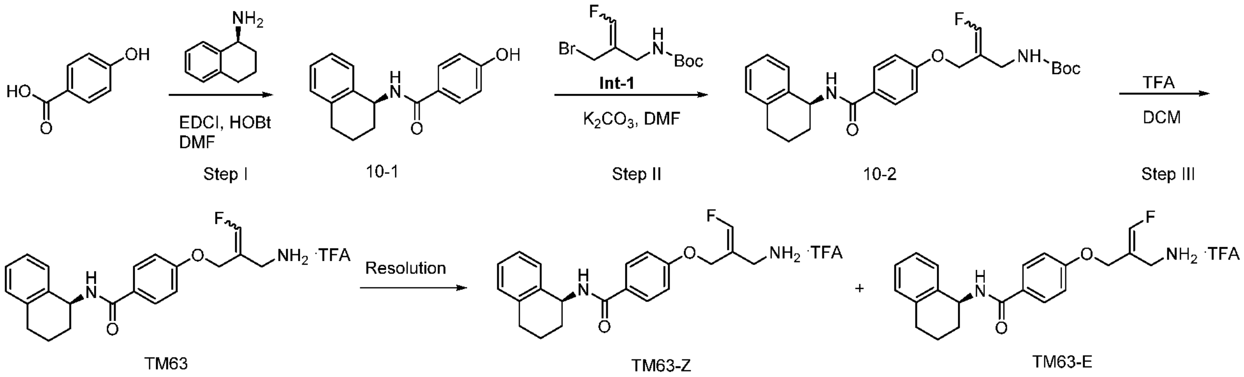


Except for that in Step I of the example, (S)-1,2,3,4-tetrahydro-1-naphthylamine replaced 2-naphthylamine in Step I of Example 1, the title compound was prepared according to a similar method to that described in Example 1, with the total yield of 46%.

A method similar to that as described in Example 9 is applied to resolute the isomers, and the retention time of the TM63-E is earlier than that of the TM63-Z.

(Z)-(S)-N-(1,2,3,4-tetrahydro-1-naphthyl)-4-((2-aminomethyl-3-fluoroallyl)oxy)-benzamide trifluoroacetate (TM63-Z).

MS m/z (ESI): 355 $[M+H]^+$
$^1$HNMR (400 MHz, $CD_3OD$) δ: 7.80 (d, *J* = 8.8 Hz, 2H), 7.30-7.23 (m, 4H), 7.10 (d, *J* = 8.8 Hz, 2H), 7.05 (d, *J* = 80.4 Hz, 1 H), 5.20 (t, *J* = 5.6 Hz, 1H), 4.92 (d, *J* = 1.6 Hz, 2H), 3.75 (s, 2H), 2.89-2.72 (m, 2H), 2.14-1.86 (m, 4H).

(E)-(S)-N-(1,2,3,4-tetrahydro-1-naphthyl)-4-((2-aminomethyl-3-fluoroallyl)oxy)-benzamide trifluoroacetate (TM63-E).

MS m/z (ESI): 355 $[M+H]^+$
$^1$HNMR (400 MHz, $CD_3OD$) δ: 7.80 (dd, *J* = 22.8 Hz, 8.8 Hz, 2H), 7.30-7.23 (m, 4H), 7.10-7.05 (m, 2H), 5.20 (t, *J* = 6.0 Hz, 1H), 4.70 (d, *J* = 3.2 Hz, 2H), 3.90 (s, 2H), 2.91-2.72 (m, 2H), 2.14-1.84 (m, 4H).

Example 11: Preparation of (4-((2-aminomethyl-3-fluoroallyl)oxy)-3-fluorophenyl)-(4-phenylpiperidin-1-yl)methanone trifluoroacetate (TM48)

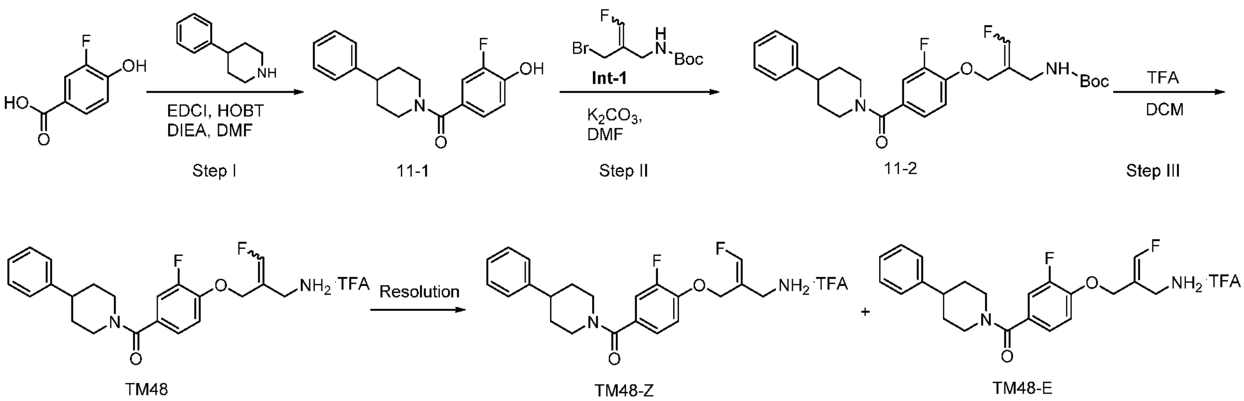


Except for that in Step I of the example, 4-phenylpiperidine replaced 2-naphthylamine in Example 1, the title compound was prepared according to a similar method to that described in Example 1, with the total yield of 57%.

A method similar to that as described in Example 9 is applied to resolute the isomers, the retention time of the TM48-E is earlier than that of the TM48-Z.

(Z)-(4-((2-(aminomethyl)-3-fluoroallyl)oxy)-3-fluorophenyl)-(4-phenylpiperidin-1-yl)methanone trifluoroacetate (TM48-Z).

MS m/z (ESI): 387 $[M+H]^+$
$^1$HNMR (400 MHz, $CD_3OD$) $\delta$: 7.48-7.02 (m, 9H), 4.93 (d, *J* = 2.4 Hz, 2H), 4.78-4.48 (m, 1H), 3.91-3.82 (m, 1H), 3.72 (d, *J* = 2.0 Hz , 2H), 3.29-3.23 (m, 1H), 2.98-2.93 (m, 1H), 2.87-2.83 (m, 1H), 1.93-1.71 (m, 4H).

(E)-(4-((2-(aminomethyl)-3-fluoroallyl)oxy)-3-fluorophenyl)-(4-phenylpiperidin-1-yl)methanone trifluoroacetate (TM48-E).

MS m/z (ESI): 387 $[M+H]^+$
$^1$HNMR (400 MHz, $CD_3OD$) $\delta$: 7.34-7.14 (m, 9H), 4.78-4.73 (m, 1H), 4.73 (d, J=3.2 Hz, 2H), 3.90-3.84 (m, 1H), 3.84 (s, 2H), 3.29-3.23 (m, 1H), 2.98-2.93 (m, 1H), 2.87-2.83 (m, 1H), 1.93-1.71 (m, 4H).

Example 12: Preparation of (4-((2-aminomethyl-3-fluoroallyl)oxy)-3-fluorophenyl)-(3,4-tetrahydroisoquinoline-2(1H)-yl)methanone trifluoroacetate (TM72)

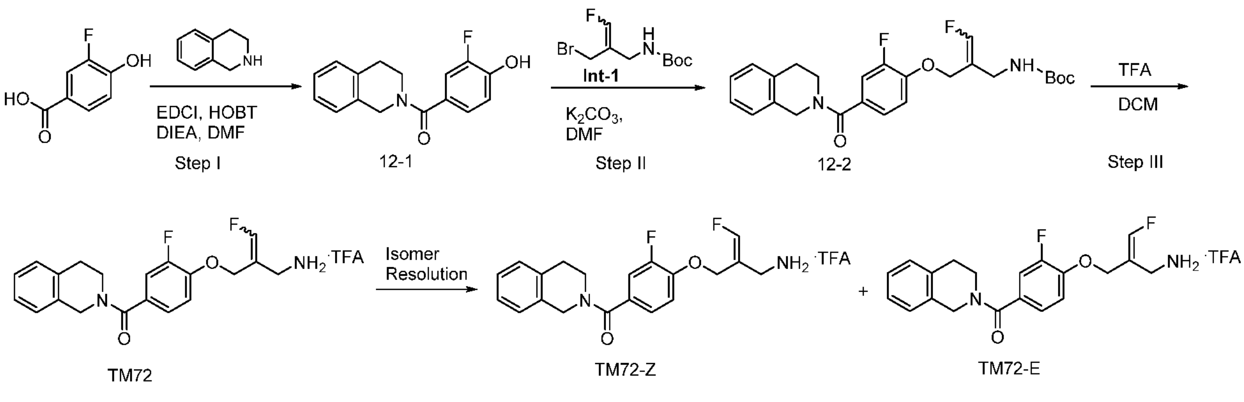


Except for that in Step I of the example, tetrahydroisoquinoline replaced 2-naphthylamine in Step I of Example 1, the title compound was prepared according to a similar method to that described in Example 1, with the total yield of 31%.

A method similar to that as described in Example 9 is applied to resolute the isomers, the retention time of the TM72-E is earlier than that of the TM27-Z.

(Z)-(4-((2-aminomethyl-3-fluoroallyl)oxy)-3-fluorophenyl)-(3,4-tetrahydroisoquinoline-2(1H)-yl)methanone trifluoroacetate (TM72-Z).

MS m/z (ESI): 359 $[M+H]^+$
$^1$HNMR (400 MHz, $CD_3OD$) δ: 7.41-6.94 (m, 8H), 4.94 (d, *J* = 2.4 Hz, 2H), 4.85-4.75 (m, 1H), 4.70-4.65 (m, 1H), 3.95-3.93 (m, 1H), 3.72 (s, 2 H), 3.72-3.70 (m, 1H), 2.95-2.93 (m, 2 H).

(E)-(4-((2-aminomethyl-3-fluoroallyl)oxy)-3-fluorophenyl)-(3,4-tetrahydroisoquinoline-2(1H)-yl)methanone trifluoroacetate (TM72-E).

MS m/z (ESI): 359 $[M+H]^+$
$^1$HNMR (400 MHz, $CD_3OD$) δ: 7.39-6.94 (m, 8H), 4.85-4.82 (m, 1H), 4.75 (d, *J* = 3.2 Hz, 2H), 4.68-4.65 (m, 1H), 3.95-3.93 (m, 1H), 3.84 (s, 2H), 3.72-3.69 (m, 1H), 2.99-2.93 (m, 2H).

Example 13: Preparation of (4-((2-aminomethyl-3-fluoroallyl)oxy)-3-fluorophenyl)-(5-chloro-3,4-dihydroisoquinoline-2(1H)-yl)methanone trifluoroacetate (TM73)

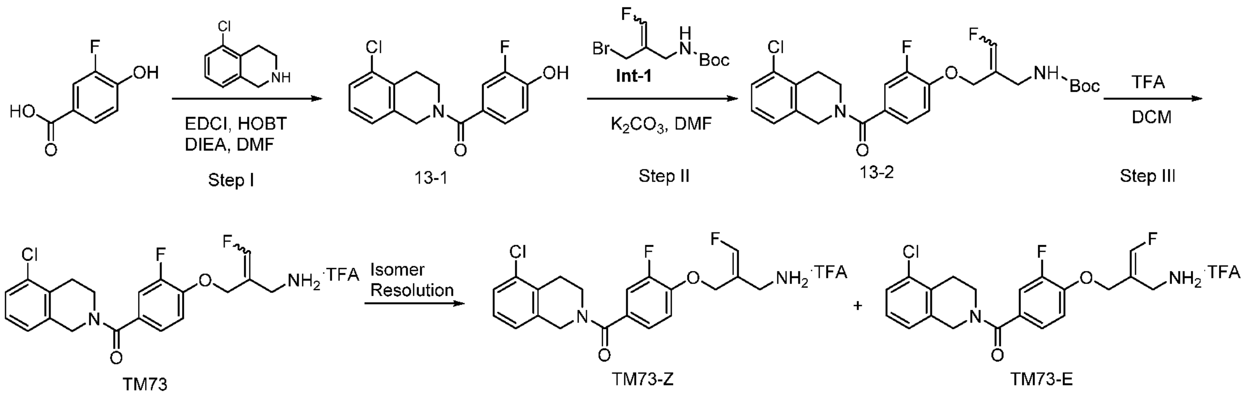


Except for that in Step I of the example, 5-chlorotetrahydroisoquinoline replaced the 2-naphthylamine in Step I of Example 1, the title compound was prepared according to a similar method to that described in Example 1, with the total yield of 32%.

A method similar to that as described in Example 9 is applied to resolute the isomers, the retention time of the TM73-E is earlier than that of the TM73-Z.

(Z)-(4-((2-aminomethyl-3-fluoroallyl)oxy)-3-fluorophenyl)-(5-chloro-3,4-dihydroisoquinoline-2(1H)-yl)methanone trifluoroacetate (TM73-Z).

MS m/z (ESI): 393 $[M+H]^+$
$^1$HNMR (400 MHz, $CD_3OD$) δ: 7.35-7.20 (m, 6H), 7.13 (d, *J* = 80.0 Hz, 1H), 4.95 (d, *J* = 2.4 Hz, 2H), 4.81-4.64 (m, 2H), 4.13-3.79 (m, 2H), 3.73 (d, *J* = 2.0 Hz, 2H), 3.00-2.92 (m, 2H).

(E)-(4-((2-aminomethyl-3-fluoroallyl)oxy)-3-fluorophenyl)-(5-chloro-3,4-dihydroisoquinoline-2(1H)-yl)methanone trifluoroacetate (TM73-E).

MS m/z (ESI): 393 $[M+H]^+$
$^1$HNMR (400 MHz, $CD_3OD$) δ: 7.44-7.15 (m, 7H), 4.75 (d, *J* = 3.2 Hz, 2H), 4.85-4.62 (m, 2H), 4.11-3.65 (m, 2H), 3.85 (s, 2H), 3.01-2.89 (m, 2H).

Example 14: Preparation of N-(4-fluorophenyl)-4-((2-aminomethyl-3-fluoroallyl)oxy)-3-chloro-benzamide trifluoroacetate (TM65)

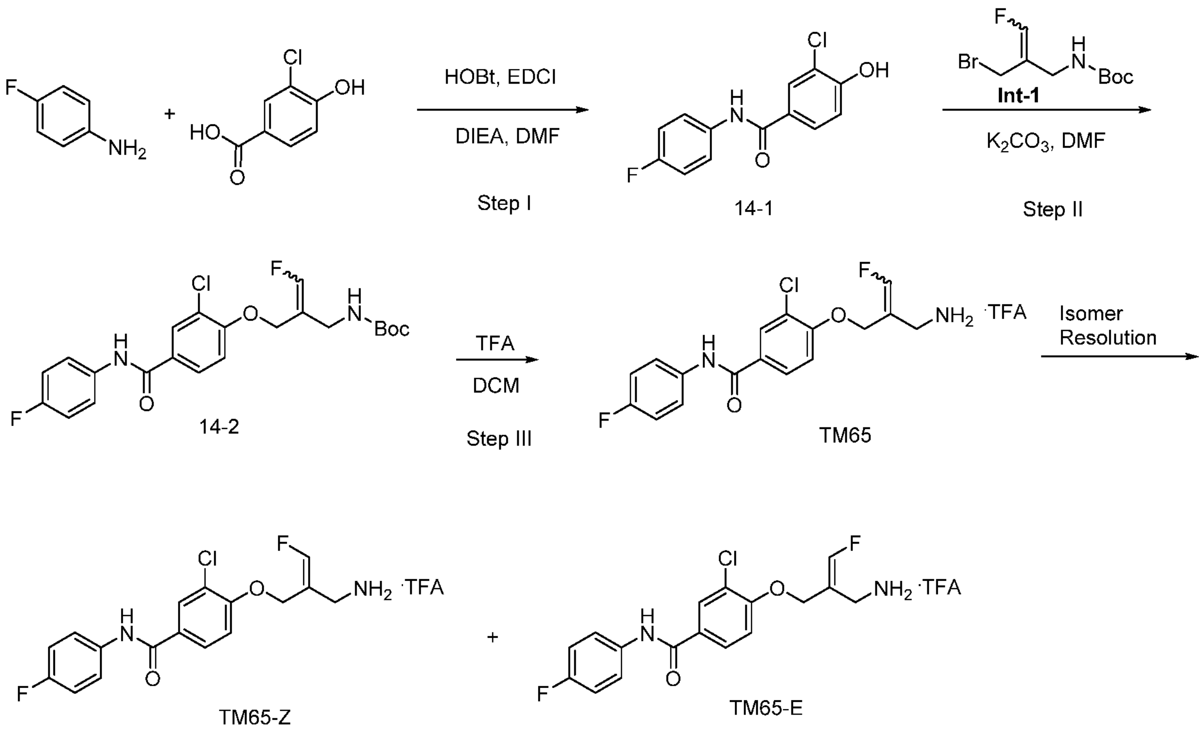


Except for that in Step I of the example, para-fluoroaniline replaced the 2-naphthylamine in Step I of Example 1, and 3-chloro-4-hydroxybenzoic acid replaced the para-hydroxybenzoic acid in Step I of Example 1, the title compound was prepared according to a similar method to that described in Example 1, with the total yield of 8%.

A method similar to that as described in Example 9 is applied to resolute the isomers, the retention time of the TM65-E is earlier than that of the TM65-Z.

(Z)-N-(4-fluorophenyl)-4-((2-aminomethyl-3-fluoroallyl)oxy)-3-chloro-benzamide trifluoroacetate (TM65-Z).

MS m/z (ESI): 353 $[M+H]^+$
$^1$HNMR (400 MHz, $CD_3OD$) $\delta$: 8.06 (d, *J* = 2.4 Hz, 1H), 7.95 (dd, *J* = 8.4 Hz, 2.0 Hz, 1H), 7.68-7.65 (m, 2H), 7.30 (d, *J* = 8.4 Hz, 1H), 7.15 (d, *J* = 80.8 Hz, 1H), 7.10 (t, *J* = 8.8 Hz, 2H), 4.99 (d, *J* = 2.4 Hz, 2H), 3.76 (d, *J* = 2.4 Hz, 2H).

(E)-N-(4-fluorophenyl)-4-((2-aminomethyl-3-fluoroallyl)oxy)-3-chloro-benzannide trifluoroacetate (TM65-E).

MS m/z (ESI): 353 $[M+H]^+$
$^1$HNMR (400 MHz, $CD_3OD$) $\delta$: 8.06 (d, *J* = 2.4 Hz, 1H), 7.94 (dd, *J* = 8.4 Hz, 2.0 Hz, 1H), 7.68-7.65 (m, 2H), 7.28 (d, *J* = 80.8 Hz, 1H), 7.27 (d, *J* = 8.4 Hz, 1H), 7.10 (t, *J* = 8.8 Hz, 2H), 4.78 (d, *J* = 3.2 Hz, 2H), 3.88 (d, *J* = 3.2 Hz, 2H).

Example 15: Preparation of (4-((2-aminomethyl-3-fluoroallyl)oxy)phenyl)-(3,4-dihydroisoquinolin-2(1H)-yl)methanone trifluoroacetate (TM45)

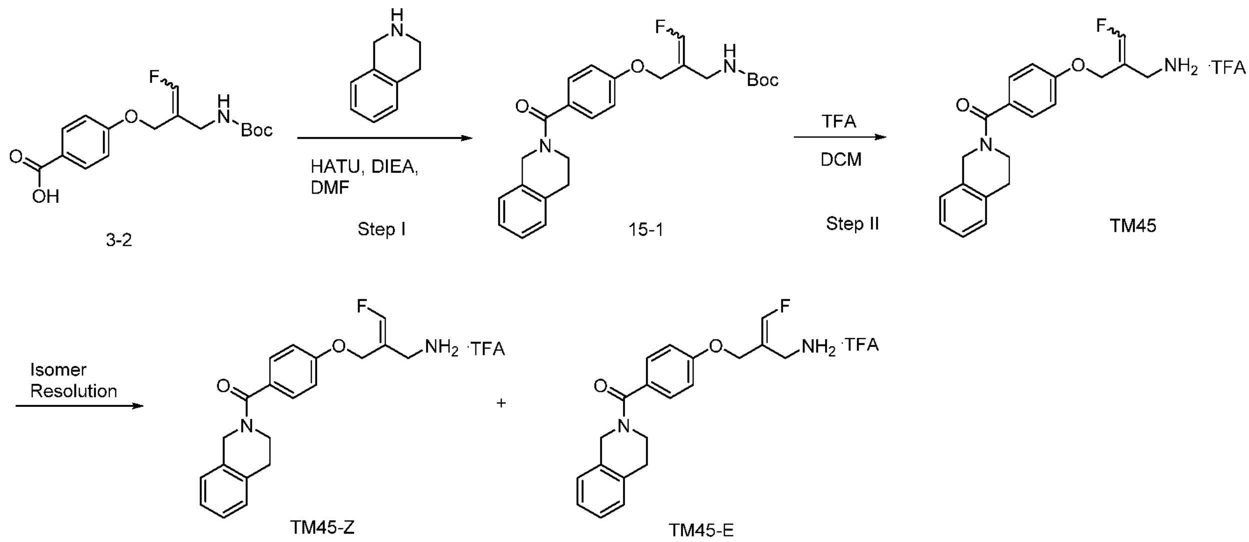


3-2 15-1 TM45 TM45-Z + TM45-E

Except for that in Step I of the example, tetrahydroisoquinoline replaced the para-fluoroaniline in Step III of Example 3, the title compound was prepared according to a similar method to that described in Steps III and IV of Example 3, with the total yield of 81%.

A method similar to that as described in Example 9 is applied to resolute the isomers, the retention time of the TM45-E is earlier than that of the TM45-Z.

(Z)-(4-((2-aminomethyl-3-fluoroallyl)oxy)phenyl)-(3,4-dihydroisoquinoline-2(1 H)-yl)methanone trifluoroacetate (TM45Z).

MS m/z (ESI): 341 $[M+H]^+$
$^1$HNMR (400 MHz, $CD_3OD$) $\delta$: 7.47 (d, *J* = 8.4 Hz, 2H), 7.21-7.11 (m, 6H), 7.11 (d, *J* = 80.8 Hz, 1H, 1H), 4.89 (d, *J* = 2.4 Hz, 2H), 4.83-4.67 (m, 2H), 3.94-3.70 (m, 2H), 3.70 (d, *J* = 2.0 Hz, 2H), 2.93 (s, 2H).

(E)-(4-((2-aminomethyl-3-fluoroallyl)oxy)phenyl)-(3,4-dihydroisoquinoline-2(1H)-yl)methanone trifluoroacetate (TM45-E).

MS m/z (ESI): 341 $[M+H]^+$
$^1$HNMR (400 MHz, $CD_3OD$) $\delta$: 7.46 (d, *J* = 8.8 Hz, 2H), 7.24 (d, *J* = 81.2 Hz, 1H), 7.19-6.92 (m, 6H), 4.82-4.67 (m, 2H), 4.67 (d, *J* = 2.8 Hz, 2H), 3.94 (br, 1H), 3.83 (s, 2H), 3.70 (br, 1H), 2.92 (s, 2H).

Example 16: Preparation of (4-((2-aminomethyl-3-fluoroallyl)oxy)phenyl)-(4-phenylpiperidin-1-yl)methanone trifluoroacetate (TM44)

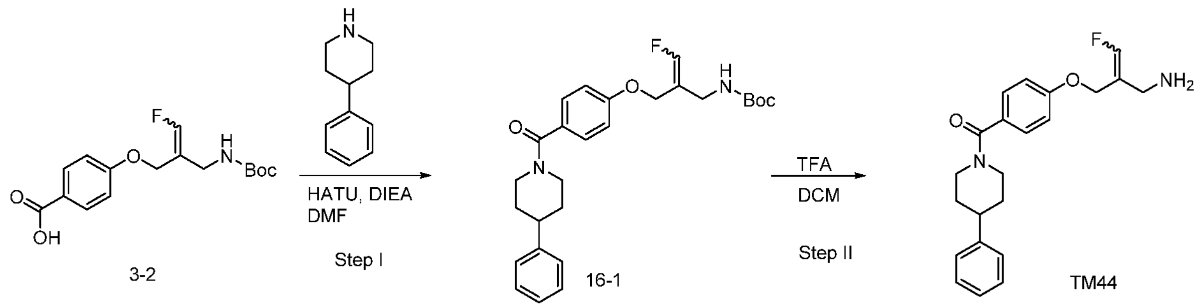


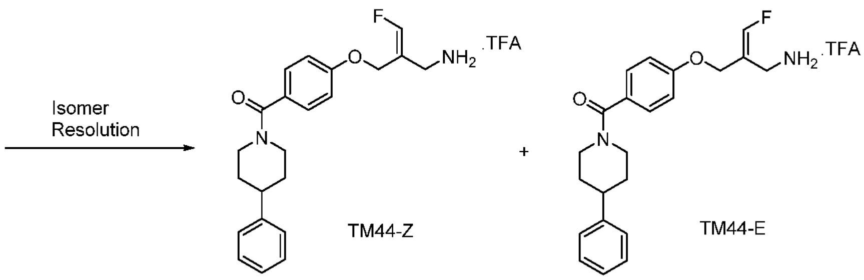


Except for that in Step I of the example, 4-phenylpiperidine replaced the para-fluoroaniline in Step III of Example 3, the title compound was prepared according to a similar method to that described in Steps III and IV of Example 3, with the total yield of 42%.

A method similar to that as described in Example 9 is applied to resolute the isomers, the retention time of the TM44-E is earlier than that of the TM44-Z.

(Z)-(4-((2-aminomethyl-3-fluoroallyl)oxy)phenyl)-(4-phenylpiperidine-1-yl)methanone trifluoroacetate (TM44-Z)

MS m/z (ESI): 369 $[M+H]^+$
$^1$HNMR (400 MHz, $CD_3OD$) δ: 7.46 (d, *J* = 8.8 Hz, 2H), 7.22-7.00 (m, 8H), 4.84 (d, *J* = 2.4 Hz, 2H), 4.81-4.56 (m, 1H), 4.01-3.83 (m, 1H), 3.69 (d, *J* = 2.4 Hz, 2H), 3.23-2.82 (m, 3H), 2.10-1.54 (m, 4H).

(E)-(4-((2-aminomethyl-3-fluoroallyl)oxy)phenyl)-(4-phenylpiperidine-1-yl)methanone trifluoroacetate (TM44-E)

MS m/z (ESI): 369 $[M+H]^+$
$^1$HNMR (400 MHz, $CD_3OD$) δ: 7.45 (d, *J* = 8.4 Hz, 2H), 7.34-7.24 (m, 6H), 7.09 (d, *J* = 8.8 Hz, 2H), 4.84-4.70 (m, 1H), 4.66 (d, *J* = 3.2 Hz, 2H), 4.01-3.86 (m, 1H), 3.82 (d, *J* = 2.0 Hz, 2H), 3.27-2.93 (m, 2H), 2.90-2.82 (m, 1H), 2.04-1.55 (m, 4H).

Example 17: Preparation of 2-(4-(2-pyrimidyl)phenoxymethyl)-3-fluoroallylamine trifluoroacetate (TM39)

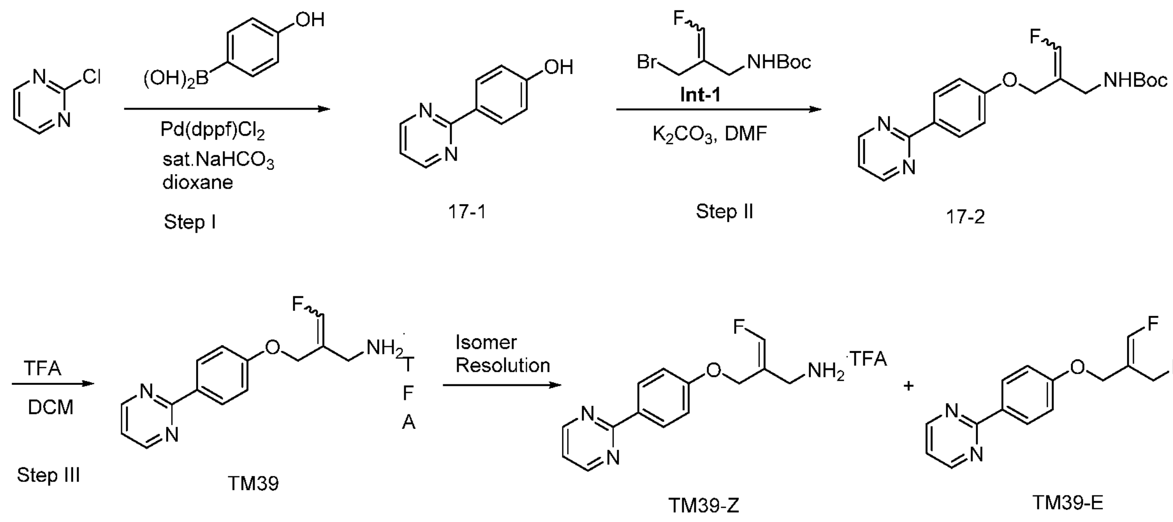


Step I: Preparation of 4-(2-pyrimidyl)phenol (17-1)

2-chloropyrimidine (1.0 g, 8.77 mmol), 4-hydroxyphenylboric acid (2.3 g, 10.53 mmol), the saturated sodium bicarbonate aqueous solution (2 mL) and dioxane (8 mL) were placed in a reaction flask and bubbled up with nitrogen gas for 5 min, and [1,1'-bis(diphenylphosphino)ferrocene]palladium dichloride (320 mg, 0.44 mmol) was added thereto. The mixture was further bubbled up with nitrogen gas for 3 min, and then heated to 100 °C and stirred for 8 hour. The reaction solution was extracted with water and ethyl acetate. The organic phases were combined, washed with water and saturated saline solution, then dried over anhydrous sodium sulfate, and concentrated to give the crude product, which was purified by silica gel column chromatograph to afford the product (150 mg, 9%).
MS m/z (ESI):173 $[M+H]^+$

Step II: Preparation of 2-(4-(2-pyrimidyl) phenoxymethyl)-3-fluoroallylamine (17-2)

The compound 17-1 (110 mg, 0.636 mmol), the compound Int-1 (204 mg, 0.763 mmol) and potassium carbonate (132 mg, 0.954 mmol) were dissolved in DMF (10 mL) and reacted at room temperature overnight. The reaction solution was extracted by adding water and ethyl acetate. The organic phases were combined, washed with water and saturated saline solution, dried over anhydrous sodium sulfate, and concentrated. The crude product was purified by TLC plate to afford the product (100 mg, 37%).

Step III: Preparation of 2-(4-(2-pyrimidyl) phenoxymethyl)-3-fluoroallylamine trifluoroacetate (TM39)

Except for that the intermediate 17-2 in this step replaced the intermediate 1-2 in Step III of Example 1, the title compound was prepared according to a similar method to that described in Step III of Example 1, with the yield of about 100%.

Method of resolution of 2-(4-(2-pyrimidyl) phenoxymethyl)-3-fluoroallylamine trifluoroacetate isomers

Apparatus: Agilent 1260, Sunfire C18, OBD™ 5 μm, 19*150 mm column; Mobile phase: A: 0.1% TFA, B: ACN; Elution gradient: flow rate 16 mL/min; 0-9.6 min: 10%-28% B, 9.7-12 min: 95% B; 12.1-15 min: 10% B.
The eluent liquid having a retention time from 6.8 to 7.7 min was collected and lyophilized to afford the TM39-E, and the eluent liquid having a retention time from 8.2 to 8.8 min was collected and lyophilized to afford the TM39-Z.

(Z)-2-(4-(2-pyrimidyl)phenoxymethyl)-3-fluoroallylamine trifluoroacetate (TM39-Z)

MS m/z (ESI): 260 $[M+H]^+$
$^1$HNMR (400 MHz, $CD_3OD$) δ: 8.80 (d, *J* = 4.8Hz, 2H), 8.37 (d, *J* = 8.8 Hz, 2H), 7.31 (t, *J* = 4.8 Hz, 1H), 7.14 (d, *J* = 7.2 Hz, 2H), 7.12 (d, *J* = 80.8 Hz, 1H), 4.90 (d, *J* = 2.4 Hz, 2H), 3.71 (d, J = 2.0 Hz, 2H).

(E)- 2-(4-(2-pyrimidyl) phenoxymethyl)-3-fluoroallylamine trifluoroacetate (TM39-E)

MS m/z (ESI): 260 $[M+H]^+$
$^1$HNMR (400 MHz, $CD_3OD$) δ: 8.80 (d, *J* = 4.8Hz, 2H), 8.37 (d, *J* = 8.8 Hz, 2H), 7.31 (t, *J* = 4.8 Hz, 1H), 7.24 (d, *J* = 81.2 Hz, 1H), 7.14-7.11 (m, 2H), 4.70 (d, *J* = 3.2 Hz, 2H), 3.85 (d, *J* = 1.2 Hz, 2H).

Example 18: Preparation of 2-(4-(1H-benzoimidazol-2-yl)phenoxymethyl)-3-fluoroallylamine trifluoroacetate (TM42)

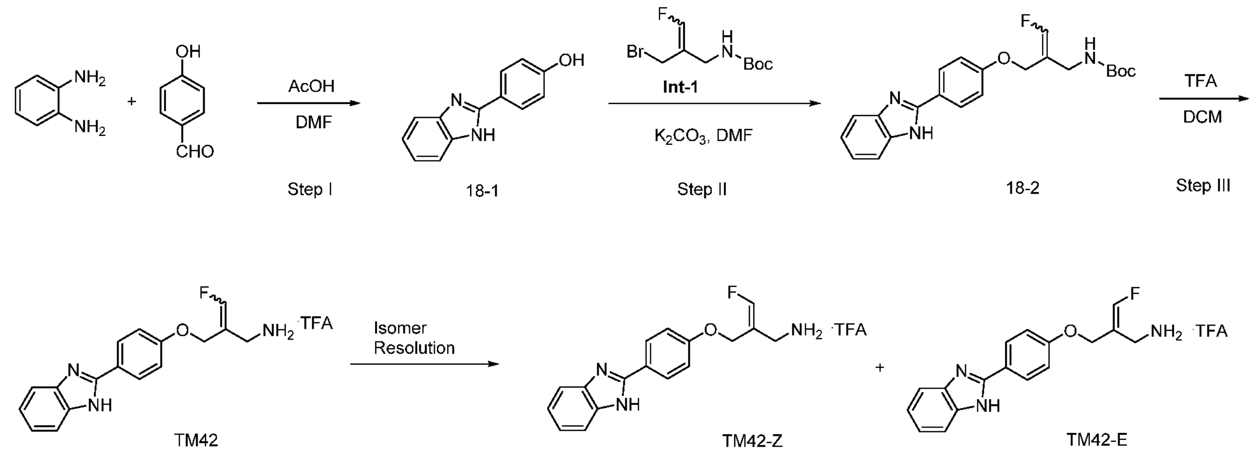


Step I: Preparation of 4-(1H-benzoimidazol-2-yl)phenol (18-1)

The compound ortho-diphenylamine (300 mg, 2.78 mmol) and parahydroxybenzaldehyde (430 mg, 3.52 mmol) were dissolved in DMF (10 mL) and then dripped with ice acetic acid (20 drops), the mixture was reacted under reflux at 145 °C for 5 h. Water was added to quench the reaction, and the reaction solution was extracted with ethyl acetate. The organic phases were combined, then added with saturated saline solution to precipitate yellow solids, and the solids were filtered and dried to afford the target product (220 mg, 38%).
MS m/z(ESI): 211 $[M+H]^+$

Step II: Preparation of tert-butyl (2-(4-(1H-benzoimidazol-2-yl) phenoxymethyl)-3-fluoroallyl)carbamate (18-2)

Except for that the intermediate 18-1 in this step replaced the intermediate 1-1 in Step II of Example 1, the title compound was prepared according to a similar method to that described in Step II of Example 1, and the crude product is directly used in the next step.
MS m/z (ESI): 398 $[M+H]^+$

Step III: Preparation of 2-(4-(1H-benzoimidazol-2-yl) phenoxymethyl)-3-fluoroallylamine trifluoroacetate (TM42)

Except for that the intermediate 18-2 in this step replaced the intermediate 1-2 in Step III of Example 1, the title compound was prepared according to a similar method to that described in Step III of Example 1, with the yield of 90%.

Method of resolution of 2-(4-(1H-benzoimidazole-2-yl)phenoxymethyl)-3-fluoroallylamine trifluoroacetate isomers

A separation method similar to that as described in Example 9 is applied, wherein elution gradient: 0-6 min: 10%-28.7% B, flow rate: 16 mL/min; 6.1-9 min: 95% B, flow rate: 16 mL/min; 9.1-12 min: 10% B, flow rate: 16 mL/min. The eluent liquid having a retention time from 5.1 to 5.3 min was collected and lyophilized to afford the TM42-E, and the eluent liquid having a retention time from 5.5 to 5.7 min was collected and lyophilized to afford the TM42-Z.

(Z)-2-(4-(1H-benzoimidazol-2-yl)phenoxymethyl)-3-fluoroallylamine trifluoroacetate (TM42-Z)

MS m/z (ESI): 298 $[M+H]^+$
$^1$HNMR (400 MHz, $CD_3OD$) δ: 8.12 (d, *J* = 8.8 Hz, 2H), 7.77-7.75 (m, 2H), 7.55-7.54 (m, 2H), 7.34 (d, *J* = 9.2 Hz, 2H),

7.16 (d, *J* = 80.4 Hz, 1H), 4.96 (d, *J* = 2.0 Hz, 2H), 3.73 (d, J = 2.0 Hz, 2H).

(E)-2-(4-(1H-benzoimidazol-2-yl)phenoxymethyl)-3-fluoroallylamine trifluoroacetate (TM20-E)

MS m/z (ESI): 298 $[M+H]^+$
$^1$HNMR (400 MHz, $CD_3OD$) δ: 8.13 (d, *J* = 8.0 Hz, 2H), 7.79-7.77 (m, 2H), 7.59-7.57 (m, 2H), 7.34 (d, *J* = 8.8 Hz, 2H), 7.29 (d, *J* = 80.8 Hz, 1H), 4.77 (d, *J* = 3.2 Hz, 2H), 3.86 (d, *J* = 2.0 Hz, 2H).

Example 19: Preparation 2-(4-(6-chloro-benzothiazol-2-yl)phenoxymethyl)-3-fluoroallylamine trifluoroacetate (TM41)

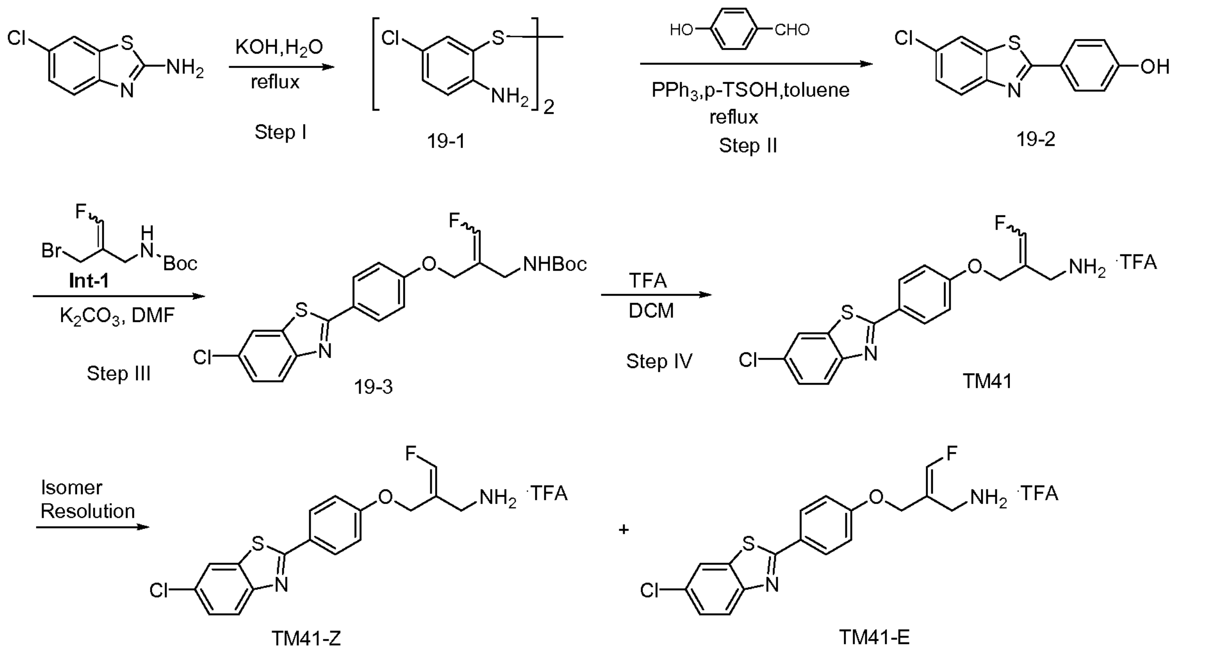


Step I: Preparation of bis(2-amino-5-chlorophenyl)dithioether (19-1)

2-amino-6-chloro-benzothiazole (2 g, 5.4 mmol) was dissolved in water (50 mL) and added with potassium hydroxide (10 g, 89 mmol). The mixture was reacted under reflux at 100 °C for 5 hours. After the completion of the reaction, the reaction solution was cooled to room temperature and regulated to pH=6-7 by adding 4N HCl. The mixture added with water and stirred for 1 hour, and then was subjected to suction filtration to afford yellow solids, and after being recrystallized with ethanol/water, yellow solids (710 mg, 32%) were obtained.

Step II: Preparation of 4-(6-chloro-benzothiazole-2-yl)-phenol (19-2)

The compound 19-1 (710 mg, 2.3 mmol), para-hydroxybenzoaldehyde (280 mg, 4.6 mmol), triphenylphosphine (600 mg, 2.3 mmol) and para-methylbenzene sulfonic acid (90 mg, 0.46 mmol) were mixed and dissolved in toluene (100 mL), and the mixture was heated with stirring to reflux and reacted for 24 hours. After the completion of the reaction, the reaction solution was cooled to room temperature and concentrated, and then dissolved with dichloromethane. The insoluble matter was filtered out. The mother liquor was concentrated and purified by preparative plate to afford white solids (120 mg, 27%).
MS m/z (ESI): 262[M+H]+

Step III: Preparation of tert-butyl (2-(4-(6-chloro-benzothiazol-2-yl)phenoxymethyl)-3-fluoroallyl)carbamate (19-3)

Except for that the intermediate 19-2 in this step replaced the intermediate 1-1 in Step II of Example 1, the title compound was prepared according to a similar method to that described in Step II of Example 1, with the yield of 32%.
MS m/z (ESI): 449$[M+H]^+$

Step IV: Preparation of 2-(4-(6-chloro-benzothiazol-2-yl)phenoxymethyl)-3-fluoroallylamine trifluoroacetate (TM41)

Except for that the intermediate 19-3 in this step replaced the intermediate 1-2 in Step III of Example 1, the title compound was prepared according to a similar method to that described in Step III of Example 1, with the yield of 90%.

A method similar to that as described in Example 9 is applied to resolute the isomers, the retention time of the TM41-E is earlier than that of the TM41-Z.

(Z)-2-(4-(6-chloro-benzothiazol-2-yl)phenoxymethyl)-3-fluoroallylamine trifluoroacetate (TM41-Z)

MS m/z (ESI): 349 $[M+H]^+$

$^1$HNMR (400 MHz, $CD_3OD$) $\delta$: 8.07 (d, *J* = 8.8 Hz, 2H), 8.03 (d, *J* = 2.0 Hz, 1H), 7.93 (d, *J* = 8.4 Hz, 1H), 7.51 (dd, *J* = 8.8 Hz, 2.0 Hz, 1H), 7.19 (d, *J* = 9.2 Hz, 2H), 7.12 (d, *J* = 80.8 Hz, 1H), 4.91 (d, *J* = 2.4 Hz, 2H), 3.71 (d, *J* = 2.4 Hz, 2H).

(E)-2-(4-(6-chloro-benzothiazol-2-yl)phenoxymethyl)-3-fluoroallylamine trifluoroacetate (TM41-E)

MS m/z (ESI): 349 $[M+H]^+$

$^1$HNMR (400 MHz, $CD_3OD$) $\delta$: 8.06 (d, *J* = 8.8 Hz, 2H), 8.03 (d, *J* = 2.0 Hz, 1H), 7.93 (d, *J* = 8.8 Hz, 1H), 7.51 (dd, *J* = 8.8 Hz, 2.0 Hz, 1H), 7.27 (d, 1H), 7.17 (d, *J* = 8.8 Hz, 2H), 4.71 (d, *J* = 3.2 Hz, 2H), 3.84 (d, *J* = 2.0 Hz, 2H).

Example 20: Preparation of 2-(4-(6-chloro-benzothiazol-2-yl)-2-fluorophenoxymethyl)-3-fluoroallylamine trifluoroacetate trifluoroacetate (TM74)

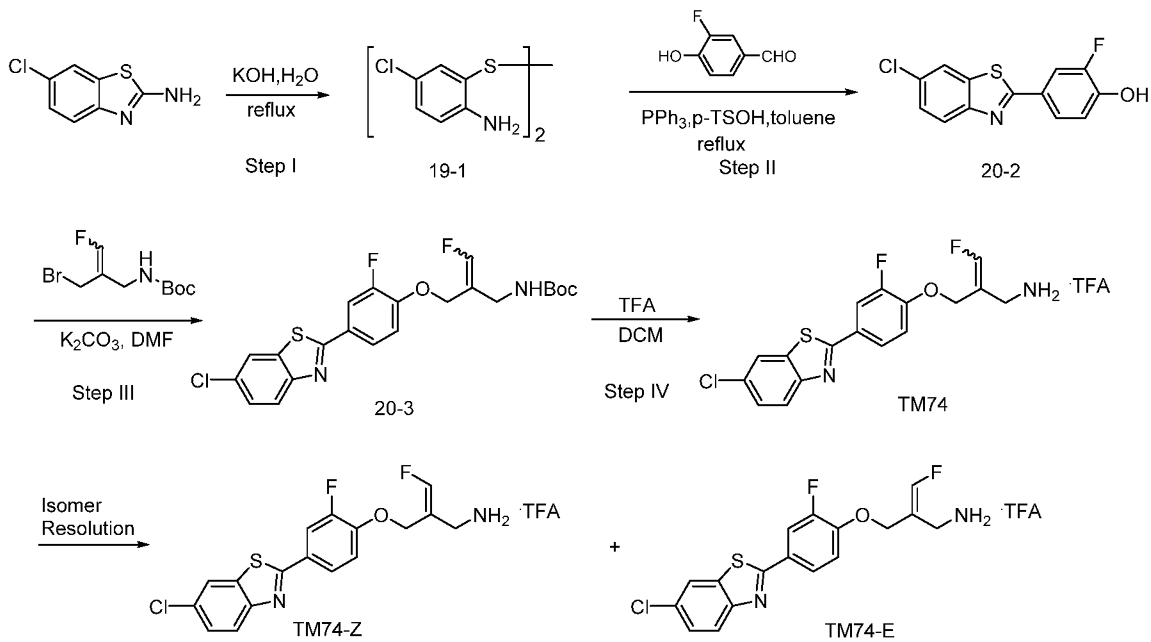


Except for that in step II of this example, 3-fluoro-4-hydroxybenzoaldehyde replaced the para-hydroxybenzoaldehyde in Step II of Example 19, the title compound was prepared according to a similar method to that described in Example 19, with the total yield of 9%.

A method similar to that as described in Example 9 is applied to resolute the isomers, the retention time of the TM74-E is earlier than that of the TM74-Z.

(Z)-2-(4-(6-chloro-benzothiazol-2-yl)-2-fluorophenoxymethyl)-3-fluoroallylamine trifluoroacetate trifluoroacetate (TM74-Z)

MS m/z (ESI): 367 $[M+H]^+$

$^1$HNMR (400 MHz, $CD_3OD$) $\delta$: 8.05 (d, *J* = 2.0 Hz, 1H), 7.97-7.87 (m, 3H), 7.52 (dd, *J* = 8.8 Hz, 2.0 Hz, 1H), 7.36 (t, *J* = 8.4 Hz, 1H), 7.15 (d, *J* = 80.4 Hz, 1H), 4.98 (d, *J* = 2.4 Hz, 2H), 3.74 (d, *J* = 2.8 Hz, 2H).

(E)-2-(4-(6-chloro-benzothiazol-2-yl)-2-fluorophenoxymethyl)-3-fluoroallylamine trifluoroacetate trifluoroacetate (TM74-E)

MS m/z (ESI): 367 $[M+H]^+$
$^1$HNMR (400 MHz, $CD_3OD$) δ: 8.05 (d, *J* = 2 Hz, 1H), 7.97-7.87 (m, 3H), 7.53 (dd, *J* = 8.8 Hz, 2.0 Hz, 1H), 7.34 (t, *J* = 8.4 Hz, 1H), 7.28 (d, *J* = 80.8 Hz, 1H), 4.79 (d, *J* = 3.2 Hz, 2H), 3.86 (d, *J* = 2.0 Hz, 2H).

Example 21: Preparation of 5-((2-aminomethyl-3-fluoroallyl)oxy)-2-(4-chlorobenzyl)isoindolin-1-one (TM33)

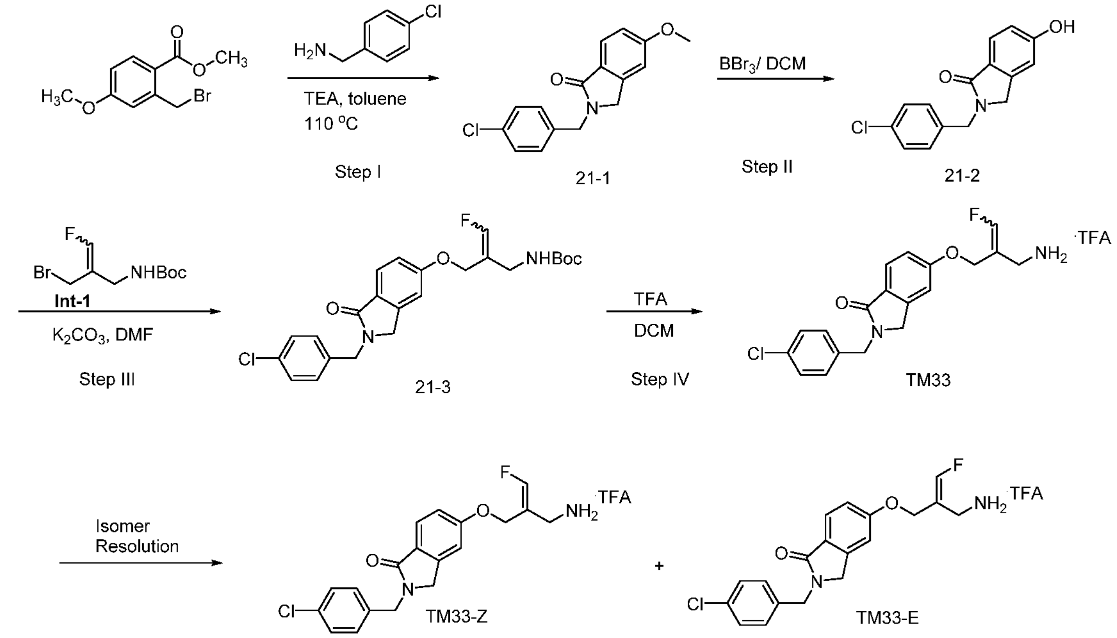


Step I: Preparation of 2-(4-chlorobenzyl)-5-methoxyisoindolin-1-one (21-1)

The reactants ethyl 2-bromomethyl-4-methoxybenzoate (500 mg, 1.93 mmol), para-chlorobenzylamine (272 mg, 1.93 mmol) and triethylamine (585 mg, 5.79 mmol) were dissolved in toluene (3 mL), then sealed and heated at 110 °C for 4 hours. After the completion of the reaction, the reaction solution was concentrated, then was extracted with water and ethyl acetate. The organic phases were combined, washed with water and saturated saline solution, dried over anhydrous sodium sulfate and concentrated. The crude product was purified by silica column chromatograph to afford the product 21-1 (275 mg, 50%).

Step II: Preparation of 2-(4-chlorobenzyl)-5-hydroxyisoindolin-1-one (21-2)

The compound 21-1 (150 mg, 0.52 mmol) was dissolved in dichloromethane (20 mL), and added with $BBr_3$ (392 mg, 1.57 mmol) at -78 °C, and then the mixture was warmed up to room temperature and reacted for 1 hour. After the reaction was complete, the reaction solution was added to ice water to quench the reaction, and extracted with dichloromethane. The organic phases were combined, washed with water and saturated saline solution, dried over anhydrous sodium sulfate and concentrated. The crude product was purified by silica gel column chromatography to afford the product 21-2 (110 mg, 96%).

Step III: Preparation of tert-butyl (2-(2-(4-chlorobenzyl)-1-oxoisoindolin-5-oxymethyl)-3-fluoroallyl)carbamate (21-3)

The compound 21-2 (80 mg, 0.293 mmol), the compound Int-1 (81 mg, 0.322 mmol) and potassium carbonate (61 mg, 0.439 mmol) were dissolved in DMF (10 mL) and reacted at room temperature overnight. The reaction solution was extracted by adding water and ethyl acetate. The organic phases were combined, washed with water and saturated saline solution, dried over anhydrous sodium sulfate and concentrated to afford the crude product, which was purified by preparative TLC to afford the product 21-3 (80 mg, 48%).

Step IV: Preparation of 5-((2-aminomethyl-3-fluoroallyl)oxy)-2-(4-chlorobenzyl)isoindolin-1-one (TM33)

Except for that the intermediate 21-3 in this step replaced the intermediate 1-2 in Step III of Example 1, the title compound was prepared according to a similar method to that described in Step III of Example 1, with the yield of 100%.

A method similar to that as described in Example 9 is applied to resolute the isomers, the retention time of the TM33-E is earlier than that of the TM33-Z.

(Z)-5-((2-aminomethyl-3-fluoroallyl)oxy)-2-(4-chlorobenzyl)isoindolin-1-one (TM33-Z)

MS m/z (ESI): 361 $[M+H]^+$
$^1$HNMR (400 MHz, DMSO-$d_6$) $\delta$: 8.08 (br, 2H), 7.66 (d, $J$ = 8.4 Hz, 1H), 7.41 (d, $J$ = 8.4 Hz, 2H), 7.32-7.07(m, 5H), 4.78 (d, $J$ = 1.6 Hz, 2H), 4.69 (s, 2H), 4.32 (s, 2H), 3.56 (s, 2H).

(E)-5-((2-aminomethyl-3-fluoroallyl)oxy)-2-(4-chlorobenzyl)isoindolin-1-one (TM33-E)

MS m/z (ESI): 361$[M+H]^+$
$^1$HNMR (400 MHz, DMSO-$d_6$) $\delta$: 8.17 (br, 2H), 7.66 (d, $J$ = 8.4 Hz, 1H), 7.45-7.24 (m, 5H), 7.18 (d, $J$ = 1.6 Hz, 1H), 7.11 (dd, $J$ = 8.4 Hz, 2.0 Hz, 1H), 4.69 (s, 2H), 4.67 (s, 2H), 4.32 (s, 2H), 3.64 (s, 2H).

Example 22: Preparation of 6-((2-aminomethyl-3-fluoroallyl)oxy)-3,4-dihydroisoquinolin-1(2H)-one trifluoroacetate (TM26)

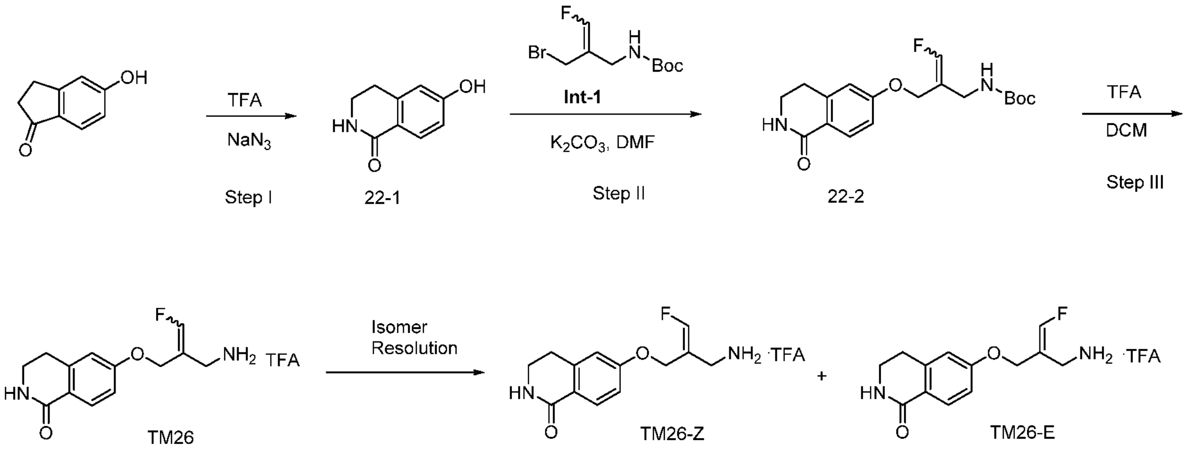


Step I: Preparation of 6-hydroxy-3,4-dihydroisoquinolin-1(2H)-one (22-1)

5-hydroxy-1-indone (300 mg, 2.03 mmol) was dissolved in trifluoroacetic acid (10 mL), and slowly added with sodium azide (200 mg, 3.08 mmol), and the mixture was refluxed and reacted at 74 °C for 5 hours. Then, the reaction solution was added with water and subjected to rotary evaporation under vacuum to remove most of trifluoroacetic acid. To the residue was added with sodium bicarbonate to regulate to weak basic, and then extracted with ethyl acetate. The organic phases were combined, washed with saturated saline solution, dried over anhydrous sodium sulfate and concentrated under vacuum to afford the target product (240 mg, 73%).
MS m/z(ESI): 164 $[M+H]^+$

Step II: Preparation of tert-butyl (2-(1-oxo-1,2,3,4-tetrahydroisoquinolin-6-oxymethyl)-3-fluoroallyl)carbamate (22-2)

Except for that the intermediate 22-1 in this step replaced the intermediate 1-1 in Step II of Example 1, the title compound was prepared according to a similar method to that described in Step II of Example 1, with the yield of 58%.
MS m/z (ESI): 351 $[M+H]^+$

Step III: Preparation of 6-((2-aminomethyl-3-fluoroallyl)oxy)-3,4-dihydroisoquinolin-1(2H)-one trifluoroacetate (TM26)

Except for that the intermediate 22-2 in this step replaced the intermediate 1-2 in Step III of Example 1, the title compound was prepared according to a similar method to that described in Step III of Example 1, with the yield of 90%.

A method similar to that as described in Example 9 is applied to resolute the isomers, the retention time of the TM26-E is earlier than that of the TM26-Z.

(Z)-6-((2-aminomethyl-3-fluoroallyl)oxy)-3,4-dihydroisoquinolin-1(2H)-one trifluoroacetate (TM26-Z)

MS m/z (ESI): 251 $[M+H]^+$
$^1$HNMR (400 MHz, $CD_3OD$) δ: 7.89 (dd, *J* = 8.8 Hz, 2.0 Hz, 1H), 7.11 (d, *J* = 80.8 Hz, 1H), 6.98 (dd, *J* = 8.4 Hz, 2.4 Hz, 1H), 6.93 (d, *J* = 2.4 Hz, 1H), 4.87 (d, *J* = 2.4 Hz, 2H), 3.69 (d, *J* = 2.4 Hz, 2H), 3.48 (t, *J* = 6.8 Hz, 2H), 2.97 (t, *J* = 6.8 Hz, 2H).

(E)-6-((2-aminomethyl-3-fluoroallyl)oxy)-3,4-dihydroisoquinolin-1(2H)-one trifluoroacetate (TM26-E)

MS m/z (ESI): 251 $[M+H]^+$
$^1$HNMR (400 MHz, $CD_3OD$) δ: 7.88 (dd, *J* = 8.8 Hz, 2.0 Hz, 1H), 7.23 (d, *J* = 80.8 Hz, 1H), 6.97 (dd, *J* = 8.4 Hz, 2.4 Hz, 1H), 6.91 (d, *J* = 2.0 Hz, 1H), 4.67 (d, *J* = 2.4 Hz, 2H), 3.82 (d, *J* = 1.2 Hz, 2H), 3.48 (t, *J* = 6.8 Hz, 2H), 2.96 (t, *J* = 6.8 Hz, 2H).

Example 23: Preparation of 6-((2-aminomethyl-3-fluoroallyl)oxy)-4,4-dimethyl-3,4-dihydroisoquinolin-1(2H)-one trifluoroacetate (TM27)

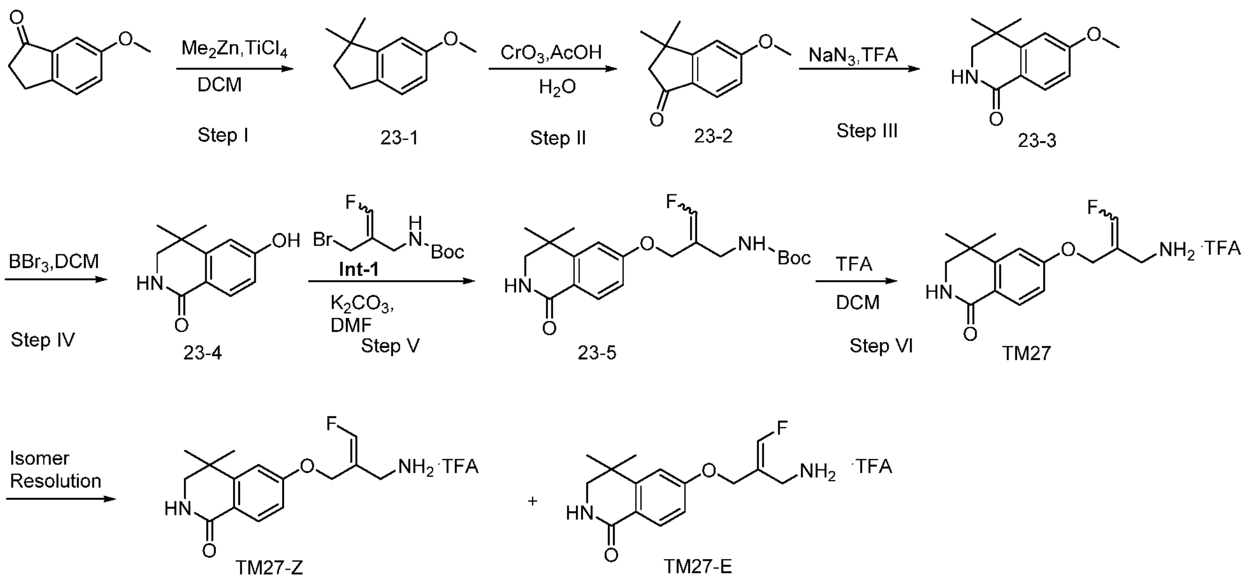


Step I: Preparation of 6-methoxy-1,1-dimethyl-2,3-dihydro-1H-indene (23-1)

$TiCl_4$ (2.2 mL, 0.02 mmol) was dissolved in 20 mL anhydrous dichloromethane and added with $Me_2Zn$ (80 mL, 0.08 mmol) at -40 °C with stirring under the nitrogen protection. The mixture was kept at the temperature and stirred for 30 min, 6-methoxy-1-indone (1.76 g, 0.01 mmol) dissolved with 5 mL dichloromethane was added and the mixture was slowly raised to room temperature and reacted overnight. To the reaction solution, 10 mL anhydrous methanol was slowly dripped to quench the reaction, and then dichloromethane and saturated $NH_4Cl$ solution were added for dilution and extraction. The organic phase was washed with saturated $NH_4Cl$ solution twice, and the aqueous phase was washed twice with dichlormethane. The organic phases were combined, dried and concentrated to afford an oily liquid (2.10 g, 72%).

Step II: Preparation of 5-methoxy-3,3-dimethyl-2,3-dihydro-1H-inden-1-one (23-2)

The compound 23-1 (1.65 g, 9.4 mmol) was dissolved with 20 ml ice acetic acid, and added with $CrO_3$ that was

dissolved with 8 mL ice acetic acid and 7 mL water with stirring at 0 °C, and the mixture was slowly raised to room temperature and reacted overnight. The reaction solution was diluted with water and then extracted twice with ethyl acetate. The organic phase was washed with saturated sodium bicarbonate solution to neutral, and then dried and concentrated to afford a yellow oily liquid (1.50 g, 83%).

Step III: Preparation of 6-methoxy-4,4-dimethyl-3,4-dihydroisoquinolin-1-(2H)-one (23-3)

The compound 23-2 (1.1 g, 5.8 mmol) and $NaN_3$ (0.65 g, 10 mmol) were mixed and placed in a three-mouth flask and added with trifluoroacetic acid (20 mL), and the mixture was reacted for 4 hours at reflux. After the reaction was complete, the reaction solution was cooled to room temperature and then diluted with water. The mixture was regulated to pH = 7-8 with saturated sodium bicarbonate, and extracted twice with dichloromethane, the organic phases were combined. The organic phase was dried and concentrated to afford brown solids, which was recrystalized from petroleum ether-ethyl acetate mixed solution (petroleum ether: ethyl acetate = 20:1) to afford brown solids (600 mg, 62%).

Step IV: Preparation of 6-hydroxy-4,4-dimethyl-3,4-dihydroisoquinolin-1-(2H)-one (23-4)

To the compound 23-3 (168 mg, 0.82 mmol), 17% $BBr_3$ solution in dichloromethane (10 mL) was added and reacted at room temperature overnight. After the reaction was complete, methanol was added to quench the reaction. The reaction solution was concentrated and dissolved with a small amount of dichloromethane, then separated by preparative TLC to afford white solids (82 mg, 52%).

Step V: Preparation of tert-butyl (2-(4,4-dimethyl-1,2,3,4-tetrahydroisoquinolin-1-one-6-oxymethyl)-3-fluoroallyl)carbamate (23-5)

Except for that the intermediate 23-4 in this step replaced the intermediate 1-1 in Step II of Example 1, the title compound was prepared according to a similar method to that described in Step II of Example 1, with the yield of 32%.

Step VI: Preparation of 6-((2-aminomethyl-3-fluoroallyl)oxy)-4,4-dimethyl-3,4-dihydroisoquinolin-1(2H)-one trifluoroacetate (TM27)

Except for that the intermediate 23-5 in this step replaced the intermediate 1-2 in Step III of Example 1, the title compound was prepared according to a similar method to that described in Step III of Example 1, with the yield of about 100%.

A method similar to that as described in Example 9 is applied to resolute the isomers, the retention time of the TM27-E is earlier than that of the TM27-Z.

(Z)-6-((2-aminomethyl-3-fluoroallyl)oxy)-4,4-dimethyl-3,4-dihydroisoquinolin-1(2H)-one trifluoroacetate (TM27-Z)

MS m/z (ESI): 279 $[M+H]^+$
$^1$HNMR (400 MHz, $CD_3OD$) $\delta$: 7.93 (d, *J* = 8.4 Hz, 1H), 7.22-6.95 (m, 3H), 4.90 (d, *J* = 2.4 Hz, 2H), 3.69 (d, *J* = 2.4 Hz, 2H), 3.27 (s, 2 H), 1.33 (s, 6H).

(E)-6-((2-aminomethyl-3-fluoroallyl)oxy)-4,4-dimethyl-3,4-dihydroisoquinolin-1(2H)-one trifluoroacetate (TM27-E).

MS m/z (ESI): 279 $[M+H]^+$
$^1$HNMR (400 MHz, $CD_3OD$) $\delta$: 7.93 (d, *J* = 8.4 Hz, 1H), 7.35-6.96 (m, 3H), 4.69 (d, *J* = 3.6 Hz, 2H), 3.83 (s, 2H), 3.27 (s, 2H), 1.33 (s, 6H).

Example 24: Preparation of 5-((2-aminomethyl-3-fluoroallyl)oxy)isoindolin-1-one trifluoroacetate (TM77)

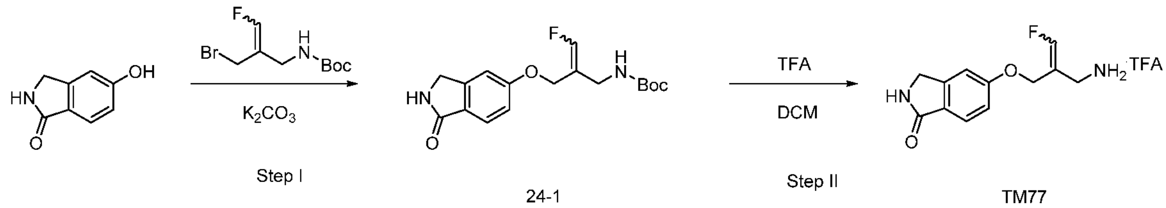


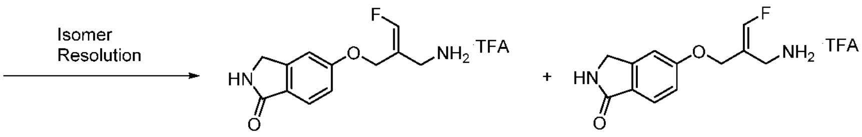


TM77-Z TM77-E

Except for that 5-hydroxyl-isoindol-1-one in this step I replaced the intermediate 1-1 in Step II of Example 1, the title compound was prepared according to method similar to that described in Steps II and III of Example 1, with the total yield of 63%.

A method similar to that as described in Example 9 is applied to resolute the isomers, the retention time of the TM77-E is earlier than that of the TM77-Z

(Z)-5-((2-aminomethyl-3-fluoroallyl)oxy)isoindol-1-one trifluoroacetate (TM77-Z).

MS m/z (ESI): 226 $[M+H]^+$
$^1$HNMR (400 MHz, $CD_3OD$) δ: 7.73 (d, *J* = 8.4 Hz, 1H), 7.22 (s, 1H), 7.15 (dd, *J* = 8.4 Hz, 2.0 Hz, 1H), 7.12 (d, *J* = 80.8 Hz, 1H), 4.90 (d, *J* = 2.4 Hz, 2H), 4.43 (s, 2H), 3.71 (d, *J* = 2.0 Hz, 2H).

(E)-5-((2-aminomethyl-3-fluoroallyl)oxy)isoindol-1-one trifluoroacetate (TM77-E).

MS m/z (ESI): 226 $[M+H]^+$
$^1$HNMR (400 MHz, $CD_3OD$) δ: 7.72 (d, *J* = 8.4 Hz, 1H), 7.35-7.12 (m, 3H), 4.70 (d, *J* = 2.8 Hz, 2H), 4.42 (s, 2 H), 3.84 (d, *J* = 2.0 Hz, 2H).

Example 25: Preparation of 6-((2-aminomethyl-3-fluoroallyl)oxy)-2,3-dihydro-1-indenone trifluoroacetate (TM7)

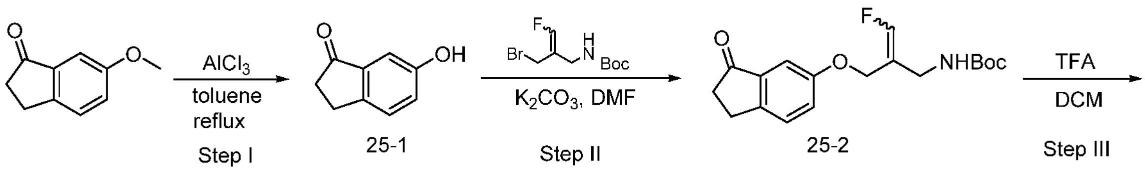


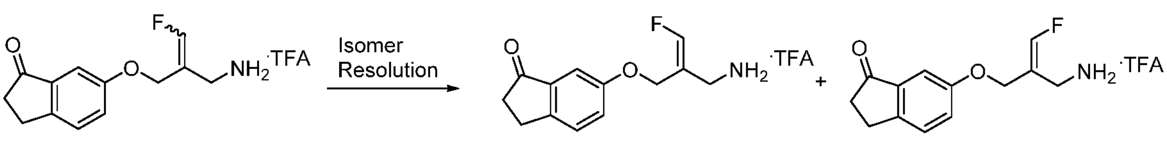


TM7 TM7-Z TM7-E

Step I: Preparation of 6-hydroxy-2,3-dihydro-1-indenone (25-1)

The compound 6-methoxy-2,3-dihydro-1-indenone (1.0 g, 6.17 mmol) was placed in a reaction flask and then added with the solvent toluene (20 mL). To the reactor with stirring, aluminum trichloride solids (2.4 g, 18.50mmol) were added and reacted at 105 °C for 2 hours. After TLC monitored the completion of the reaction, the solvent was removed by concentration, and the reaction solution was added with water and extracted with ethyl acetate. The organic phases

were combined, washed with saturated saline solution, dried and concentrated to afford a crude product, which was purified by column chromatography to afford the product 25-1 (420 mg, 46%).

Step II: Preparation of 6-(2-tert-butoxycarbonylaminomethyl-3-fluoroallyloxy)-2,3-dihydro-1-indenone (25-2)

Except for that the intermediate 25-1 in this step replaced the intermediate 1-1 in Step II of Example 1, the title compound was prepared according to a similar method to that described in Step II of Example 1.

Step III: Preparation of 6-((2-aminomethyl-3-fluoroallyl)oxy)-2,3-dihydro-1-indenone trifluoroacetate (TM7)

Except for that the intermediate 25-2 in this step replaced the intermediate 1-2 in Step III of Example 1, the title compound was prepared according to a similar method to that described in Step III of Example 1.
The total yield of Step II and Step III is 77%.

Method of resolution of 6-((2-aminomethyl-3-fluoroallyl)oxy)-2,3-dihydro-1-indenone trifluoroacetate isomers

A method similar to that as described in Example 9 is applied to resolute the isomers, wherein, elution gradient: 0-9 min: 10%-26.8% B, flow rate: 16 mL/min; 9.1-11 min: 95% B, flow rate: 20 mL/min; 11.1-13 min: 10% B, flow rate: 20 mL/min. The eluent liquid having a retention time from 6.6 to 7.6 min was collected and lyophilized to afford the TM7-E, and the eluent liquid having a retention time from 7.8 to 8.7 min was collected and lyophilized to afford the TM7-Z.

(Z)-6-((2-aminomethyl-3-fluoroallyl)oxy)-2,3-dihydro-1-indenone trifluoroacetate (TM7-Z)

MS m/z (ESI): 236 $[M+H]^+$
$^1$HNMR (400 MHz, $CD_3OD$) $\delta$: 7.49 (d, *J* = 8.4 Hz, 1H), 7.35 (dd, *J* = 8.4 Hz, 2.8 Hz, 1H), 7.26 (d, *J* = 2.8 Hz, 1H), 7.10 (d, *J* = 80.8 Hz, 1H), 4.85 (d, *J* = 2.4 Hz, 2H), 3.70 (d, *J* = 2.4 Hz, 2H), 3.11-3.09 (m, 2H), 2.73-2.70 (m, 2H).

(E)-6-((2-aminomethyl-3-fluoroallyl)oxy)-2,3-dihydro-1-indenone trifluoroacetate (TM7-E)

MS m/z (ESI): 236 $[M+H]^+$
$^1$HNMR (400 MHz, $CD_3OD$) $\delta$: 7.48 (d, *J* = 8.4 Hz, 1H), 7.35-7.32 (m, 1H), 7.25 (s, 1H), 7.23 (d, *J* = 81.2 Hz,1H), 4.65 (d, *J* = 3.6 Hz, 2H), 3.83 (d, *J* = 1.6 Hz, 2H), 3.10-3.18 (m, 2H), 2.72-2.70 (m, 2H).

Example 26: Preparation of 6-((2-aminomethyl-3-fluoroallyl)oxy)-3,3-dimethyl-3,4-dihydroisoquinolin-1(2H)-one trifluoroacetate (TM38)

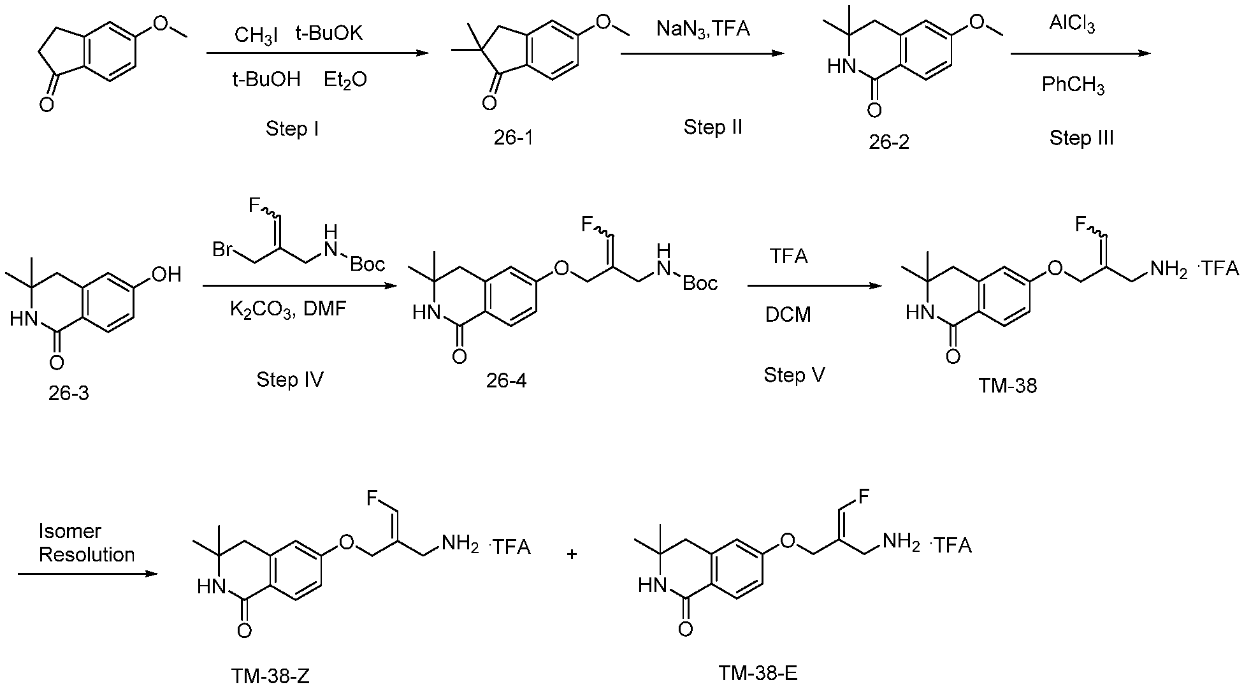


Step I: Preparation of 5-methoxy-2,2-dimethyl-2,3-dihydro-1H-inden-1-one (26-1)

The compound 5-methoxy-2,3-dihydro-1-indenone (1.5 g, 9.3 mmol) was dissolved in ether (40 mL), and followed by dripping with iodomethane (2.9 mL, 46.2 mmol) in ice bath. Then in the ice bath, the mixture was added with the solution of potassium tert-butanol (3.4 g, 30.4 mmol) in tert-butanol (20 mL) and reacted at reflux for 6 hours. Water was added to quench the reaction, and the reaction solution was extracted twice with ether. The organic phase was washed with a saturated saline solution. The organic phase was collected, dried over anhydrous sodium sulfate and concentrated by column chromatography to afford the target molecules (1.2 g, 68 %).

Step II: Preparation of 6-methoxy-3,3-dimethyl-3,4-dihydroisoquinolin-1(2H)-one (26-2)

Except for that the intermediate 26-1 in this step replaced the intermediate 23-2 in Step III of Example 23, the title compound was prepared according to a similar method to that described in Step III of Example 23, with the yield of 15%.

Step III: Preparation of 6-hydroxyl-3,3-dimethyl-3,4-dihydroisoquinolin-1(2H)-one (26-3)

Except for that the intermediate 26-2 in this step replaced the 6-methoxy-2,3-dihydro-1-indenone in Step I of Example 25, the title compound was prepared according to a similar method to that described in Step I of Example 25.

Step IV: Preparation of 6-(2-tert-butoxycarbonylaminomethyl-3-fluoroallyloxy)-3,3-dimethyl-3,4-dihydroisoquinolin-1(2H)-one (26-4)

Except for that the intermediate 26-3 in this step replaced the intermediate 1-1 in Step II of Example 1, the title compound was prepared according to a similar method to that described in Step II of Example 1.

Step V: Preparation of 6-(2-aminomethyl-3-fluoroallyloxy)-3,3-dimethyl-3,4-dihydroisoquinolin-1(2H)-one trifluoroacetate (TM38)

Except for that the intermediate 26-4 in this step replaced the intermediate 1-2 in Step III of Example 1, the title compound was prepared according to a similar method to that described in Step III of Example 1.
The total yield of Step III to Step V is 46%.

Method of resolution of 6-((2-aminomethyl-3-fluoroallyl)oxy)-3,3-dimethyl-3,4-dihydroisoquinolin-1(2H)-one trifluoroacetate isomers

A method similar to that as described in Example 9 is applied to resolute the isomers, wherein elution gradient: 0-7.4 min: 10%-28.5% B, flow rate: 16 mL/min; 7.5-9 min: 95% B, flow rate: 20 mL/min; 9.1-11 min: 10% B, flow rate: 20 mL/min. The eluent liquid having a retention time from 5.6 to 6.2 min was collected and lyophilized to afford the TM38-E, and the eluent liquid having a retention time from 6.4 to 6.9 min was collected and lyophilized to afford the TM38-Z.

(Z)-6-((2-(methylamino)-3-fluoroallyl)oxy)-3,3-dimethyl-3,4-dihydroisoquinolin-1(2H)-one trifluoroacetate (TM38-Z).

MS m/z (ESI): 279 [M+H]+
$^1$HNMR (400 MHz, $CD_3OD$) δ: 7.89 (d, *J* = *8.4* Hz, 1H), 7.11 (d, *J* = 80.8 Hz, 1H), 7.01-6.98 (m, 1H), 6.92-6.91 (m, 1H), 4.87 (s, 2H), 3.69 (d, *J* = 2.4 Hz, 2H), 2.92 (s, 2H), 1.29 (s, 6H).

(E)-6-((2-(methylamino)-3-fluoroallyl)oxy)-3,3-dimethyl-3,4-dihydroisoquinolin-1(2H)-one trifluoroacetate (TM38-E).

MS m/z (ESI): 279 [M+H]+
$^1$HNMR (400 MHz, $CD_3OD$) δ: 7.89 (d, *J* = 8.8 Hz, 1H), 7.24 (d, *J* = 81.2 Hz, 1H), 6.98 (dd, *J* = 8.8 Hz, 2.4 Hz, 1H), 6.90-6.89 (m, 1H), 4.67 (d, *J* = 2.8 Hz, 2H), 3.82 (s, 2H), 2.92 (s, 2H), 1.29 (s, 6H).

Example 27: Preparation of 6-((2-aminomethyl-3-fluoroallyl)oxy)-2-methyl-3,4-dihydroisoquinolin-1(2H)-one trifluoroacetate (TM66)

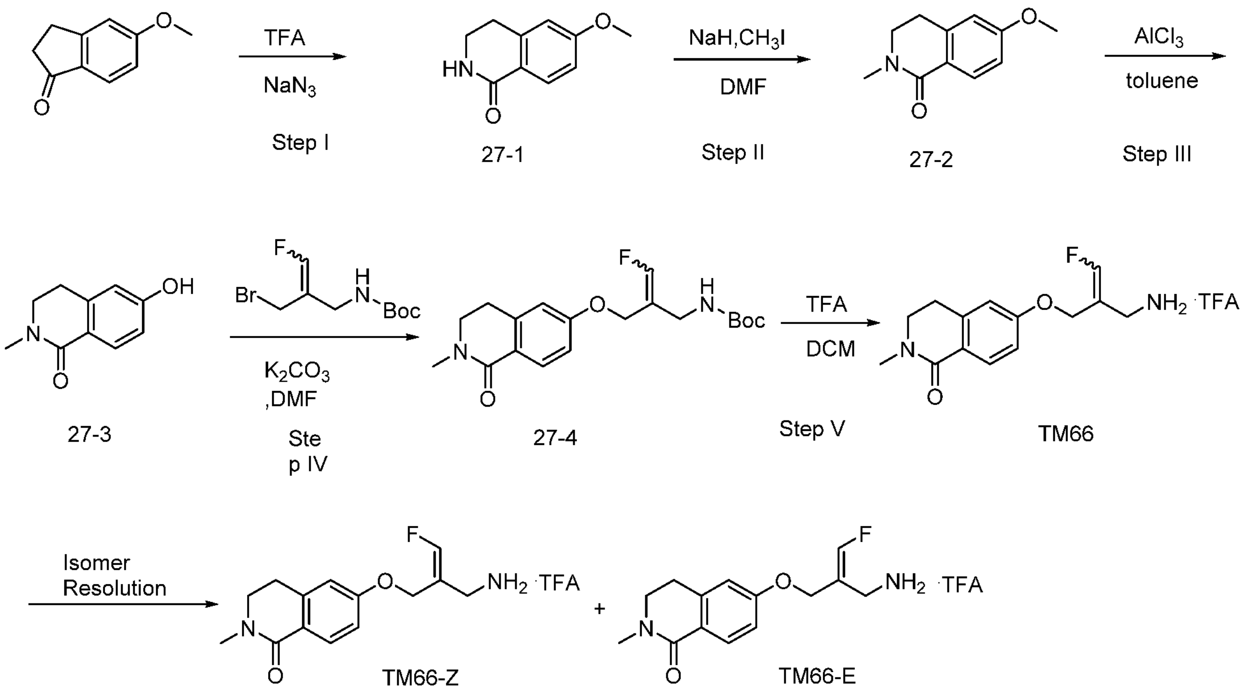


Step I: Preparation of 6-methoxy-3,4-dihydroisoquinolin-1(2H)-one (27-1)

Except for that 5-methoxy-1-indenone in this step replaced the intermediate 23-2 in Step III of Example 23, the title compound was prepared according to a similar method to that described in Step III of Example 23, with the total yield of 48%.

Step II: Preparation of 6-methoxy-2-methyl-3,4-dihydroisoquinolin-1(2H)-one (27-2)

The compound 27-1 (250 mg, 1.41 mmol) was dissolved in DMF (10 mL), and in ice bath, added with NaH (84 mg, 2.11 mmol, 60%), after reacting for 10 min, the mixture was dripped with iodomethane (300 mg, 2.11 mmol) and

warmed slowly to room temperature, then further reacted for 2 hours. LCMS monitored the completion of the reaction, the reaction solution was extracted by adding saturated saline solution and ethyl acetate. The organic phase was washed twice by saturated saline solution. The organic phases were combined, dried and concentrated to dryness, to afford the brown liquid product 27-2 (225 mg, 83%).

Step III: Preparation of 6-hydroxyl-2-methyl-3,4-dihydroisoquinolin-1(2H)-one (27-3)

Except for that the intermediate 27-2 in this step replaced the 6-methoxy-2,3-dihydro-1-indenone in Step I of Example 25, the title compound was prepared according to a similar method to that described in Step I of Example 25.

Step IV: Preparation of 6-(2-tert-butoxycarbonylaminomethyl-3-fluoroallyloxy)-2-methyl-3,4-dihydroisoquinolin-1(2H)-one (27-4)

Except for that the intermediate 27-3 in this step replaced the intermediate 1-1 in Step II of Example 1, the title compound was prepared according to a similar method to that described in Step II of Example 1.

Step V: Preparation of 6-((2-aminomethyl-3-fluoroallyl)oxy)-2-methyl-3,4-dihydroisoquinolin-1(2H)-one trifluoroacetate (TM66)

Except for that the intermediate 27-4 in this step replaced the intermediate 1-2 in Step III of Example 1, the title compound was prepared according to a similar method to that described in Step III of Example 1.
The total yield of Step III to Step V is 77%.

Method of resolution of 6-((2-aminomethyl-3-fluoroallyl)oxy)-2-methyl-3,4-dihydroisoquinolin-1(2H)-one trifluoroacetate isomers

A method similar to that as described in Example 9 is applied to resolute the isomers, wherein elution gradient: 0-7.8 min: 10%-24.6% B, flow rate: 16 mL/min; 7.9-10 min: 95% B, flow rate: 20 mL/min; 10.1-12 min: 10% B, flow rate: 20 mL/min. The eluent liquid having a retention time from 5.6 to 6.2 min was collected and lyophilized to afford the TM66-E, and the eluent liquid having a retention time from 6.8 to 7.3 min was collected and lyophilized to afford the TM66-Z.

(Z)-6-((2-(methylamino)-3-fluoroallyl)oxy)-2-methyl-3,4-dihydroisoquinolin-1(2H)-one trifluoroacetate (TM66-Z)

MS m/z (ESI): 265 $[M+H]^+$
$^1$HNMR (400 MHz, $CD_3OD$) δ: 7.89 (d, *J* = 8.8 Hz, 1H), 7.10 (d, *J* = 80.8 Hz, 1H), 6.98-6.96 (m, 1H), 6.90 (d, *J* = 2.4 Hz, 1H), 4.86 (d, *J* = 2.4 Hz, 2H), 3.69 (d, *J* = 2.0 Hz, 2H), 3.60 (t, *J* = 6.8 Hz, 2H), 3.12 (s, 3H), 3.01 (t, *J* = 6.8 Hz, 2H).

(E)-6-((2-(methylamino)-3-fluoroallyl)oxy)-2-methyl-3,4-dihydroisoquinolin-1(2H)-one trifluoroacetate (TM66-E)

MS m/z (ESI): 265 $[M+H]^+$
$^1$HNMR (400 MHz, $CD_3OD$) δ: 7.89 (d, *J* = 8.4 Hz, 1H), 7.24 (d, *J* = 81.2 Hz, 1H), 6.96 (dd, *J* = 8.8 Hz, 2.4 Hz,1H), 6.88 (d, *J* = 2.4 Hz, 1H), 4.66 (d, *J* = 2.8 Hz, 2H), 3.82 (s, 2H), 3.60 (t, *J* = 6.8 Hz, 2H), 3.12 (s, 3H), 3.01 (t, *J* = 6.8 Hz, 2H).

Example 28: Preparation of 7-((2-aminomethyl-3-fluoroallyl)oxy)-3,4-dihydroisoquinolin-1(2H)-one trifluoroacetate (TM75)

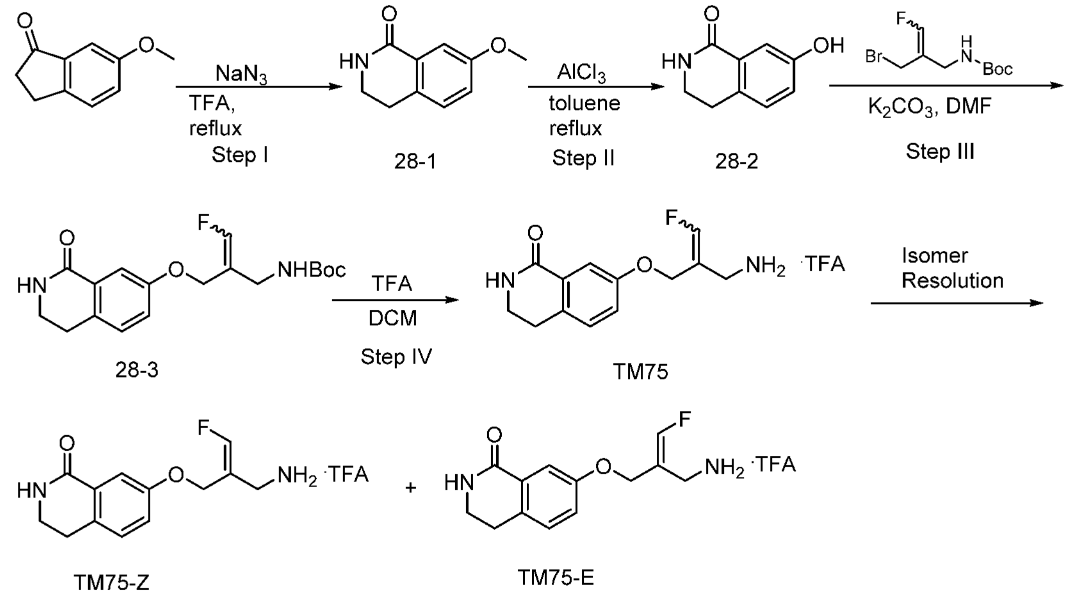


Step I: Preparation of 7-methoxy-3,4-dihydroisoquinolin-1(2H)-one (28-1)

Except for that 6-methoxy-1-indanone in this step replaced the intermediate 23-2 in Step III of Example 23, the title compound was prepared according to a similar method to that described in Step III in Example 23, with the total yield of 49%.

Step II: Preparation of 7-hydroxyl-3,4-dihydroisoquinolin-1(2H)-one (28-2)

Except for that the intermediate 28-1 in this step replaced the 6-methoxy-2,3-dihydro-1-indenone in Step I of Example 25, the title compound was prepared according to a similar method to that described in Step I in Example 25, with the total yield of 67%.

Step III: Preparation tert-butyl (2-(1-oxo-1,2,3,4-tetrahydroisoquinoline-7-oxymethyl)-3-fluoroallyl)carbamate (28-3)

Except for that in the step, the intermediate 1-1 in Step II of Example 1 was replaced by the intermediate 28-2, the title compound was prepared according to a similar method to that described in Step II of Example 1, with the yield of 53%.

Step IV: Preparation of 7-((2-aminomethyl-3-fluoroallyl)oxy)-3,4-dihydroisoquinolin-1(2H)-one trifluoroacetate (TM75)

Except for that the intermediate 28-3 in this step replaced the intermediate 1-2 in Step III of Example 1, the title compound was prepared according to a similar method to that described in Step III of Example 1, with the yield of about 100%.

A method similar to that as described in Example 9 is applied to resolute the isomers, the retention time of the TM75-E is earlier than that of the TM75-Z

(Z)-7-((2-aminomethyl-3-fluoroallyl)oxy)-3,4-dihydroisoquinolin-1(2H)-one trifluoroacetate (TM75-Z)

MS m/z (ESI): 251 $[M+H]^+$
$^1$HNMR (400 MHz, $CD_3OD$) δ: 7.07 (d, *J* = 81.2 Hz, 1H), 7.11 (d, *J* = 8.0 Hz, 1H), 6.64 (dd, *J* = 8.4 Hz, 2.8 Hz, 1H) 6.53 (d, *J* = 2.8 Hz, 1H), 4.77 (d, *J* = 2.0 Hz, 2H), 3.67 (d, *J* = 2.4 Hz, 2H), 2.88 (t, *J* = 7.6 Hz, 2H), 2.53 (t, *J* = 7.6 Hz, 2H).

(E)-7-((2-aminomethyl-3-fluoroallyl)oxy)-3,4-dihydroisoquinolin-1(2H)-one trifluoroacetate (TM75-E)

MS m/z (ESI): 251 $[M+H]^+$
$^1$HNMR (400 MHz, $CD_3OD$) δ: 7.18 (d, *J* = 81.8 Hz, 1H), 7.09 (d, *J* = 8.4 Hz, 1H), 6.63 (dd, *J* = 8.4 Hz, 2.4 Hz, 1H), 6.52 (d, *J* = 2.4 Hz, 1H), 4.56 (d, *J* = 3.6 Hz, 2H), 3.81 (s, 2H), 2.86 (t, *J* = 7.6 Hz 2H), 2.51 (t, *J* = 7.6 Hz, 2H).

Example 29: Preparation of N-(tetrahydro-2H-pyran-4-yl)-4-((2-aminomethyl-3-fluoroallyl)oxy)-benzamide trifluoroacetate (TM76)

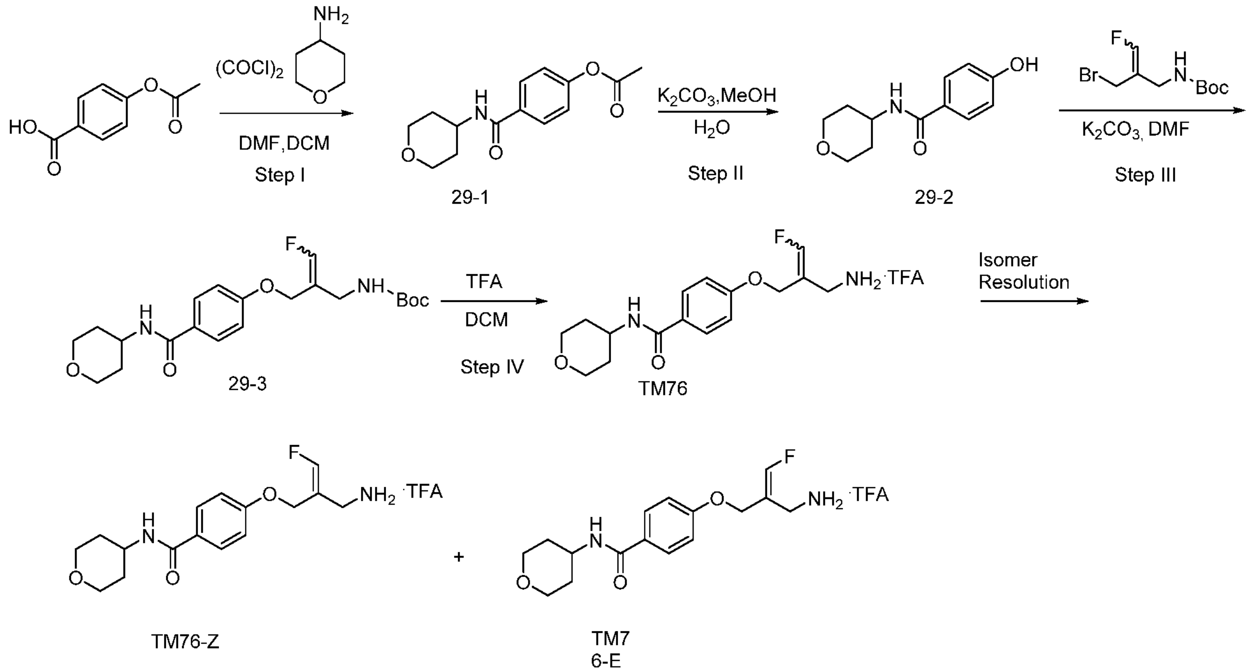


29-1 29-2 29-3 TM76 TM76-Z TM76-E

Step I: Preparation of 4-((tetrahydro-2*H*-pyran-4-yl)aminoformyl)phenyl acetate(29-1)

4-acetoxybenzoic acid (500 mg, 2.78 mmol) was dissolved in anhydrous dichloromethane (10 mL) and DMF (10 mL), and in ice bath, added with oxalyl chloride (1.06 g, 8.34 mmol), and the mixture was reacted for half an hour. The reaction solution was then concentrated for further use. 4-aminotetrahydro-2*H*-pyran (336 mg, 3.33 mmol) was dissolved in DMF and added with the above prepared acyl chloride solution, and the mixture was reacted at room temperature. LCMS was used to monitor the reaction. After 5 hours, the reaction solution was added with water and extracted with ethyl acetate. The organic phase was dried and concentrated, then subject to column chromatography separation to afford the title compound, which was a brown liquid (225 mg, 31%).

Step II: Preparation of 4-hydroxyl-N-(tetrahydro-2*H*-pyran-4-yl)benzamide (29-2)

The compound 29-1 (225 mg, 0.86 mmol) was dissolved in 10 mL anhydrous methanol and added with 1ml water and potassium carbonate (354 mg, 2.58 mmol), and the mixture was reacted at room temperature for 1 hour. The reaction solution was then concentrated, added with water, and extracted with ethyl acetate. The organic phase was concentrated to dryness to afford the title compound, which was an oily liquid (125 mg, 66%).

Step III: Preparation of N-(tetrahydro-2*H*-pyran-4-yl)-4-((2-tert-butoxycarbonyl aminomethyl-3-fluoroallyl)oxy)-benzamide (29-3)

Except for that the intermediate 29-2 in this step replaced the intermediate 1-1 in Step II of Example 1, the title compound was prepared according to a similar method to that described in Step II of Example 1, with the yield of 42%.

Step IV: Preparation of N-(tetrahydro-2*H*-pyran-4-yl)-4-((2-aminomethyl-3-fluoroallyl)oxy)-benzamide trifluoroacetate (TM76)

Except for that the intermediate 29-3 in this step replaced the intermediate 1-2 in Step III of Example 1, the title compound was prepared according to a similar method to that described in Step III of Example 1, with the yield of 99%.

A method similar to that as described in Example 9 is applied to resolute the isomers, the retention time of the TM76-E is earlier than that of the TM76-Z

(Z)-N-(tetrahydro-2*H*-pyran-4-yl)-4-((2-aminomethyl-3-fluoroallyl)oxy)-benzamide trifluoroacetate (TM76-Z)

MS m/z (ESI): 309 $[M+H]^+$
$^1$HNMR (400 MHz, $CD_3OD$) δ: 7.83 (d, *J* = 9.2 Hz, 2H), 7.10 (d, *J* = 80.8 Hz, 1H), 7.08 (d, *J* = 8.8 Hz, 2H), 4.86 (d, *J* = 2.4 Hz, 2H), 4.12-4.06 (m, 1H), 3.98 (dd, *J* = 8.0 Hz, 2.4 Hz, 2H), 3.69 (s, 2H), 3.52 (td, *J* = 12.0 Hz, 2.0 Hz, 2H), 1.89-1.86 (m, 2H), 1.71-1.61 (m, 2H).

(E)-N-(tetrahydro-2*H*-pyran-4-yl)-4-((2-aminomethyl-3-fluoroallyl)oxy)-benzamide trifluoroacetate (TM76-E)

MS m/z (ESI): 309 $[M+H]^+$
$^1$HNMR (400 MHz, $CD_3OD$) δ: 7.83 (d, *J* = 8.8 Hz, 2H), 7.24 (d, *J* = 80.8 Hz, 1H), 7.06 (d, *J* = 8.8 Hz, 2H), 4.66 (d, *J* = 3.6 Hz, 2H), 4.12-4.06 (m, 1H), 3.98 (dd, J = 12.0 Hz, 2.4 Hz, 2H), 3.82 (s, 2H), 3.52 (td, 12.0 Hz, 2.0 Hz, 2H), 1.90-1.86 (m, 2H), 1.71-.61 (m, 2H).

Example 30: Preparation of 4-((2-aminomethyl-3-fluoroallyl)oxy)-N-(pyridin-2-ylmethyl)benzamide trifluoroacetate (TM112)

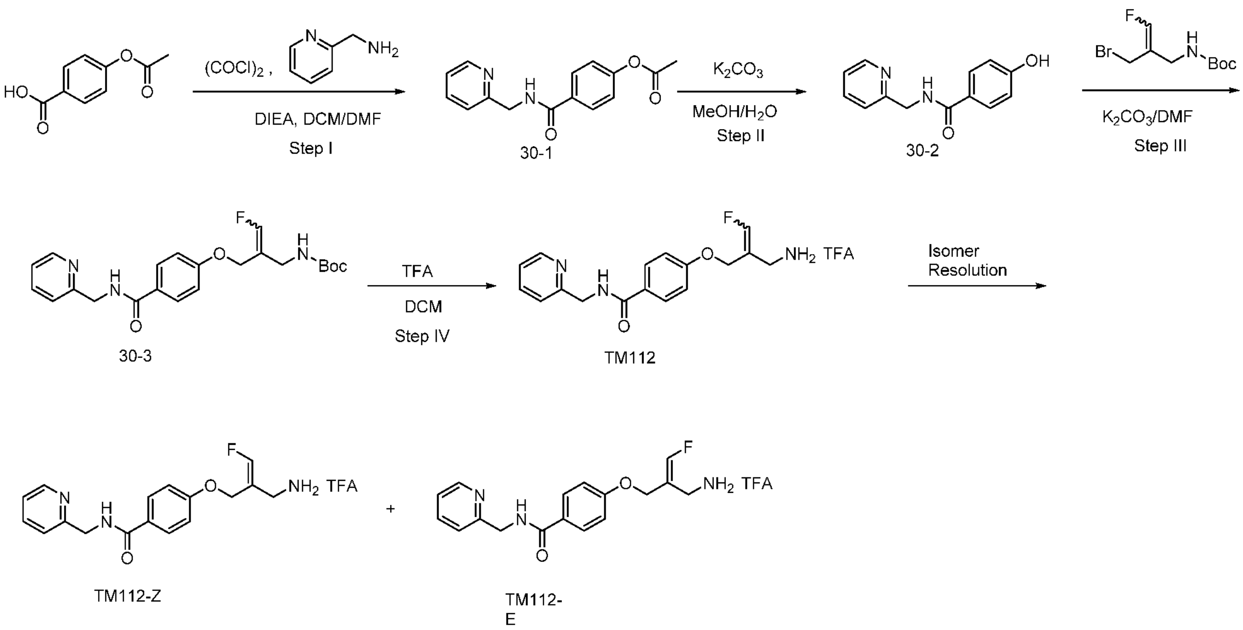


Step I: Preparation of 4-((pyrid-2-yl-methyl)aminoformyl)phenyl acetate (30-1)

The compound 4-acetoxybenzoic acid (2.00 g, 11.1 mmol) was dissolved in dichloromethane (24 mL) and dripped with oxalyl chloride (2.11 g, 16.65 mmol) and DMF (5 drops), the mixture was reacted at room temperature for 3 hours. Supplementary Oxalyl chloride (1.50 g, 11.81 mmol) was added, the mixture was reacted at room temperature for 3 hours, and then subjected to dryness by rotary evaporation for further use. The compound 2-aminomethylpyridine (0.5 g, 4.62 mmol) was dissolved in dichloromethane (10 mL) and added with DIPEA (1.79 g, 13.86 mmol) and the above prepared acryl chloride (0.92 g, 4.62 mmol), the mixture was reacted at room temperature for 6 hours. The reaction was quenched by adding water, and the reaction solution was extracted twice with dichloromethane, the organic phase was washed with saturated saline solution. The organic phase was collected, dried over anhydrous sodium sulfate, concentrated and purified by column chromatography. The crude product was directly used in the next step.

Step II: Preparation of 4-hydroxyl-N-(pyridin-2-methyl)benzamide (30-2)

The crude product of the compound 30-1 was dissolved in methanol (20 mL) and added with water (20 mL) and potassium carbonate (2.50 g, 18.10 mmol), the mixture was reacted at room temperature overnight. The reaction mixture was diluted with water, and requlated with dilute hydrochloric acid to pH = about 3. A saturated sodium bicarbonate was further added to regulate the pH = about 8. The resulting mixture was extracted twice with ethyl acetate. The organic

phase was washed with saturated saline solution. The organic phase was collected, dried over anhydrous sodium sulfate and concentrated and purified by column chromatography, to afford the target product (0.20 g, the yield of the two steps is 19 %).

Step III: Preparation of 4-((2-tert-butoxycarbonylaminomethyl-3-fluoroallyl)oxy)-N-(pyridin-2-ylmethyl)benzamide (30-3)

Except for that the intermediate 30-2 in this step replaced the intermediate 1-1 in Step II of Example 1, the title compound was prepared according to a similar method to that described in Step II of Example 1, with the yield of 56%.

Step IV: Preparation of 4-((2-aminomethyl-3-fluoroallyl)oxy)-N-(pyridin-2-ylmethyl)benzamide trifluoroacetate (TM112)

Except for that the intermediate 30-3 in this step replaced the intermediate 1-2 in Step III of Example 1, the title compound was prepared according to a similar method to that described in Step III in Example 1, with the total yield of 99%.

A method similar to that as described in Example 9 is applied to resolute the isomers, the retention time of the TM112-E is earlier than that of the TM112-Z

(Z)-4-((2-aminomethyl-3-fluoroallyl)oxy)-N-(pyridin-2-ylmethyl)benzamide trifluoroacetate (TM112-Z)

MS m/z (ESI): 316 $[M+H]^+$
$^1$HNMR (400 MHz, $CD_3OD$) $\delta$: 8.72 (d, *J* = 4.8 Hz, 1H), 8.42 (td, *J* = 8.0 Hz, 1.6 Hz, 1H), 7.93-7.91 (m, 3H), 7.85-7.82 (m, 1H), 7.11 (d, *J* = 80.8 Hz, 1H), 7.14-7.12 (m, 2H), 4.88 (d, *J* = 2.4 Hz, 2H), 4,84 (s, 2H), 3.70 (d, *J* = 2.0 Hz, 2H).

(E)-4-((2-aminomethyl-3-fluoroallyl)oxy)-N-(pyridin-2-ylmethyl)benzannide trifluoroacetate (TM112-E)

MS m/z (ESI): 316 $[M+H]^+$
$^1$HNMR (400 MHz, $CD_3OD$) $\delta$: 8.72 (d, *J* = 5.6 Hz, 1H), 8.42 (td, *J* = 8.0 Hz, 1.6 Hz, 1H), 7.93-7.90 (m, 3H), 7.86-7.82 (m, 1H), 7.24 (d, *J* = 80.8 Hz, 1H), 7.13-7.09 (m, 2H), 4.84 (s, 2H), 4.68 (d, *J* = 3.2 Hz, 2H), 3.83 (d, *J* = 1.2 Hz, 2H).

Example 31: Preparation of 4-((2-aminomethyl-3-fluoroallyl)oxy)-N-(2,3-dihydrobenzo[b][1,4]dioxa-6-yl)benzamide trifluoroacetate (TM114)

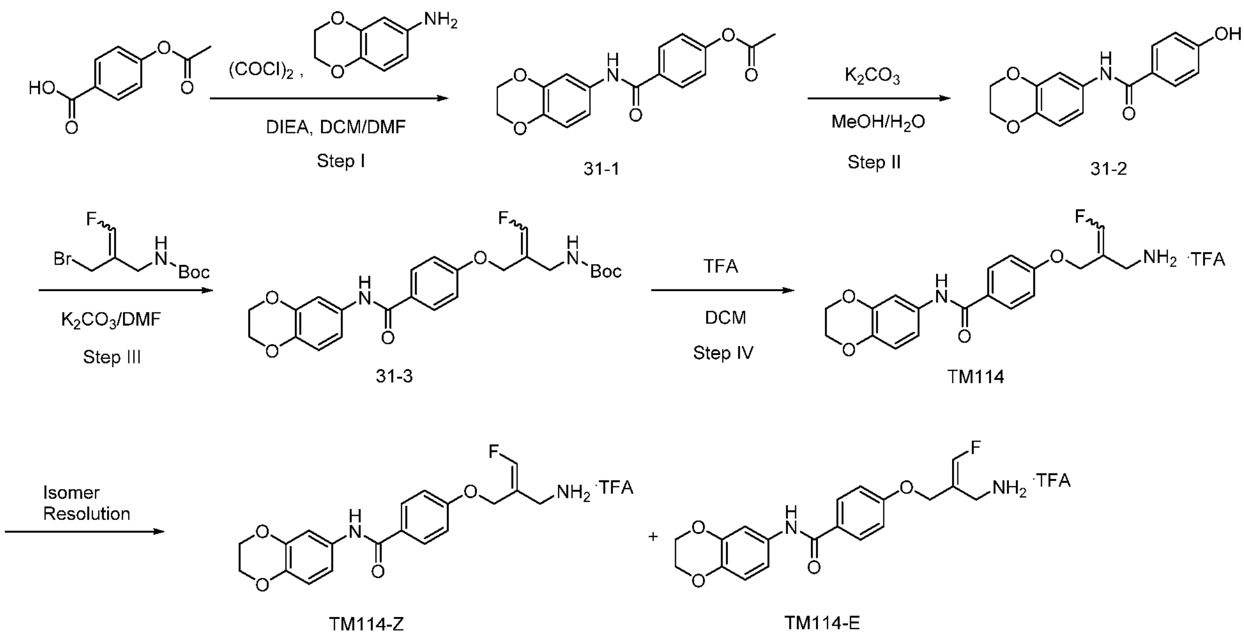


Except for that 6-amino-1,4-benzodioxane in step I of the example replaced the 2-aminomethylpyridine in Step I of Example 30, the title compound was prepared according to a similar method to that described in Example 30, with the total yield of 9%.

A method similar to that as described in Example 9 is applied to resolute the isomers, the retention time of the TM114-E is earlier than that of the TM114-Z

(Z)-4-((2-aminomethyl-3-fluoroallyl)oxy)-N-(2,3-dihydrobenzo[b][1,4]dioxa-6-yl)benzamide trifluoroacetate (TM114-Z)

MS m/z (ESI): 359 $[M+H]^+$
$^1$HNMR (400 MHz, $CD_3OD$) δ: 7.92 (d, *J* = 8.8 Hz, 2H), 7.26-7.25 (m, 1H), 7.12 (d, *J* = 8.8 Hz, 2H), 7.11 (d, *J* = 80.8 Hz, 1H), 7.05-7.03 (m, 1H), 6.79 (d, *J* = 8.8 Hz, 1H), 4.88 (s, 2H), 4.25-4.22 (m, 4H), 3.70 (d, *J* = 2.4 Hz, 2H).

(E)-4-((2-aminomethyl-3-fluoroallyl)oxy)-N-(2,3-dihydrobenzo[b][1,4]dioxa-6-yl)benzamide trifluoroacetate (TM114-E)

MS m/z (ESI): 359 $[M+H]^+$
$^1$HNMR (400 MHz, $CD_3OD$) δ: 7.91 (d, *J* = 8.8 Hz, 2H), 7.25 (d, *J* = 80.8 Hz, 1H), 7.26-7.25 (m, 1H), 7.10 (d, *J* = 8.8 Hz, 2H), 7.04 (dd, *J* = 8.8 Hz, 2.4 Hz, 1H), 6.80 (d, *J* = 8.8 Hz, 1H), 4.68 (d, *J* = 3.2 Hz, 2H), 4.25-4.22 (m, 4H), 3.83 (s, 2H).

Example 32: Preparation of 4-((2-aminomethyl-3-fluoroallyl)oxy)-N-(3-(2-methoxyethoxy)phenyl)benzamide trifluoroacetate (TM106)

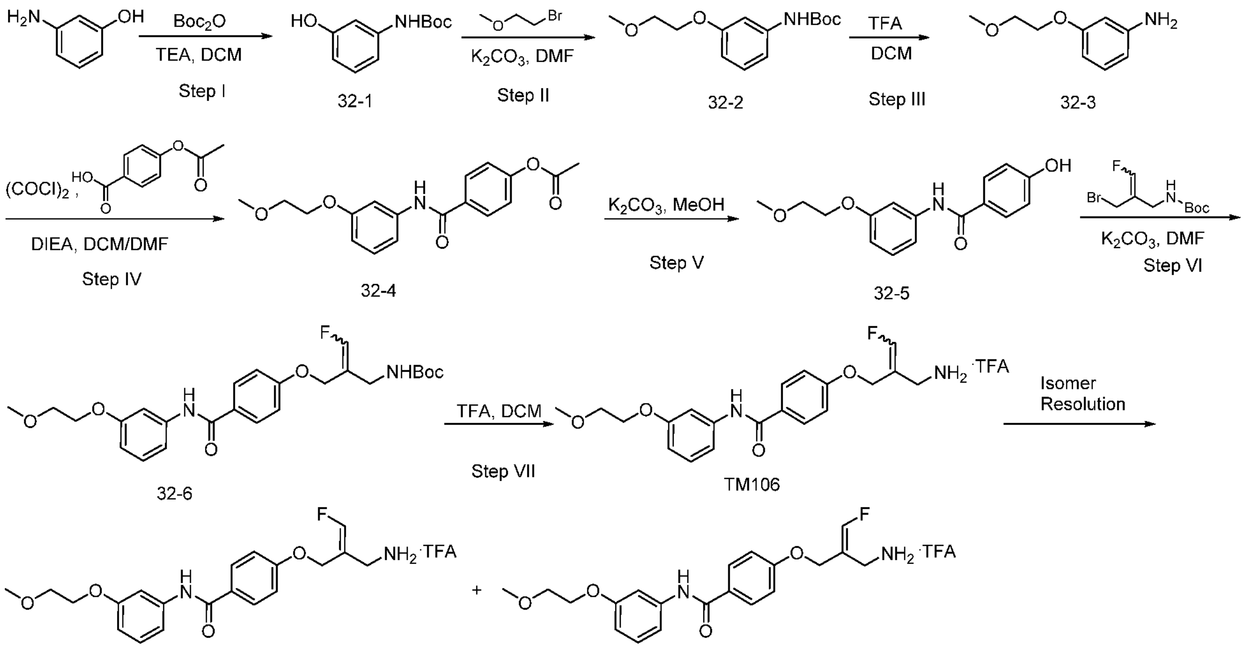

TM106-Z TM106-E

Step I: Preparation of N-tert-butoxycarbonyl-3-aminophenol (32-1)

The compound 3-aminophenol (10.0 g, 91.7 mmol) was placed in a reaction flask, and successively added with DCM (100 mL), TEA (27.8 g, 275.1 mmol) and $Boc_2O$ (21.8 g, 100.0 mmol), the resulting mixture was reacted at room temperature for 3 hours. The mixture was concentrated at low temperature, then added with water, the mixture was extracted with ethyl acetate. The organic phases were combined, washed with saturated saline solution, dried and concentrated to afford a crude product, which was purified by column chromatography to afford the title compound (3.8 g, 20%).

Step II: Preparation of N-tert-butoxycarbonyl-3-(2-methoxyethoxy)aniline (32-2)

The compound 32-1 (3.8 g, 18.2 mmol) was dissolved DMF (10 mL), and added with $K_2CO_3$ (7.5 g, 54.6 mmol) and 2-bromoethylmethylether (2.8 g, 20.0 mmol), and the mixture was reacted at room temperature for 10 hours. After the reaction solution was diluted by adding water, it was extracted with ethyl acetate. After the organic phase was washed with water, it was washed with a saturated saline solution three times, and then it was dried over sodium sulfate and

concentrated by filtration. The crude product was purified by a column chromatograph to afford the title compound (2.9 g, 60%).

Step III: Preparation of 3-(2-methoxyethoxy)aniline (32-3)

The compound 32-2 (2.9 g, 10.8 mmol) was dissolved in DCM (10 mL), and at low temperature, added with TFA (3 mL), the mixture was reacted at room temperature for 1 hour. After TLC monitored the completion of the reaction, the reaction solution was concentrated at low temperature. The crude product was dissolved with water, then alkalized with saturated sodium carbonate, and extracted with ethyl acetate. The organic phases were combined, washed with saturated saline solution three times, dried over sodium sulfate and concentrated by filtration, to afford the title compound. The title compound was directly used in the text step without any further purification.

Step IV: Preparation of 4-((3-(2-methoxyethoxy)phenyl)carbamoyl)phenyl acetate (32-4)

Except for that the intermediate 32-3 in this step replaced the 2-aminomethylpyridine in Step I of Example 30, the title compound was prepared according to a similar method to that described in Step I in Example 30, with the total yield of 49%.

Step V: Preparation of 4-hydroxyl-N-(3-(2-methoxyethoxy)phenyl)benzamide (32-5)

Except for that the intermediate 32-4 in this step replaced the intermediate 30-1 in Step II of Example 30, the title compound was prepared according to a similar method to that described in Step II of Example 30, with the yield of 91%.

Step VI: Preparation of 4-((2-tert-butoxycarbonylaminomethyl-3-fluoroallyl)oxy)-N-(3-(2-methoxyethoxy)phenyl)benzamide (32-6)

Except for that the intermediate 32-5 in this step replaced the intermediate 1-1 in Step II of Example 1, the title compound was prepared according to a similar method to that described in Step II of Example 1, with the yield of 37%.

Step VII: Preparation of 4-((2-aminomethyl-3-fluoroallyl)oxy)-N-(3-(2-methoxyethoxy)phenyl)benzamide trifluoroacetate (TM106)

Except for that the intermediate 32-6 in this step replaced the intermediate 1-2 in Step III of Example 1, the title compound was prepared according to a similar method to that described in Step III of Example 1, with the yield of 99%.

A method similar to that as described in Example 9 is applied to resolute the isomers, the retention time of the TM106-E is earlier than that of the TM106-Z.

(Z)-4-((2-aminomethyl-3-fluoroallyl)oxy)-N-(3-(2-methoxyethoxy)phenyl)benzamide trifluoroacetate (TM106-Z)

MS m/z (ESI): 375 $[M+H]^+$
$^1$HNMR (400 MHz, $CD_3OD$) $\delta$: 7.94 (d, *J* = 8.8 Hz, 2H), 7.40 (s, 1H), 7.25-7.23 (m, 2H), 7.12 (d, *J* = 8.8 Hz, 2H), 7.11 (d, *J* = 80.8 Hz, 1H), 6.75-6.72 (m, 1H), 4.89 (s, 2H), 4.13-4.11 (m, 2H), 3.76-3.74 (m, 2H), 3.70 (d, *J* = 2.0 Hz, 2H), 3.43 (s, 3H).

(E)-4-((2-aminomethyl-3-fluoroallyl)oxy)-N-(3-(2-methoxyethoxy)phenyl)benzamide trifluoroacetate (TM106-E)

MS m/z (ESI): 375 $[M+H]^+$
$^1$HNMR (400 MHz, $CD_3OD$) $\delta$: 7.93 (d, *J* = 8.8 Hz, 2H), 7.40 (s, 1H), 7.23 (d, *J* = 81.2 Hz, 1H), 7.25-7.23 (m, 2H), 7.10 (d, *J* = 8.8 Hz, 2H), 6.75-6.72 (m, 1H), 4.68 (d, *J* = 3.6 Hz, 2H), 4.13-4.11 (m, 2H), 3.83 (s, 2H), 3.76-3.74 (m, 2H), 3.43 (s, 3H).

Example 33: Preparation of (E)-6-((2-(aminomethyl)-3-fluoroallyl)oxy)-3,3-dimethyl-3,4-dihydroisoquinolin-1(2H)-one hydrochloride (HC38-E)

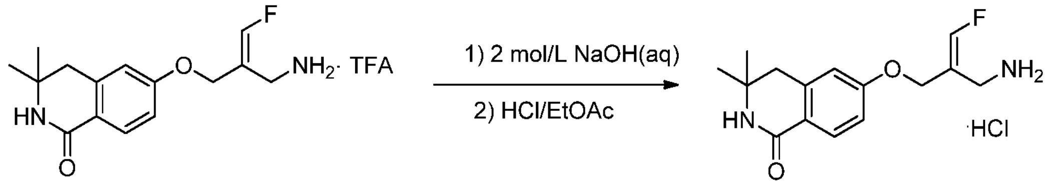


TM-38-E HC38-E

The collected solution of the compound TM-38-E (1.74 g, 5.8 mmol) after isomer resolution was added with 2 mol/L sodium hydroxide solution to pH=9-10 and extracted with DCM twice, and then the organic phases were combined. The organic phase was washed with a saturated saline solution and dried. Then the organic phase was added with 10 mL of 2 mol/L hydrogen chloride solution in ethyl acetate and concentrated to afford the title compound (1.44 g, 98%).

(E)-6-((2-aminomethyl-3-fluoroallyl)oxy)-3,3-dimethyl-3,4-dihydroisoquinolin-1(2H)-one hydrochloride (HC38-E)

MS m/z (ESI): 279 $[M+H]^+$
$^1$HNMR (400 MHz, $CD_3OD$) $\delta$: 7.88 (d, $J$ = 8.4 Hz, 1H), 7.24 (d, $J$ = 81.2 Hz, 1H), 6.97 (dd, $J$ = 8.4 Hz, 2.4 Hz, 1H), 6.91-6.90 (m, 1H), 4.68 (d, $J$ = 2.8 Hz, 2H), 3.83 (d, $J$ = 2.0 Hz, 2H), 2.92 (s, 2H), 1.29 (s, 6H).

Example 34: Preparation of (E)-6-((2-aminomethyl-3-fluoroallyl)oxy)-2-methyl-3,4-dihydroisoquinolin-1(2H)-one hydrochloride (HC66-E)

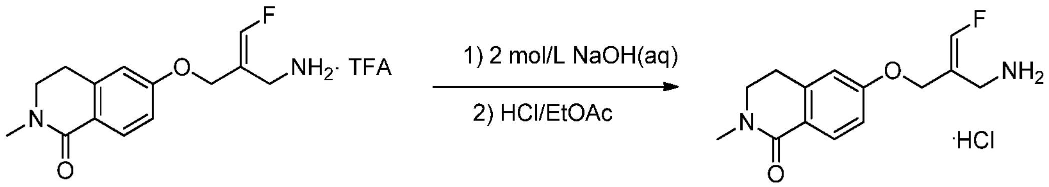


TM-66-E HC66-E

The collected solution of the compound TM-66-E (4.2 g, 11.6 mmol) after isomer resolution was added with 2 mol/L sodium hydroxide solution to pH=9-10, and extracted with DCM twice, and then the organic phases were combined. The organic phase was washed with saturated saline solution and dried. The organic phase was added with 30 mL of a 2 mol/L hydrogen chloride solution in ethyl acetate and concentrated to afford the title compound (2.8 g, 80%).

(E)-6-((2-aminomethyl-3-fluoroallyl)oxy)-2-methyl-3,4-dihydroisoquinolin-1(2H)-one hydrochloride (HC66-E)

MS m/z (ESI): 265 $[M+H]^+$
$^1$HNMR (400 MHz, $CD_3OD$) $\delta$: 7.88 (d, $J$ = 8.4 Hz, 1H), 7.24 (d, $J$ = 81.2 Hz, 1H), 6.97 (dd, $J$ = 8.4 Hz, 2.4 Hz,1H), 6.90 (s, 1H), 4.67 (s, 2H), 3.82 (s, 2H), 3.60 (t, $J$ = 6.8 Hz, 2H), 3.12 (s, 3H), 3.00 (t, $J$ = 6.8 Hz, 2H).

Experimental Example 1: Inhibition experiment against the enzymatic activity of VAP-1(Vascular adhesion protein 1) in vitro

Reagent:

Test kit: Amplex® Red Monoamine Oxidase Assay, Manufacturer: ThermoFisher
Reaction buffer solution: 50 mM PBS, pH 7.4
Enzyme: VAP-1, Manufacturer: R&D Systems

VAP-1 protein (about 20 nM) and different concentrations of the test compound were pre-incubated at room temperature for 10 minutes, followed by an addition of 500 μM benzylamine hydrochloride, 1 U/mL HRP and 200 μM Amplex Red reagent according to the specification of the test kit to start the reaction. The reaction plate was placed in the enzyme marker, and in kinetic mode, fluorescence signals were measured at Ex/Em=540/590 nm. The relative inhibition activity of each concentration group was calculated, wherein the ratio (slope) of fluorescence signal to reaction

time in the linear range is the reaction rate, the vehicle group (DMSO) is the negative control, and the reaction buffer solution group (without enzymes and the compounds) is the blank control. The half inhibition concentration (IC50) of the compound was calculated according to four-parameter model fitting curve.

Experimental Results

According to the above method, the inhibition of the compound against the activity of VAP-1 was measured, and the results were shown in Table 1:

Table 1. Results of inhibition against the enzymatic activity of VAP-1

| Compound No. | $IC_{50}$ (nM) |
|---|---|
| TM70 | 0.2 ± 0.0 |
| TM1 | 0.5 ± 0.0 |
| TM68 | 9.9 ± 1.8 |
| TM67 | 11.5 ± 1.3 |
| TM8 | 2.2 ± 0.1 |
| TM62 | 1.8 ± 0.2 |
| TM26 | 5.8 ± 1.0 |
| TM42 | 1.6 ± 0.2 |
| TM41 | 4.8 ± 0.7 |
| TM39 | 1.0 ± 0.1 |
| TM33 | 3.3 ± 0.2 |
| TM48 | 6.5 ± 0.5 |
| TM61 | 0.5 ± 0.0 |
| TM72 | 3.4 ± 0.5 |
| TM71 | 2.4 ± 0.2 |
| TM44 | 3.4 ± 0.4 |
| TM45 | 0.9 ± 0.1 |
| TM69 | 10.4 ± 1.4 |
| TM45-Z | 1.1 ± 0.1 |
| TM45-E | 2.4 ± 0.7 |
| TM33-Z | 6.3 ± 1.2 |
| TM33-E | 4.9 ± 0.5 |
| TM41-Z | 3.5 ± 0.4 |
| TM41-E | 3.1 ± 0.5 |
| TM48-Z | 10.6 ± 1.4 |
| TM48-E | 9.2 ± 1.1 |
| TM63-Z | 4.4 ± 0.8 |
| TM63-E | 5.7 ± 0.8 |
| TM65-Z | 1.7 ± 0.3 |
| TM65-E | 4.1 ± 0.7 |
| TM73-Z | 3.7 ± 0.3 |
| TM73-E | 4.2 ± 0.3 |

(continued)

| Compound No. | $IC_{50}$ (nM) |
|---|---|
| TM1-Z | 0.7 ± 0.1 |
| TM1-E | 1.6 ± 0.2 |
| TM42-Z | 1.5 ± 0.1 |
| TM42-E | 2.0 ± 0.3 |
| TM26-Z | 3.7 ± 0.8 |
| TM26-E | 9.9 ± 1.2 |
| TM44-Z | 9.7 ± 0.9 |
| TM44-E | 12.9 ± 4.4 |
| TM74-Z | 5.3 ± 0.2 |
| TM74-E | 3.2 ± 0.2 |
| TM39-Z | 0.7 ± 0.1 |
| TM39-E | 1.2 ± 0.2 |
| TM7-Z | 3.8 ± 0.4 |
| TM7-E | 3.0 ± 0.7 |
| TM38-Z | 2.9 ± 0.3 |
| TM38-E | 4.7 ± 1.1 |
| TM66-Z | 1.6 ± 0.2 |
| TM66-E | 2.0 ± 0.4 |
| TM75-Z | 1.3 ± 0.2 |
| TM75-E | 23.4 ± 2.2 |
| TM76-Z | 1.8 ± 0.2 |
| TM76-E | 4.3 ± 0.6 |
| TM112-Z | 6.5 ± 1.8 |
| TM112-E | 3.2 ± 0.4 |
| TM114-Z | 0.8 ± 0.1 |
| TM114-E | 1.1 ± 0.2 |
| HC38-E | 2.85 ± 0.30 |

The results show that the compounds of the invention exhibit a strong inhibition against the activity of VAP-1.

Experimental Example 2: Inhibition experiment against the enzymatic activity (ex-vivo)

Reagent:

Tissue homogenate buffer solution (20 mM HEPES, pH 7.2, 1 mM EDTA, 250 mM sucrose, PMSF 0.2 mM)
Reaction buffer solution: 50 mM PBS, pH 7.4
Test kit: Amplex® Red Monoamine Oxidase Assay, Manufacturer: ThermoFisher

Experimental method

1. Sample collection and treatment

BALBc mice were administrated orally with different doses of the test compound, and the vehicle group was used as the control group. After six hours, abdominal fat was collected and homogenized at 1:20 (w/v) with the pre-cooled homogenate buffer solution, and thereafter, the homogenate was centrifuged at 10,000 g and 4 °C or 10 minutes, the supernatant was collected and stored at -80 °C for further use.

2. Enzymatic activity measurement

A test sample was diluted with the reaction buffer solution and successively added with the inhibition solution A: 1 μM Clorgyline+1 μM pargylin, to inhibit the activities of MAO-A and MAO-B, and the inhibition solution B: 1 μM Clorgyline +1 μM Pargylin+1 μM Mofegiline as the blank control, and after being incubated at room temperature for 20 min, 500 μM benzylamine hydrochloride, 1 U/mL HRP and 200 μM Amplex Red reagent were added to start the reaction. By referring to the method in "Experiment of inhibiting the enzymatic activity of VAP-1", relative activity of each dose group was calculated, wherein the ratio of the fluorescent signal to reaction time in the linear range is the reaction rate, and the vehicle group is the positive control, thereby to study the inhibition of the compound against the enzymatic activity of VAP-1 in abdominal fat of mice.

Experimental results

According to the above method, the inhibition of the compound against the enzymatic activity of VAP-1 in abdominal fats of mouse was measured, as shown in Tables 2 and 3.

Table 2. Results of inhibition against the enzymatic activity of VAP-1 (ex-vivo)

| Compound No. | Inhibition rate (%) |
|---|---|
| | 2 mg/kg |
| TM26 | 112.3 ± 7.1 |
| TM42 | 106.2 ± 8.4 |
| TM41 | 96.1 ± 17.4 |
| TM33 | 105.1 ± 9.1 |
| TM61 | 99.7 ± 8.7 |
| TM44 | 95.8 ± 6.2 |
| TM45 | 91.9 ± 6.4 |

Table 3. Results of inhibition against the enzymatic activity of VAP-1 (ex-vivo)

| Compound No. | Inhibition rate (%) |
|---|---|
| | 0.5 mg/kg |
| TM1-Z | 93.3 ± 5.4 |
| TM1-E | 96.7 ± 2.2 |
| TM26-Z | 86.6 ± 5.5 |
| TM39-Z | 93.2 ± 3.9 |
| TM39-E | 93.0 ± 2.7 |
| TM38-E | 92.7 ± 3.0 |
| TM66-E | 92.3 ± 1.9 |

The results show that VAP-1 was completely inhibited in vivo six hours after being orally administrated with the

compounds TM26, TM33, and TM42 of the invention at a dose of 2 mg/kg. The other compounds of the invention can totally or strong inhibit VAP-1 in vivo at the dose of 2 mg/kg. The compound of the invention exhibits strong inhibitory effect against VAP-1 in vivo even at a low administration dose of 0.5 mg/kg.

Thus, the compound of the invention exhibits strong inhibition against VAP-1 in vivo.

Experimental Example 3: Inhibition experiment against the enzymatic activity of MAO-A (Monoamine oxidase type A)

1. Test kit: Amplex® Red Monoamine Oxidase Assay, manufacturer: ThermoFisher

2. Reaction buffer solution: 50 mM PBS, pH 7.4
Protein: MAO-A, manufacturer: Sigma Aldrich

3. Test method of inhibition against the enzymatic activity of MAO-A

According to the method as described in the specification of the kit, MAO-A protein (15 μg/ml) and different concentrations of the test compound were pre-incubated at room temperature for 30 minutes, and then 200 μM p-tyramine hydrochloride, 1 U/mL HRP and 200 μM Amplex Red reagent were added to start the reaction. In kinetic mode, fluorescence signals were measured at Ex/Em=540/590 nm. The inhibition rate of each concentration group was calculated, wherein the ratio of fluorescence signal to reaction time ratio is the reaction rate, the vehicle group (DMSO) is the negative control, and the reaction buffer solution group (without enzymes and the compounds) is the blank control.

Percent inhibition rate (%) = (1-(reaction rate of each compound concentration group - reaction rate of the blank group)/(reaction rate of vehicle group – reaction rate of the blank group))*100.

3. Experimental results

According to the above method, the inhibition of the compound against the enzymatic activity of MAO-A was measured, and the range of $IC_{50}$ was obtained according to the inhibition rates at 100 μM and 10 μM. The results are shown in Table 4:

Table 4. Results of inhibition against the enzymatic activity of MAO-A

| Compound No. | $IC_{50}$ (μM) |
|---|---|
| TM26-Z | >100 |
| TM39-E | >10 |
| TM77-Z | >100 |
| TM7-E | >100 |
| TM38-E | >100 |
| TM66-E | >10 |
| TM75-Z | >100 |
| TM76-Z | >100 |
| TM76-E | >100 |
| TM112-Z | >100 |
| TM112-E | >100 |
| TM114-Z | >10 |
| TM114-E | >10 |

The results show that the inhibition of the compound of the invention against the activity of MAO-A is weaker than that of VAP-1.

Experimental Example 4: Inhibition experiment against the enzymatic activity of MAO-B (Monoamine oxidase type B)

1. Test kit: Amplex® Red Monoamine Oxidase Assay, manufacturer: ThermoFisher
Protein: MAO-B, manufacturer: Sigma Aldrich

2. Test method of the inhibition against the enzymatic activity of MAO-B

According to the method as described in the specification of the test kit, MAO-B protein (5 μg/ml) and different concentrations of the test compound were pre-incubated at room temperature for 10 minutes, and then 150 μM benzylamine hydrochloride, 1 U/mL HRP and 200 μM Amplex Red reagent were added to start the reaction. The relative inhibition rate of each concentration group were calculated according to the experimental method of MAO-A.

3. Experimental Results

According to the above method, the inhibition of the compound against the activity of the MAO-B was measured, and the range of $IC_{50}$ was obtained according to the inhibition rates at 10 μM, 1 μM and 0.1 μM. The results are shown in Table 5:

Table 5. Results of inhibition against the enzymatic activity of MAO-B

| Compound No. | $IC_{50}$ (μM) |
|---|---|
| TM26-Z | >1 |
| TM7-Z | >10 |
| TM7-E | >10 |
| TM38-Z | >10 |
| TM38-E | >10 |
| TM66-Z | >10 |
| TM66-E | >10 |
| TM75-Z | >10 |
| TM76-Z | >0.1 |
| TM76-E | >1 |
| TM112-Z | >0.1 |
| TM112-E | >0.1 |

The results show that the inhibition of the compound of the invention against the enzymatic activity of MAO-B is weaker than that of VAP-1.

Experimental Example 5: Inhibition experiment against the enzymatic activity of DAO (Diamine oxidase)

1. Test kit: Amplex® Red Monoamine Oxidase Assay, manufacturer: ThermoFisher
Protein: DAO, manufacturer: R&D Systems

2. Test method of inhibition against the enzymatic activity of DAO

DAO protein (15 μg/ml) and different concentrations of the test compound were preincubated at room temperature for 20 minutes, and then 100 μM Histamine-dihydrochloride, 1 U/mL HRP and 200 μM Amplex Red reagent were added to start the reaction. The inhibition rate of each concentration groups were calculated according to the experimental method of MAO-A.

3. Experimental results

According to the above method, the inhibition of the compound against the activity of DAO was measured, and the results are shown in Table 6:

Table 6. Results of inhibition against the enzymatic activity of DAO

| Compound No. | $IC_{50}$ (μM) |
|---|---|
| TM1-E | >0.1 |
| TM7-Z | >0.1 |
| TM7-E | >1 |
| TM38-Z | >0.1 |
| TM38-E | >1 |
| TM66-E | >0.1 |
| TM76-E | >0.1 |
| TM112-Z | >0.1 |

The results show that the inhibition of the compound of the invention against the activity of DAO is weaker than that of VAP-1.

Experimental Example 6. hERG (Ether-a-go-go-related gene potassium channel) inhibition experiment

Reagent: Predictor™ hERG Fluorescence Polarization Assay Kit, manufacturer: ThermoFisher

Experimental method

The above test kit was used to evaluate the potential of the compound for inducing heart QT prolongation. According to the method as indicated in the specification of the test kit, the test compound, the positive control (E4031) and negative control (Experimental buffer solution) in the kit were added to a microwell plate containing hERG cell membrane, and then a tracer having high affinity to hERG was added. The microwell plate was then incubated at 25 °C for 2 hours, a BMG PHAREStar polyfunctional enzyme marker was used to test the variations in the fluorescent polarization value. By calculating the percent inhibition rates (%) at different concentrations, the range of the half inhibition concentration ($IC_{50}$) was determined:

Percent inhibition rate (%) = (1-(mP of the test compound-mP of 30 μM E4031)/(mP of experimental buffer solution-mP of 30 μM E4031))*100.

Experimental results

According to the above method, the inhibition of the test compound against the activity of the hERG was measured, and the results are shown in Table 7:

Table 7. Experimental results of inhibition against hERG

| Compound No. | hERG (μM) |
|---|---|
| TM70 | >30 |
| TM1 | >10 |

(continued)

| Compound No. | hERG ($\mu$M) |
|---|---|
| TM67 | >30 |
| TM62 | >30 |
| TM26 | >30 |
| TM42 | >10 |
| TM39 | >30 |
| TM61 | >10 |
| TM44 | >10 |
| TM45 | >10 |
| TM45-Z | >10 |
| TM45-E | >10 |
| TM33-E | >10 |
| TM63-Z | >10 |
| TM63-E | >10 |
| TM1-Z | >10 |
| TM1-E | >10 |
| TM42-Z | >10 |
| TM42-E | >10 |
| TM26-Z | >10 |
| TM26-E | >10 |
| TM39-Z | >10 |
| TM39-E | >10 |
| TM77-Z | >10 |
| TM7-E | >10 |
| TM38-E | >10 |
| TM66-E | >10 |
| TM76-E | >10 |
| TM112-Z | >10 |

The results show that the compound of the invention has a low affinity to hERG. $IC_{50}$ of the test compounds competitively binding to hERG, in relative to the affinitive tracer, are all greater than 10 $\mu$M.

Experimental Example 7: Inhibition Experiment against the enzymatic activity at different does and time conditions (ex-vivo)

Reagent:

Tissue homogenate buffer solution (20 mM HEPES, pH 7.2, 1 mM EDTA, 250 mM sucrose, PMSF 0.2 mM)
Reaction buffer solution: 50 mM PBS, pH 7.4
Test kit: Amplex® Red Monoamine Oxidase Assay, manufacturer: ThermoFisher

Experimental method

1. Sample collections and treatments

BALBc mice were orally administrated with different doses (0.5 mg/kg, 3 mg/kg) of the test compound, and the vehicle group was used as the control group. Abdominal fat was collected at 6 hrs, 10 hrs, 24 hrs respectively, and homogenized with a pre-cooled homogenate buffer solution, and thereafter, the supernatant was collected and stored for use.

2. Enzymatic activity measurement

A test sample was diluted with the reaction buffer solution, and respectively added with the inhibition solution A: 1 μM Clorgyline+1 μM pargylin, to inhibit the activities of MAO-A and MAO-B, and the inhibition solution B: 1 μM Clorgyline+1 μM Pargylin+1 μM Mofegiline as the blank control, and after being incubated at room temperature, 500 μM benzylamine hydrochloride, 1 U/mL HRP and 200 μM Amplex Red reagent were added to start the reaction. By referring to the method in "Experiment of inhibiting the enzymatic activity of VAP-1", the relative activities of each dose groups were calculated, wherein ratio of the fluorescent signal to reaction time in the linear range is the reaction rate, and the vehicle group is the positive control, thereby to study the inhibition of the compound against the enzymatic activity of VAP-1 in abdominal fat of mice.

Experimental results

According to the above method, the inhibition of the compound on the enzymatic activity of VAP-1 in abdominal fat of mice was measured, as shown in Table 8:

Table 8. Inhibition rate of the compound HC38-E against the enzymatic activity of VAP-1 in abdominal fat of mice at different time and dose conditions

| dose/time | 6 hours | 10 hours | 24 hours |
|---|---|---|---|
| 0.5 mg/kg | 95.8±3.6 % | 90.3± 3.2 % | 68.1± 7.4 % |
| 3.0 mg/kg | 96.4±5.6 % | 95.3± 1.9 % | 78.0± 9.3 % |

The results show that the inhibition rate of the compound HC38-E against the activity of VAP-1 in abdominal fat is up to 95% 6 hours after administration at the dose of 0.5 mg/kg, the inhibition is still significant 24 hours after administration. The inhibition rate of the compound HC38-E against the activity of VAP-1 in abdominal fat is still up to 95% 10 hours after administrate at the dose of 3.0 mg/kg, and the inhibition is still significant 24 hours after administration.

Experimental Example 8: Experiment of carbon tetrachloride-induced liver injury of mice

Regent

Olive oil: Sinapharm Chemical Reagent Co., Ltd
Carbon tetrachloride: Sinapharm Chemical Reagent Co., Ltd

Experimental method

1. Model establishment method

Female BALB/c mice received an intraperitoneal injection of a 25% carbon tetrachloride solution (carbon tetrachloride: olive oil=1: 4, volume rate) in an injection volume of 4 ml/kg twice a week for six weeks.

2. Grouping and administration

Mice, according to body weights, were randomly divided into a model group (0.5% methylcellulose), a group administrated with 3 mg/kg of the compound HC38-E, and a group administrated with 10 mg/kg of the compound HC38-E, each group including ten animals.
Whilst establishing the models, the administration started with the administration volume of 10 mL/kg, and the

models were intragastrically administrated once a day for six weeks.

3. Fibrosis quantitative analysis

After the administration, liver tissue was taken from euthanized animals and fixed with 10% formalin. Thereafter, liver tissue slices were prepared and stained with Sirius red. The slices were subjected to calculate the fibrosis areas by using semi-automatic digital image analysis system, measuring software (Osteo Metrics, Inc., Atlanta, GA) and Olympus optical microscope.

Experimental results

According to the above method, the impacts of the compound on the liver fibrosis of the mice are measured, as shown in the following Table 9:

Table 9. Impacts of the compound HC38-E on the fibrosis areas of mice induced by carbon tetrachloride

| Dose | Fibrosis area |
|---|---|
| Solvent group | 1.05 ± 0.27 % |
| 10 mg/kg | 0.56 ± 0.23 % |
| 3 mg/kg | 0.73 ± 0.21 % |

The results show that after a six-week administration, the compound HC38-E can reduce the degree of the liver fibrosis at the doses of 10mg/kg and 3mg/kg, especially, the compound have apparent antifibrosis at the dose of 10 mg/kg.

Experimental Example 9: Inhibition experiment against cytochrome P450 isoenzyme (CYP)

Reagent:

P450-Glo™ CYP1A2 Screening System, manufacturer: Promega
P450-Glo™ CYP2D6 Screening System, manufacturer: Promega
P450-Glo™ CYP3A4 Screening System, manufacturer: Promega

Experimental method:

According to the specification of the test kit, 10 nM CYP1A2, 5 nMCYP2D6, and 1 nM CYP3A4 proteins and corresponding substrates (100 μM, 30 μM, 3 μM) as well as the compound were pre-incubating respectively for 10 min, G6PDH-G6P-NADP regeneration system was added to start the reaction, a test reagent was added later, after 20 min, the chemical luminescent signals were detectted. The inhibition rate was calculated with vehicle group (DMSO) as the negative control and Membrance (inactive enzymes) as the blank control:

Percent inhibition rate (%)=(1-(signal of each compound concentration group-signal of the blank group)/(signal of the negative control-signal of the blank group))*100.

According to the inhibition rates of the compound at different concentrations against P450 enzyme, the half inhibition concentration ($IC_{50}$) or range was estimated.

$$IC_{50} = X*(1\text{-percent inhibition rate }(\%))/\text{percent inhibition rate }(\%),$$

wherein, X is the test concentration of the compound.

Experimental results

According to the above method, the inhibition of the compound at the concentrations 10 μM, and 1 μM, against three CYPs were measured, and the results were shown in the following Tables 10-12:

Table 10. Results of inhibition against CYP1A2

| Compound No. | $IC_{50}$ (μM) |
|---|---|
| TM38-E | 6.73 ± 0.39 |

Table 11. Results of inhibition against CYP2D6

| Compound No. | $IC_{50}$ (μM) |
|---|---|
| TM38-E | >10 |
| TM66-E | >10 |

Table 12. Results of inhibition against CYP3A4

| Compound No. | $IC_{50}$ (μM) |
|---|---|
| TM38-E | >10 |
| TM66-E | >10 |

The above results show that the compound of the invention (for example, the compounds TM38-E, TM66-E) do not have significant inhibitions against three primary CYP-subtypes, which shows that the compound has low possibility of potential drug interactions, and thus has good drug-forming property.

Experimental Example 10: Stability of liver microsomes

Reagents:

Formulation of a compound reservation solution: the test compound, diclofenac acid, testosterone and propafenone were weighted in appropriate amounts, and calibrated by adding about 1 mL of DMSO to provide a 10 mM reservation solution, then stored in a 4 °C refrigerator;
Formulation of a compound working solution: 10 μL of the reservation solution were added to 990 μL acetonitrile-water (1:1) to provide a middle solution having a concentration of 100 μM, and 120 μL of the middle solution is further diluted with 2880 μL PBS to provide a 4 μM working solution;
Formulation of a NADPH working solution: the NADPH was weighted in an appreciate amount and calibrated with 33 mM $MgCl_2$ to provide a 4 mM NADPH working solution;
Formulation of a liver microsome working solution: 100 μL of liver microsomes of human, monkey, dog, rat and mouse were taken respectively and diluted with 1900 μL PBS to prepare a 1 mg/mL liver microsome working solution.

Experimental method:

According to the experimental conditions as shown in the following Table 13, the reaction solution of the test compound was formulated:

Table 13. Experimental conditions

| Incubation conditions | Incubation time (min) |
|---|---|
| 50 μL the test sample + 50 μL NADPH + 100 μL microsome | 0, 30, 60, 120 |

After being incubated for a certain period of time, an equivalent volume of cooled acetonitrile (4 °C) was added to terminate the reaction, and LC-MS/MS was used to determine the concentrations of the compound after being incubated in various liver microsomes for different time periods. With the incubation time as the horizontal ordinate and the natural logarithm of the percentages of the remained compound at each time point as the longitudinal coordinate, a diagram was plotted, wherein the slope was the velocity constant (Ke), and the half-life ($T_{1/2}$) and clearance rate ($CL_{liver}$) of the compound were calculated according to the following equation to evaluate the staiblity of the compound in the liver microsomes.

$$T_{1/2}=\ln(2)/Ke$$

Liver clearance rate ($CL_{liver}$)=Ke/protein quantity of microsomes contained in per milter of reaction solution (mg)xprotein quantity of microsomes contained in per gram of liver (mg)×liver weight correspondent to body weight per kilogram (g).

Experimental results

The stability test results of the compound in liver microsomes of different animals are shown in the following Table 14.

Table 14. Results of the stability in liver microsomes

| Compound No. | Parameters | Unit | human | Monkey | Dog | Rat | Mouse |
|---|---|---|---|---|---|---|---|
| HC38-E | $T_{1/2}$ | min | 2677 | 1249 | 7081 | 855 | 829 |
| | $CL_{liver}$ | μL/min/mg | 0.48 | 1.11 | 0.29 | 1.08 | 2.78 |

The above results show that the compound of the invention (for example, the compound HC38-E) has long half-life ($T_{1/2}$) and low clearance rate in vivo in liver microsomes of human, monkey, dog, rate, and mouse, indicating its good staiblity.

Experimental Example 11: Caco-2 permeation experiment

Reagents:

Culture medium: 10%FBS+1% unnecessary amino acid+90%DMEM (high glucose)
Permeation solution (pH 7.4): HBSS ($Ca^+$, $Mg^+$)+10 mM Hepes
Compound reservation solution: an amount of the test compound was weighted and added with a certain amount of DMSO to formulate a 10 mM reservation solution.

Experimental method:

Cells were inoculated to Transwell cells in a quantity of $6*10^4/cm^2$, and at the A side (top side), were added with 200 μL of the culture medium, and at the B side (base side), were added with 1000 μL of the culture medium. After the inoculation, the solution was replaced once every 2 days, and the culture was conducted for 21 to 28 day.
After 21 to 28 days since the inoculation of Caco-2 cells, the transmembrane resistance was measured.
Permeation experiment: after the transepithelial electrical resistance was measured, the buffer solution at the two sides was removed by suction, then added thereto the solution according to the following system.
According to the experimental conditions as shown in the following Table 15, the reaction solution in each experimental group was formulated:

Table 15. Experimental conditions and grouping

| System | Concentration (μM) | Top side A (200 μL permeation solution) | Base side B (1000 μL permeation solution) |
|---|---|---|---|
| Experimental group (A→B) | 2 | 2 μM test compound, 10 μg/ml Fluorescein | - |

(continued)

| System | Concentration (μM) | Top side A (200 μL permeation solution) | Base side B (1000 μL permeation solution) |
|---|---|---|---|
| Experimental group (B→A) | 2 | - | 2 μM test compound, 10 μg/ml Fluorescein |
| Incubation time/min | 120 min | | |
| wherein, the symbol "-" represents "none". | | | |

Fluorescein: after 2-hours incubation, 100 μL of the permeation solution were taken from each side of side A and side B, and the fluorescent value at Ex=485nm, Em=530nm were measured by an enzyme marker, and the permeation quantity was calculated (less than 1%).

Sample treatment: after 2-hours incubation, 50 μL of the permeation solution were sucked up from each side of side A and side B, and equivalent volume of cold acetonitrile was added to terminate the reaction, and the concentrations of the compound at the A and B sides were measured by LC-MS/MS. The apparent permeation (Papp) and the Efflux Ratio were calculated according to the following equations:

$$Papp=(dCr/dt)\times Vr/(A \times C_0)$$

$$Efflux\ Ratio=Papp(BA)/Papp\ (AB)$$

Wherein, dCr/dt is the integration of the concentration of the compound at the base side realted to time, Vr is the reaction volume at the base side, A is the area of a single layer of cells, being 0.33 $cm^2$, and $C_0$ is the concentration of the compound at the top side.

Experimental results

The experimental results of the permeability of the compound to Caco-2 cells are shown in the following Table 16:

Table 16. Results of Caco-2 permeability

| Compound No. | Concentration | Average apparent permeability ($10^{-6}$ cm/s) | | Efflux ratio |
|---|---|---|---|---|
| | | A to B | B to A | |
| HC38-E | 2 μM | 2.60 | 3.26 | 1.26 |

The above results show that the compounds of the invention (for example HC38-E) have a high permeability, and a low possibility of active efflux.

Experimental Example 12: Rat PK experiment

Formulation of the test samples:

The test compounds were formulated according to the method as shown in Table 17:

Table 17. Formulating table of the tested compounds

| Subject | Group No. | Concentration (mg/mL) | Administration volume (mL/kg) | Solvent (v/v) |
|---|---|---|---|---|
| HC38-E | *iv* | 0.6 | 5 | 5%DMSO+5%solutol+90%saline 0.5%MC |
| | po | 0.6 | 5 | |

Method:

Male rats were intravenously (iv) and intragastrically (po) administered the test compounds respectively, and LC-MS/MS was used to measure the blood plasma concentration of the compound in the bodies of the rat. The main pharmacokinetic parameters were calculated by utilizing WinNonlin 6.3 software. The grouping of the experimental designs was shown in the following table 18:

Table 18. Grouping Table of experimental design

| subject | Administration route | Quantity | Animal species | Time point of collecting blood or tissue |
|---|---|---|---|---|
| HC38-E | i.v | *n*=3 | Rat | i.v: 0, 0.083, 0.25, 0.5, 1, 2, 4, 6, 8 and 24 h |
| | p.o | *n*=3 | Rat | p.o: 0, 0.25, 0.5, 1, 2, 4, 6, 8 and 24 h |

Results

The PK test results of the test compounds in rat blood plasma were shown in the following Table 19:

Table 19 PK data of the test compounds in rats blood plasma

| Compound No. | Administration route | iv | po |
|---|---|---|---|
| HC38-E | Dose (mg/kg) | 3 | 3 |
| | AUC(0-∞) hr*ng/mL | 1161±120 | 1042±129 |
| | $C_{max}$ ng/mL | 1041±181 | 343±24 |
| | $t_{1/2}$ hr | 1.43±0.15 | 1.37±0.01 |
| | F% | / | 89.9±11.1 |
| Symbol "/" represents "none". | | | |

The results show that the compound of the invention have good pharmacokinetic parameters both administered intravenously (iv) and intragastrically (po), and the compound of the invention (for example, the average bioavailability (F%) of the compound HC38-E can reach 89.9%) have good oral bioavailability.

The above examples will not restrict the invention in any way. Although the specific embodiments in the invention have been illustrated in detail, according to all the disclosed teachings, a person skilled in the art can make various amendments and replacements to the details of the technical solution of the invention, and these changes all fall within the protection scope of the invention. All the scope of the invention will be provided by the appended claims and any equivalent thereof.

**Claims**

1. A compound of Formula I, or a pharmaceutically acceptable salt, an ester, a solvate, a hydrate, an isomer, or an isotope-labeled compound thereof; any crystal form or racemate thereof; a metabolite thereof; or a mixture thereof:

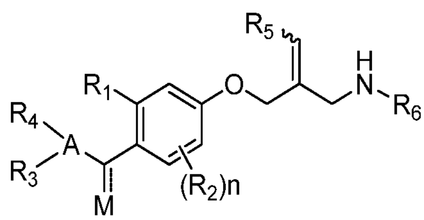

Formula I

Wherein:

$R_1$ is selected from the group consisting of hydrogen, halogen, $C_{1-6}$ alkyl, 3-10-membered cycloalkyl-$CH_2NHC(O)$-, 3-8-membred aliphatic heterocyclyl-$CH_2NHC(O)$-, 6-20-membered aryl-$CH_2NHC(O)$-, 5-20-membered heteroaryl-$CH_2NHC(O)$-, 5-20-membered fused heteroaryl-$CH_2NHC(O)$-, benzo-fused 3-10-membered cycloalkyl-NHC(O)-, 3-8-membered aliphatic heterocyclyl-NHC(O)-, 6-20-membered aryl-NHC(O)-, 5-20-membered heteroaryl-NHC(O)-, and 5-20-membered fused heteroaryl-NHC(O)-; and wherein the $C_{1-6}$ alkyl, 3-10-membered cycloalkyl-$CH_2NHC(O)$-, 3-8-membered aliphatic heterocyclyl-$CH_2NHC(O)$-, 6-20-membered aryl-$CH_2NHC(O)$-, 5-20-membered heteroaryl-$CH_2NHC(O)$-, 5-20-membered fused heteroaryl-$CH_2NHC(O)$-, benzo-fused 3-10-membered cycloalkyl-NHC(O)-, 3-8-membered aliphatic heterocyclyl-NHC(O)-, 6-20-membered aryl-NHC(O)-, 5-20-membered heteroaryl-NHC(O)-, and 5-20-membered fused heteroaryl-NHC(O)- are unsubstituted or substituted independently by one or more (for example 1, 2, 3 or 4) substituents selected from the group consisting of halogen, hydroxyl, -NRR', $C_{1-3}$ alkyl, $C_{1-3}$ alkoxyl, and cyano;
each of $R_2$ is independently selected from the group consisting of hydrogen, cyano, nitro, hydroxyl, halogen, $C_{1-6}$ alkyl, 3-10-membered cycloalkyl, 3-8-membered aliphatic heterocyclyl, halogenated $C_{1-6}$ alkyl, $C_{1-6}$ alkoxyl, and halogenated $C_{1-6}$ alkoxyl;
n=1, 2 or 3;
$R_3$ and $R_4$ are such that

(a) $R_3$ is selected from the group consisting of 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl phenyl, 3-8-membered aliphatic heterocyclyl, 6-20-membered aryl, 5-20-membered heteroaryl, and 5-20-membered fused heteroaryl; and wherein the 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl phenyl, 3-8-membered aliphatic heterocyclyl, 6-20-membered aryl, 5-20-membered heteroaryl, and 5-20-membered fused heteroaryl are unsubstituted or substituted independently by one or more (for example 1, 2, 3 or 4) substituents selected from the group consisting of halogen, hydroxyl, -NRR', $C_{1-3}$ alkyl, $C_{1-3}$ alkoxyl, cyano, 3-10-membered cycloalkyl, 3-8-membered aliphatic heterocyclyl, 6-20-membered aryl, 5-20-membered heteroaryl, 5-20-membered fused heteroaryl, halogenated $C_{1-3}$ alkyl, halogenated $C_{1-3}$ alkoxyl, $C_{1-3}$ alkoxy-$C_{1-3}$ alkyl, $C_{1-3}$ alkoxy-$C_{1-3}$ alkoxyl, and C1-3 alkoxy-$C_{1-3}$ alkoxy-$C_{1-3}$ alkyl; $R_4$ is selected from hydrogen, $C_{1-6}$ alkyl, and halogenated $C_{1-6}$ alkyl;
(b) $R_3$ and $R_1$ are linked together to form a ring X, wherein the ring X is a 5-8-membered aliphatic ring or a 5-8-membered aliphatic heterocycle; and wherein the 5-8-membered aliphatic ring or 5-8-membered aliphatic heterocycle is unsubstituted or each substituted independently by one or more (for example 1, 2, 3 or 4) substituents selected from the group consisting of O=, halogen, cyano, -NRR', nitro, hydroxyl, $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl; or the aforementioned 5-8-membered aliphatic ring or 5-8-membered aliphatic heterocycle is fused with a 3-8-membred aliphatic ring to form a spiro structure; $R_4$ is selected from the group consisting of hydrogen, $C_{1-6}$ alkyl, 3-8-membered cycloalkyl, 3-8-membered aliphatic heterocyclyl, 6-10-membered aryl, 5-10-membered heteroaryl, and 5-10-membered fused heteroaryl; and wherein the $C_{1-6}$ alkyl, 3-8-membered cycloalkyl, 3-8-membered aliphatic heterocyclyl, 6-10-membered aryl, 5-10-membered heteroaryl or 5-10-membered fused heteroaryl is optionally substituted by one or more (for example 1, 2, 3 or 4) substituents selected from the group consisting of halogen, -NRR', cyano, hydroxyl, $C_{1-4}$ alkyl, $C_{1-4}$ alkoxyl, 3-8-membered cycloalkyl, 3-8-membered aliphatic heterocyclyl, 6-10-membered aryl, 5-10-membered heteroaryl, and 5-10-membered fused heteroaryl; wherein the $C_{1-4}$ alkyl, 3-8-membered cycloalkyl, 3-8-membered aliphatic heterocyclyl, 6-10-membered aryl, 5-10-membered heteroaryl or 5-10-membered fused heteroaryl is optionally substituted by one or more (for example, 1, 2, 3 or 4) substituents selected from halogen, -NRR', $C_{1-3}$ alkyl, and $C_{1-3}$ alkoxyl;
(c) $R_3$ and $R_4$ are linked together to form a ring Y, wherein the ring Y is a 5-8-membered aliphatic ring or a 5-8-membered aliphatic heterocycle; and wherein the 5-8-membered aliphatic ring or 5-8-membered aliphatic heterocycle is unsubstituted or each substituted independently by one or more (for example 1, 2, 3 or 4) substituents selected from the group consisting of halogen, cyano, -NRR', nitro, hydroxyl, $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl; wherein each of $C_{1-6}$ alkyl, $C_{1-6}$ alkoxyl, halogenated $C_{1-6}$

alkyl, halogenated $C_{1-6}$ alkoxyl, 6-20-membered aryl, 5-20-membered heteroaryl, and 5-20-membered fused heteroaryl is unsubstituted or substituted independently by one or more (for example, 1, 2, 3 or 4) substituents selected from the group consisting of halogen, cyano, -NRR', $C_{1-6}$ alkyl, $C_{1-6}$ alkoxyl, 6-20-membered aryl, 5-20-membered heteroaryl, and 5-20-membered fused heteroaryl; or the aforementioned 5-8-membered aliphatic ring or 5-8-membered aliphatic heterocycle is fused with a 6-20-membered aromatic ring or a 5-20-membered aromatic heterocycle to form a fused ring system, wherein the fused ring system is unsubstituted or substituted independently by one or more (for example 1, 2, 3 or 4) substituents selected from the group consisting of halogen, cyano, -NRR', $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl; or

(d) $R_3$ is linked together with M to form a ring Z, wherein the ring Z is a 3-10-membered aliphatic heterocycle, a 6-20-membered aromatic ring or a 5-20-membered aromatic heterocycle; and wherein the 3-10-membered aliphatic heterocycle, 6-20-membered aromatic ring or 5-20-membered aromatic heterocycle is unsubstituted or each substituted independently by one or more (for example 1, 2, 3 or 4) substituents selected from the group consisting of halogen, cyano, -NRR', nitro, hydroxyl, =O, $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroary; or the aforementioned 3-10-membered aliphatic heterocycle, 6-20-membered aromatic ring or 5-20-membered aromatic heterocycle is fused with a 6-20-membered aromatic ring or a 5-20-membered aromatic heterocycle to form a fused ring system, wherein the fused ring system is unsubstituted or each substituted independently by one or more (for example 1, 2, 3 or 4) substituents selected from the group consisting of halogen, cyano, -NRR', $C_{1-6}$ alkyl, halogenated $C_{1-6}$ alkyl, halogenated $C_{1-6}$ alkoxyl, 6-20-membered aryl, 5-20-membered heteroaryl, and 5-20-membered fused heteroaryl; $R_4$ is selected from hydrogen, $C_{1-6}$ alkyl, and $C_{1-6}$ alkoxyl, or $R_4$ is absent or forms a covalent bond;

Rs is halogen;
$R_6$ is selected from hydrogen, $C_{1-6}$ alkyl, and -COOR; wherein $C_{1-6}$ alkyl is unsubstituted or substituted by one or more (for example 1, 2, 3 or 4) substituents selected from halogen, hydroxyl, amino, and cyano;
atom A is selected from C, N, O and S;
M is selected from C, N, O, $H_2$ and =NR;
R and R' are independently selected from the group consisting of hydrogen, halogen, cyano, hydroxyl, $C_{1-3}$ alkyl, $C_{1-3}$ alkoxyl, 3-8-membered cycloalkyl, 3-8-membered aliphatic heterocyclyl, 6-10-membered aryl, 5-10-membered heteroaryl, and 5-10-membered fused heteroaryl;
preferably, $R_1$ is not a hydrogen, and

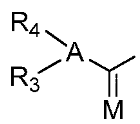

as a whole is independently a hydrogen;
preferably, the compound is a mixture of the cis-configuration and the trans-configuration in any ratio;
preferably, the compound is in cis (Z)-configuration;
preferably, the compound is in trans (E)-configuration.

**2.** The compound of claim 1, or a pharmaceutically acceptable salt, an ester, a solvate, a hydrate, an isomer, or an isotope-labeled compound thereof; any crystal form or racemate thereof; a metabolite thereof; or a mixture thereof, wherein the compound has a structure as represented by Formula II:

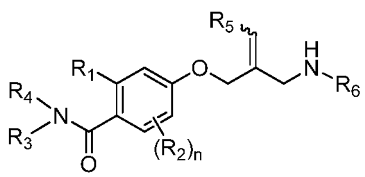

Formula II

wherein, in the compound of Formula II, $R_3$ is selected from the group consisting of 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl-phenyl, 3-8-membered aliphatic heterocyclyl, 6-20-membered aryl, 5-20-membered heteroaryl, and 5-20-membered fused heteroaryl; and wherein the 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl-phenyl, 3-8-membered aliphatic heterocyclyl, 6-20-membered aryl, 5-20-membered hetebroaryl, and 5-20-membered fused heteroaryl are unsubstituted or substituted independently by one or more (for example 1, 2, 3 or 4) substituents selected from the group consisting of halogen, hydroxyl, -NRR', $C_{1-3}$ alkyl, $C_{1-3}$ alkoxyl, cyano, 3-10-membered cycloalkyl, 3-8-membered aliphatic heterocyclyl, 6-20-membered aryl, 5-20-membered heteroaryl, 5-20-membered fused heteroaryl, halogenated $C_{1-3}$ alkyl, halogenated $C_{1-3}$ alkoxyl, $C_{1-3}$ alkoxyl-$C_{1-3}$ alkyl, $C_{1-3}$ alkoxyl-$C_{1-3}$ alkoxyl, and $C_{1-3}$ alkoxyl-$C_{1-3}$ alkoxyl-$C_{1-3}$ alkyl; $R_4$ is selected from hydrogen, $C_{1-6}$ alkyl, and halogenated $C_{1-6}$ alkyl;

$R_1$, $R_2$, $R_5$, $R_6$, R, R' and n are as defined in Formula I;

preferably, in the compound of Formula II, $R_3$ is selected from the group consisting of 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl-phenyl, 3-8-membered aliphatic heterocyclyl, 6-20-membered aryl, 5-20-membered heteroaryl, and 5-20-membered fused heteroaryl; and wherein the 3-10-membered cycloalkyl-$CH_2$, 3-8-membered aliphatic heterocyclyl-$CH_2$, 6-20-membered aryl-$CH_2$, 5-20-membered heteroaryl-$CH_2$, 5-20-membered fused heteroaryl-$CH_2$, benzo-fused 3-10-membered cycloalkyl, benzo-fused 3-8-membered aliphatic heterocyclyl, phenyl-3-10-membered cycloalkyl, 5-20-membered heteroaryl-phenyl, 5-20-membered fused heteroaryl-phenyl, 3-8-membered aliphatic heterocyclyl, 6-20-membered aryl, 5-20-membered heteroaryl, and 5-20-membered fused heteroaryl are unsubstituted or substituted independently by one or more (for example 1 or 2) substituents selected from the group consisting of halogen, hydroxyl, -NRR', $C_{1-3}$ alkyl, $C_{1-3}$ alkoxyl, cyano, 3-10-membered cycloalkyl, 3-8-membered aliphatic heterocyclyl, 6-20-membered aryl, 5-20-membered heteroaryl, 5-20-membered fused heteroaryl, halogenated $C_{1-3}$ alkyl, halogenated $C_{1-3}$ alkoxyl, $C_{1-3}$ alkoxy-$C_{1-3}$ alkyl, $C_{1-3}$ alkoxy-$C_{1-3}$ alkoxyl, and $C_{1-3}$ alkoxy-$C_{1-3}$ alkoxy-$C_{1-3}$ alkyl; $R_4$ is selected from H and methyl;

$R_1$ is selected from H, halogen, and $C_{1-3}$ alkyl; $R_2$ is selected from the group consisting of hydrogen, cyano, nitro, halogen, $C_{1-3}$ alkyl, and $C_{1-3}$ alkoxyl; and n=1;

$R_5$, $R_6$, R and R' are as defined in Formula I;

preferably, in the compound of Formula II, $R_3$ is selected from the group consisting of 6-15-membered aryl, benzo-fused 3-8-membered cycloalkyl, benzo-fused 3-8-membered aliphatic heterocyclyl, 3-8-membered aliphatic heterocyclyl, 5-10-membered heteroaryl-$CH_2$, 5-10-membered fused heteroaryl-$CH_2$, 3-8-membered cycloalkyl-$CH_2$, 3-8-membered aliphatic heterocyclyl-$CH_2$, 6-10-membered aryl-$CH_2$, 5-10-membered heteroaryl, and 5-10-membered fused heteroaryl; and wherein the 6-15-membered aryl, benzo-fused 3-8-membered cycloalkyl, benzo-fused 3-8-membered aliphatic heterocyclyl, 3-8-membered aliphatic heterocyclyl, 5-10-membered heteroaryl-$CH_2$, 5-10-membered fused heteroaryl-$CH_2$, 3-8-membered cycloalkyl-$CH_2$, and 3-8-membered aliphatic heterocyclyl-$CH_2$ are unsubstituted or substituted independently by one or more (for example 1 or 2) substituents selected from the group consisting of halogen, $C_{1-3}$ alkyl, $C_{1-3}$ alkoxyl-$C_{1-3}$ alkoxyl, and NRR', wherein R and R' are independently selected from H and $C_{1-3}$ alkyl; $R_4$ is H or methyl;

$R_1$ is selected from H and halogen; $R_2$ is selected from the group consisting of H, halogen, $C_{1-3}$ alkyl, and $C_{1-3}$ alkoxyl, and n=1;

$R_5$ and $R_6$ are as defined in Formula I;

preferably, $R_3$ is selected from the group consisting of 3-7-membered cycloalkyl-$CH_2$, 3-7-membered aliphatic heterocyclyl-$CH_2$, 6-10-membered aryl-$CH_2$, 5-6-membered heteroaryl-$CH_2$, benzo-fused 3-7-membered cycloalkyl, 3-7-membered aliphatic heterocyclyl, 6-10-membered aryl, 5-10-membered heteroaryl, and 5-10-membered fused heteroaryl-$CH_2$; $R_4$ is H;

$R_1$ is H; $R_2$ is H or halogen, and n=1; $R_5$ is F; $R_6$ is H.

**3.** The compound of claim 1, or a pharmaceutically acceptable salt, an ester, a solvate, a hydrate, an isomer, or an isotope-labeled compound thereof; any crystal form or racemate thereof; a metabolite thereof; or a mixture thereof, wherein the compound has a structure as represented by Formula III:

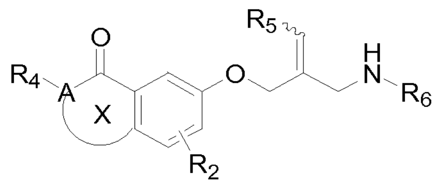

Formula III

wherein, the ring X is a 5-8-membered aliphatic ring or a 5-8-membered aliphatic heterocyclic ring; and wherein each of 5-8-membered aliphatic ring or the 5-8-membered aliphatic heterocyclic ring is unsubstituted or substituted independently by one or more (for example 1, 2, 3 or 4) substituents selected from the group consisting of =O, halogen, cyano, -NRR', $C_{1-4}$ alkyl, $C_{1-4}$ alkoxyl, halogenated $C_{1-4}$ alkyl, halogenated $C_{1-4}$ alkoxyl, 6-10-membered aryl, 5-10-membered heteroaryl, and 5-10-membered fused heteroaryl; or the aforementioned 5-8-membered aliphatic ring or the 5-8-membered aliphatic heterocyclic ring is fused with a 3-8-membered aliphatic ring to form a spiro structure;

$R_4$ is selected from the group consisting of hydrogen, $C_{1-6}$ alkyl, 3-8-membered cycloalkyl, 3-8-membered aliphatic heterocyclyl, 6-10-membered aryl, 5-10-membered heteroaryl and 5-10-membered fused heteroaryl; and wherein the $C_{1-6}$ alkyl, 3-8-membered cycloalkyl, 3-8-membered aliphatic heterocyclyl, 6-10-membered aryl, 5-10-membered heteroaryl or 5-10-membered fused heteroaryl is optionally substituted by one or more (for example 1, 2, 3 or 4) substituents selected from the group consisting of halogen, cyano, -NRR', hydroxyl, $C_{1-4}$ alkyl, $C_{1-4}$ alkoxyl, 3-8-membered cycloalkyl, 3-8-membered aliphatic heterocyclyl, 6-10-membered aryl, 5-10-membered heteroaryl, and 5-10-membered fused heteroaryl;

$R_2$, $R_5$, $R_6$, R, R' and A are as defined in Formula I;

preferably, in the compound of Formula III, the ring X is a 5-7-membered aliphatic ring or a 5-7-membered aliphatic heterocyclic ring; and wherein the 5-7-membered aliphatic ring or the 5-7-membered aliphatic heterocyclic ring is unsubstituted or substituted by one or more (for example 1, 2, 3 or 4) substituents selected from the group consisting of =O, halogen, $C_{1-4}$ alkyl, 6-10-membered aryl, 5-10-membered heteroaryl, and 5-10-membered fused heteroaryl; or the aforementioned 5-7-membered aliphatic ring or the 5-7-membered aliphatic heterocyclic ring is fused with a 3-6-membered aliphatic ring to form a spiro structure; A is selected from N and C; $R_2$ is selected from F, Cl, and H; $R_4$ is selected from H, $CH_3$, and $CH_2CH_3$; $R_5$ is F; $R_6$ is H;

preferably, in the compound of Formula III, the ring X is a 5-6-membered aliphatic ring or a 5-6-membered aliphatic heterocyclic ring; and wherein the 5-6-membered aliphatic ring or the 5-6-membered aliphatic heterocyclic ring is unsubstituted or substituted by one or more (for example 1, 2 or 3) substituents selected from the group consisting of O=, halogen, and $C_{1-3}$ alkyl; A is selected from N and C; $R_2$ is selected from F and H; $R_4$ is selected from H, $CH_3$, and $CH_2CH_3$; $R_5$ is F; $R_6$ is H.

**4.** The compound of claim 1, or a pharmaceutically acceptable salt, an ester, a solvate, a hydrate, an isomer, or an isotope-labeled compound thereof; any crystal form or racemate thereof; a metabolite thereof; or a mixture thereof, wherein the compound has a structure as represented by the Formula IV:

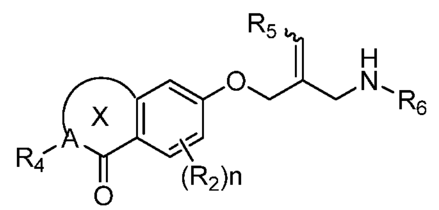

Formula IV

wherein, the ring X is a 5-8-membered aliphatic ring or a 5-8-membered aliphatic heterocyclic ring; and wherein the 5-8-membered aliphatic ring or the 5-8-membered aliphatic heterocyclic ring is unsubstituted or each substituted independently by one or more (for example 1, 2, 3 or 4) substituents selected from the group consisting of =O, halogen, cyano, -NRR', $C_{1-4}$ alkyl, $C_{1-4}$ alkoxyl, halogenated $C_{1-4}$ alkyl, halogenated $C_{1-4}$ alkoxyl, 6-10-

membered aryl, 5-10-membered heteroaryl, and 5-10-membered fused heteroaryl; or the aforementioned 5-8-membered aliphatic ring or the 5-8-membered aliphatic heterocyclic ring is fused with a 3-8-membered aliphatic ring to form a spiro structure;

$R_4$ is selected from the group consisting of hydrogen, $C_{1-6}$ alkyl, 3-8-membered cycloalkyl, 3-8-membered aliphatic heterocyclyl, 6-10-membered aryl, 5-10-membered heteroaryl, and 5-10-membered fused heteroaryl; and wherein the $C_{1-6}$ alkyl, 3-8-membered cycloalkyl, 3-8-membered aliphatic heterocyclyl, 6-10-membered aryl, 5-10-membered heteroaryl, and 5-10-membered fused heteroaryl are optionally substituted by one or more (for example 1, 2, 3 or 4) substituents selected from the group consisting of halogen, cyano, hydroxyl, $C_{1-4}$ alkyl, $C_{1-4}$ alkoxyl, 3-8-membered cycloalkyl, 3-8-membered aliphatic heterocyclyl, 6-10-membered aryl, 5-10-membered heteroaryl, and 5-10-membered fused heteroaryl; wherein the $C_{1-4}$ alkyl, 3-8-membered cycloalkyl, 3-8-membered aliphatic heterocyclyl, 6-10-membered aryl, 5-10-membered heteroaryl or 5-10-membered fused heteroaryl is optionally substituted by one or more (for example 1, 2 or 3) substituents selected from halogen, -NRR', and $C_{1-3}$ alkyl;

$R_2$, $R_5$, $R_6$, R, R', A and n are as defined in Formula I;

preferably, in the compound of Formula IV, the ring X is a 5-7-membered aliphatic ring or a 5-7-membered aliphatic heterocyclic ring; and wherein the 5-7-membered aliphatic ring or the 5-7-membered aliphatic heterocyclic ring is unsubstituted or substituted by one or more(for example 1, 2, 3 or 4) substituents selected from the group consisting of =O, halogen, $C_{1-4}$ alkyl, 6-10-membered aryl, 5-10-membered heteroaryl, and 5-10-membered fused heteroaryl; or the aforementioned 5-7-membered aliphatic ring or the 5-7-membered aliphatic heterocyclic ring is fused with a 3-6-membered aliphatic ring to form a spiro structure; A is selected from N and C; each of $R_2$ is independently selected from F, Cl, and H, and n=1 or 2; $R_4$ is selected from the group consisting of H, $C_{1-4}$ alkyl, benzyl, and halogenated benzyl; $R_5$ is F; $R_6$ is H;

preferably, in the compound of Formula IV, the ring X is a 5-6-membered aliphatic ring or a 5-6-membered aliphatic heterocyclic ring; and wherein the 5-6-membered aliphatic ring or the 5-6-membered aliphatic heterocyclic ring is unsubstituted or substituted by one or more (for example 1, 2, 3 or 4) substituents selected from =O, halogen, and $C_{1-4}$ alkyl; A is selected from N and C; each of $R_2$ is independently is selected from F, Cl, and H, and n=1; $R_4$ is selected from H, $C_{1-4}$ alkyl, benzyl and chloro benzyl; $R_5$ is F; $R_6$ is H;

preferably, in the compound of Formula IV, the ring X is a 6-membered aliphatic heterocyclic ring (for example a 6-membered nitrogen-containing aliphatic heterocyclic ring, such as piperidine ring); and wherein the 6-membered aliphatic heterocyclic ring is unsubstituted or substituted by one or more (for example 1, 2 or 3) substituents selected from =O, halogen, and $C_{1-3}$ alkyl; A is N; each of $R_2$ is independently is selected from F and H, and n=1; $R_4$ is selected from H, $CH_3$, and $CH_2CH_3$; $R_5$ is F; $R_6$ is H.

**5.** The compound of claim 1, or a pharmaceutically acceptable salt, an ester, a solvate, a hydrate, an isomer, or an isotope-labeled compound thereof; any crystal form or racemate thereof; a metabolite thereof; or a mixture thereof, wherein the compound has a structure as represented by formula V:

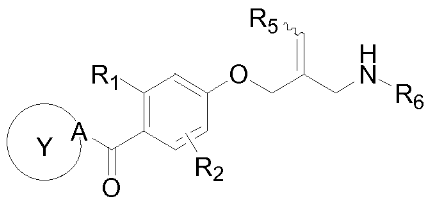

Formula V

wherein, the ring Y is a 5-8-membered aliphatic heterocyclic ring or a 5-8-membered aliphatic ring; and wherein the 5-8-membered aliphatic heterocyclic ring or the 5-8-membered aliphatic ring is unsubstituted or each substituted independently by one or more (for example 1, 2, 3 or 4) substituents selected from the group consisting of halogen, cyano, -NRR', nitro, hydroxyl, $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl, wherein the $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl are unsubstituted or each substituted independently by one or more (for example 1, 2, 3 or 4) substituents selected from the group consisting of halogen, cyano, -NRR', $C_{1-6}$ alkyl, 6-20-membered aryl, 5-20-membered heteroaryl, and 5-20-membered fused heteroaryl; or the aforementioned 5-8-membered aliphatic heterocyclic ring or the 5-8-membered aliphatic ring is fused with a 6-20-membered aromatic ring or a 5-20-membered aromatic heterocycle to form a fused ring system, wherein the fused ring system is unsubstituted or each substituted independently by one or more (for example 1, 2, 3 or 4) substituents selected from the group consisting of halogen, cyano, -NRR', $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl;

$R_1$, $R_2$, $R_5$, $R_6$, R and R' are as defined in Formula I; A is selected from N and C;

preferably, in the compound of formula V, the ring Y is a 5-7-membered aliphatic heterocyclic ring; and wherein the 5-7-membered aliphatic heterocyclic ring is unsubstituted or substituted by one or more (for example 1, 2, 3 or 4) substituents selected from the group consisting of halogen, cyano, -NRR', 6-15-membered aryl, and $C_{1-4}$ alkyl, wherein the 6-15-membered aryl and $C_{1-4}$ alkyl are unsubstituted or substituted independently by one or more (for example 1, 2, 3 or 4) substituents selected from the group consisting of halogen, $C_{1-4}$ alkyl, halogenated $C_{1-4}$ alkyl, and 6-10-membered aryl; or the aforementioned 5-7-membered aliphatic heterocyclic ring is fused with a 6-10-membered aromatic ring or a 5-10-membered aromatic heterocycle to form a fused ring system, and wherein the fused ring system is unsubstituted or each substituted independently by one or more (for example 1, 2, 3 or 4) substituents selected from the group consisting of halogen, $C_{1-4}$ alkyl, halogenated $C_{1-4}$ alkyl, and 6-10-membered aryl;

$R_1$, $R_2$, $R_5$, $R_6$, R and R' are as defined in Formula I; A is N;

preferably, in the compound of formula V, the ring Y is a 5-7-membered aliphatic heterocyclic ring; and wherein the 5-7-membered aliphatic heterocyclic ring is unsubstituted or substituted by one or more (for example 1, 2, 3 or 4) substituents selected from 6-10-membered aryl; A is N, $R_1$ is H; $R_2$ is H or F; $R_5$ is F; $R_6$ is H.

**6.** The compound of claim 1, or a pharmaceutically acceptable salt, an ester, a solvate, a hydrate, an isomer, or an isotope-labeled compound thereof; any crystal form or racemate thereof; a metabolite thereof; or a mixture thereof, wherein the compound has a structure as represented by Formula VI:

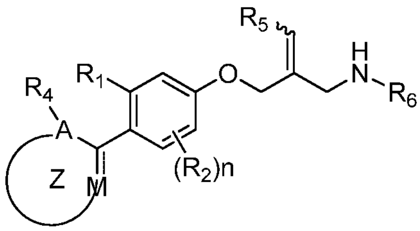

Formula VI

wherein, the ring Z is a 3-10-membered aliphatic heterocycle, a 6-20-membered aromatic ring or a 5-20-membered aromatic heterocycle; and wherein each of 3-10-membered aliphatic heterocycle, 6-20-membered aromatic ring or 5-20-membered aromatic heterocycle is unsubstituted or substituted independently by one or more (for example 1, 2, 3 or 4) substituents selected from the group consisting of halogen, cyano, -NRR', nitro, hydroxyl, =O, $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl; or the aforementioned 3-10-membered aliphatic heterocycle, 6-20-membered aromatic ring or 5-20-membered aromatic heterocycle is fused with a 6-20-membered aromatic ring or a 5-20-membered aromatic heterocycle to form a fused ring system, wherein the fused ring system is unsubstituted or each substituted independently by one or more (for example 1, 2, 3 or 4) subsituents selected from the group consisting of halogen, cyano, -NRR', $C_{1-6}$ alkyl, $C_{1-6}$ alkoxyl, halogenated $C_{1-6}$ alkyl, halogenated $C_{1-6}$ alkoxyl, 6-20-membered aryl, 5-20-membered heteroaryl, and 5-20-membered fused heteroaryl;

$R_4$ is selected from hydrogen, $C_{1-6}$ alkyl, and $C_{1-6}$ alkoxyl, or $R_4$ is absent or forms a covalent bond;

$R_1$, $R_2$, $R_5$, $R_6$, R, R', A, M and n are as defined in Formula I;

preferably, in the compound of formula VI, the ring Z is a 5-7-membered aliphatic heterocyclic ring, a 6-10-membered aromatic ring or a 5-10-membered aromatic heterocycle; and wherein each of 5-7-membered aliphatic heterocyclic ring, 6-10-membered aromatic ring or 5-10-membered aromatic heterocycle is unsubstituted or substituted independently by one or more (for example 1, 2, 3 or 4) subsituents selected from the group consisting of halogen, cyano, -NRR', nitro, hydroxyl, $C_{1-3}$ alkyl, $C_{1-3}$ alkoxyl, halogenated $C_{1-3}$ alkyl, and halogenated $C_{1-6}$ alkoxyl; or the aforementioned 5-7-membered aliphatic heterocyclic ring, 6-10-membered aromatic ring or 5-10-membered aromatic heterocycle is fused with a 6-10-membered aromatic ring or a 5-10-membered aromatic heterocycle to form a fused ring system, wherein the fused ring system is unsubstituted or substituted independently by one or more (for example 1, 2, 3 or 4) subsituents selected from the group consisting of halogen, cyano, -NRR', $C_{1-3}$ alkyl, $C_{1-3}$ alkoxyl, halogenated $C_{1-3}$ alkyl, halogenated $C_{1-3}$ alkoxyl, 6-10-membered aryl, 5-10-membered heteroaryl, and 5-10-membered fused heteroaryl;

$R_4$ is selected from hydrogen and $C_{1-6}$ alkyl, or $R_4$ is absent or forms a covalent bond;

$R_1$ is selected from hydrogen and halogen; each of $R_2$ is independently selected from hydrogen, halogen, and cyano, and n=1 or 2; $R_5$ is F or Cl; $R_6$ is selected from hydrogen and $C_{1-6}$ alkyl; A is selected from N and S; M is N; R and R' are as defined in Formula I;

preferably, in the compound of formula VI, the ring Z is a 5-7-membered aliphatic heterocyclic ring, a 6-10-membered aromatic ring or a 5-10-membered aromatic heterocycle; and wherein each of 5-7-membered aliphatic heterocyclic ring, 6-10-membered aromatic ring or 5-10-membered aromatic heterocycle is unsubstituted or substituted independently by one or more (for example 1, 2 or 3) subsituents selected from the group consisting of halogen, cyano, -NRR', nitro, hydroxyl, $C_{1-3}$ alkyl, $C_{1-3}$ alkoxyl, halogenated $C_{1-3}$ alkyl, and halogenated $C_{1-6}$ alkoxyl; wherein R and R' are independently selected from hydrogen and $C_{1-3}$ alkyl; or the aforementioned 5-7-membered aliphatic heterocyclic ring, 6-10-membered aromatic ring or 5-10-membered aromatic heterocycle is fused with a 6-10-membered aromatic ring or a 5-10-membered aromatic heterocycle to form a fused ring system, wherein the fused ring system is unsubstituted or substituted independently by one or more (for example 1, 2, 3 or 4) subsituents selected from the group consisting of halogen, cyano, -NRR', $C_{1-3}$ alkyl, $C_{1-3}$ alkoxyl, halogenated $C_{1-3}$ alkyl, and halogenated $C_{1-3}$ alkoxyl; wherein R and R' are independently selected from hydrogen and $C_{1-3}$ alkyl;

$R_4$ is selected from hydrogen and $C_{1-6}$ alkyl, or $R_4$ is absent or forms a covalent bond;

$R_1$ is selected from H and halogen; each of $R_2$ independently is H or F, and n=1 or 2;

$R_5$ is F or Cl;

$R_6$ is selected from H and $C_{1-6}$ alkyl;

A is N;

M is N.

**7.** The compound of any one of claims 1 to 6, or a pharmaceutically acceptable salt, an ester, a solvate, a hydrate, an isomer, or an isotope-labeled compound thereof; any crystal form or racemate thereof; a metabolite thereof; or a mixture thereof, wherein the compound is selected from the group consisting of:

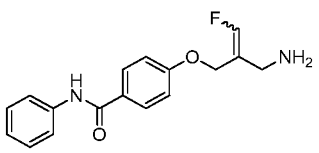

1,

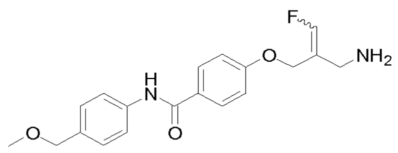

2,

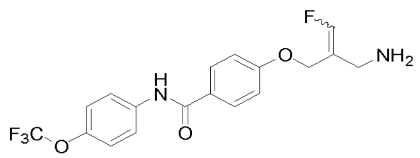

3,

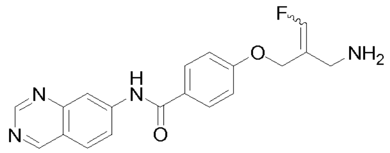

4,

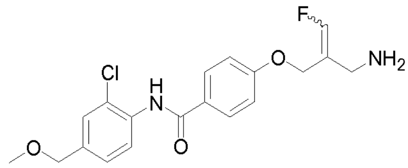

5,

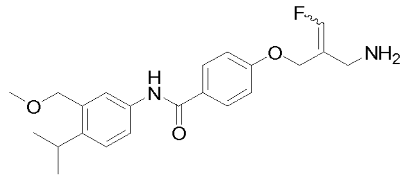

6,

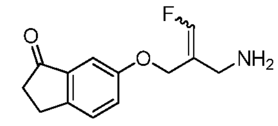

7,

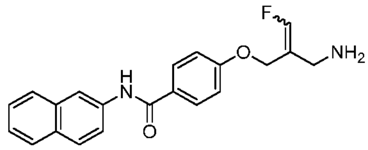

8,

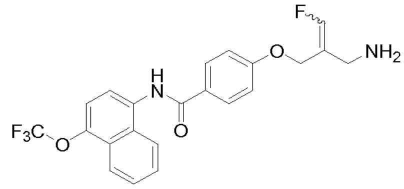

9,

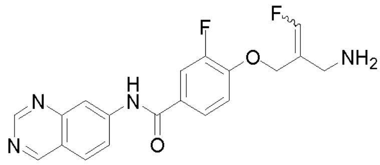

10,

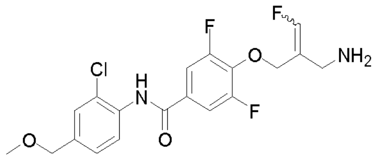

11,

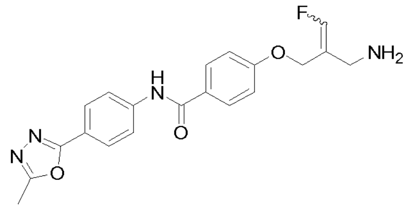

12,

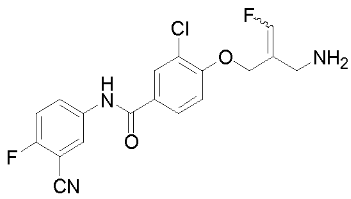

13,

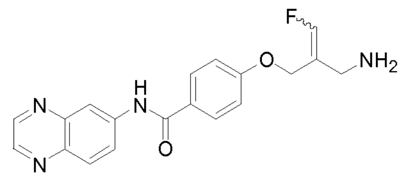

14,

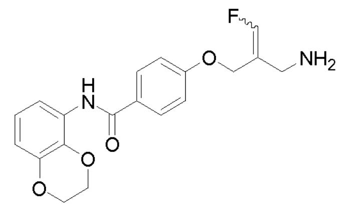

15,

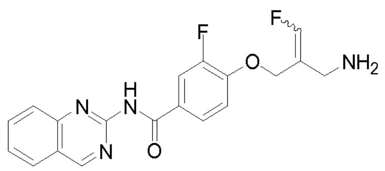

16,

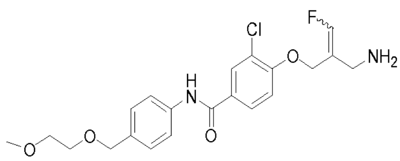

17,

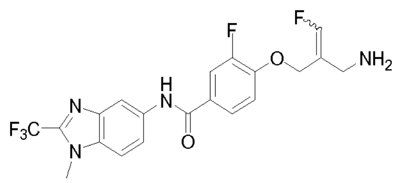

18,

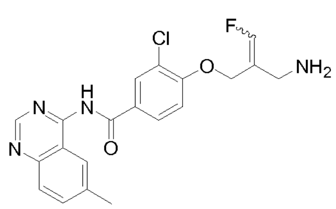

19,

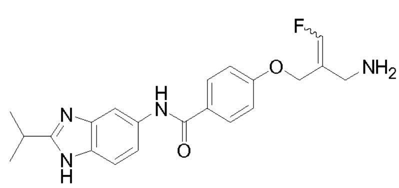

20,

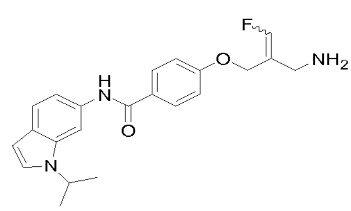

21,

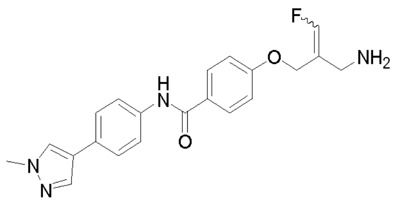

22,

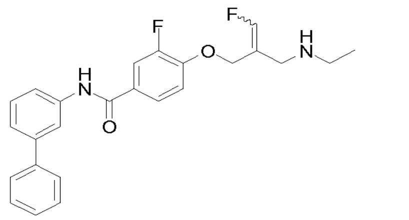

23,

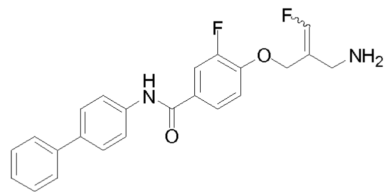

24,

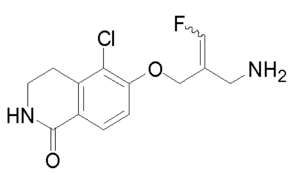

25,

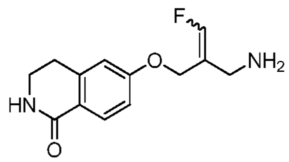

26,

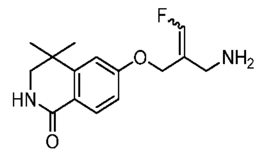

27,

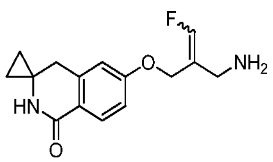

28,

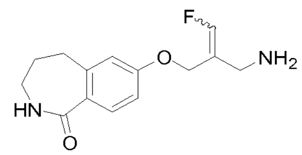

29,

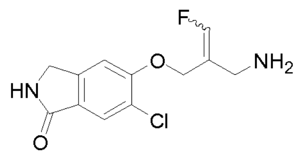

30,

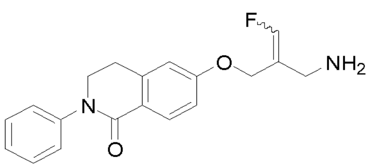

31,

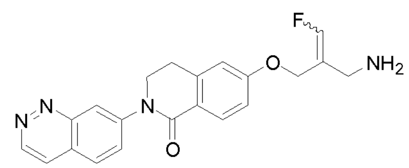

32,

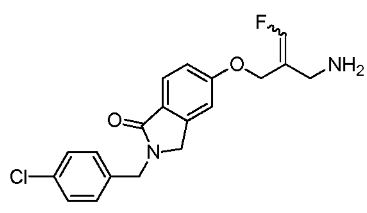

33,

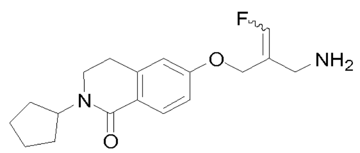

34,

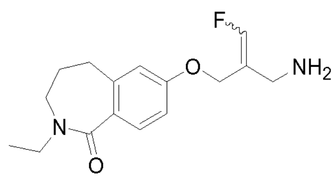

35,

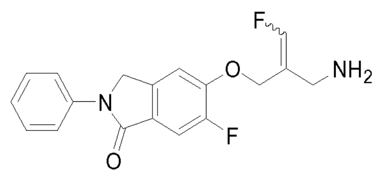

36,

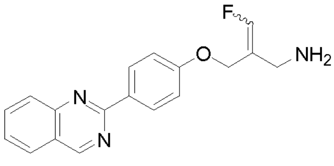

37,

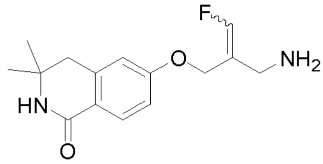

38,

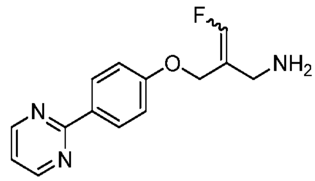

39,

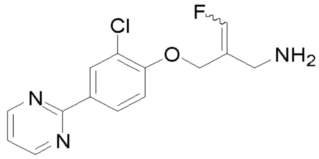

40,

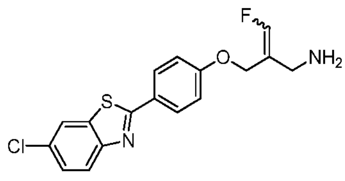

41,

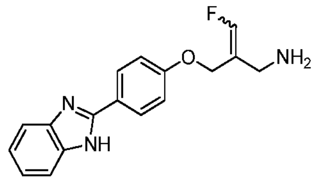

42,

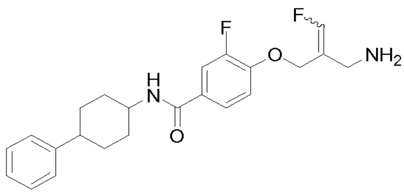

43,

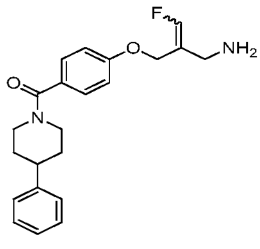

44,

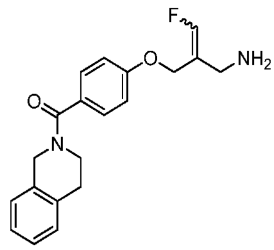

45,

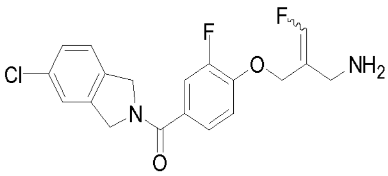

46,

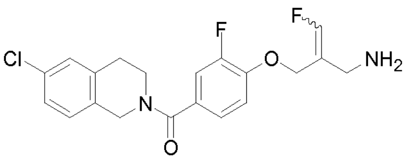

47,

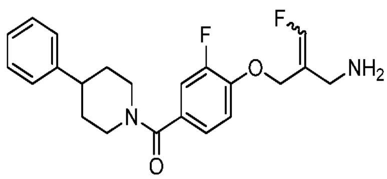

48,

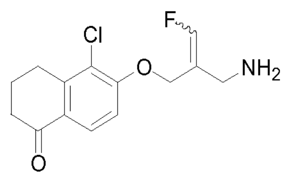

49,

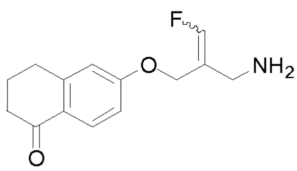

50,

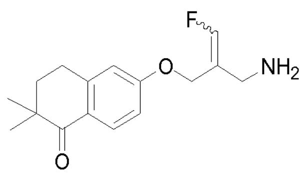

51,

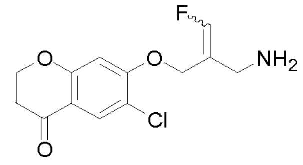

52,

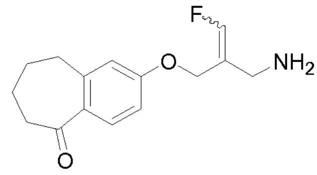

53,

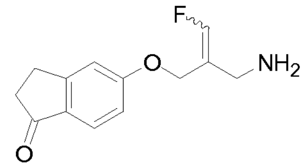

54,

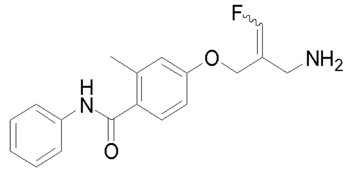

55,

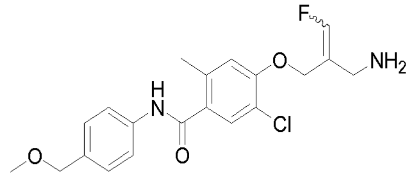

56,

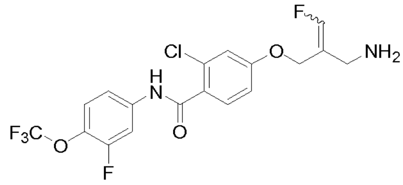

57,

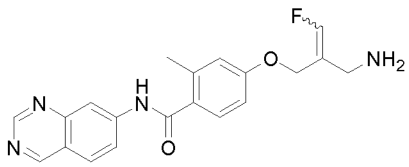

58,

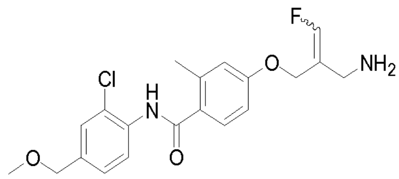

59,

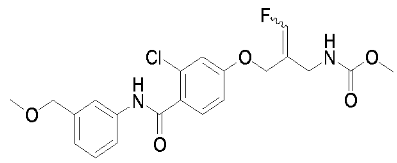

60,

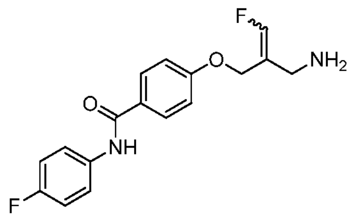

61,

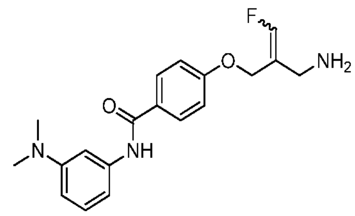

62,

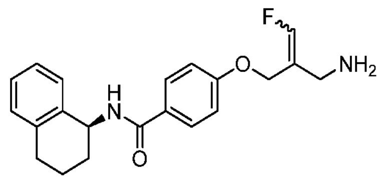

63,

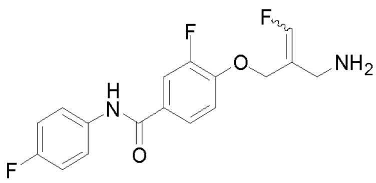

64,

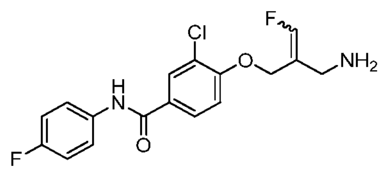

65,

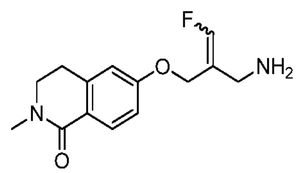

66,

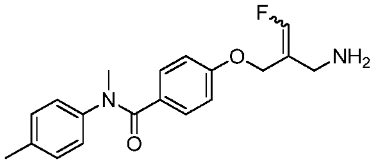

67,

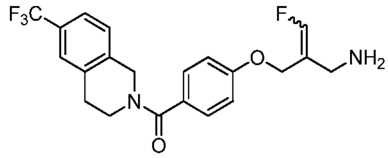

68,

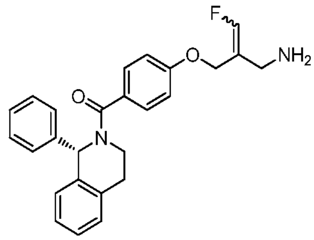

69,

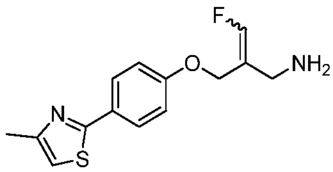

70,

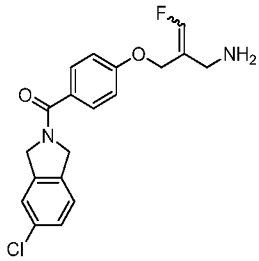

71,

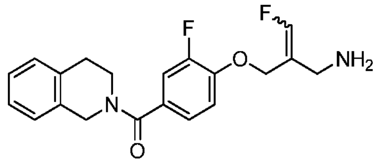

72,

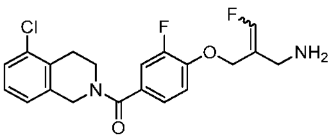

73,

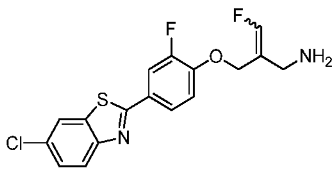

74,

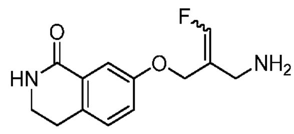

75,

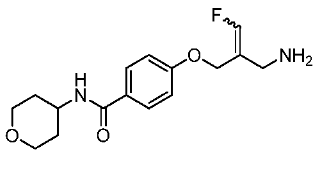

76,

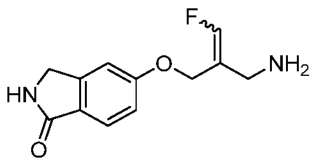

77,

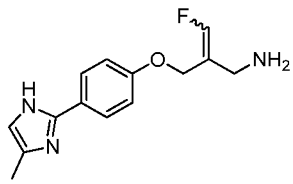

78,

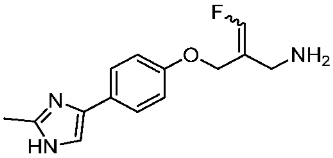

79,

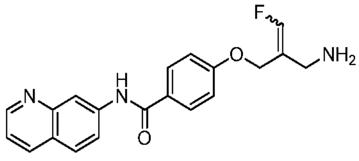

80,

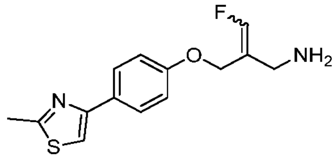

81,

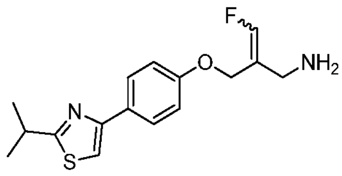

82,

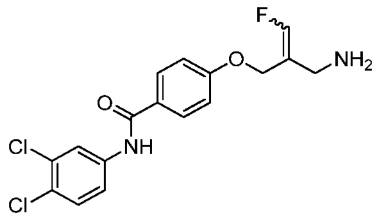

83,

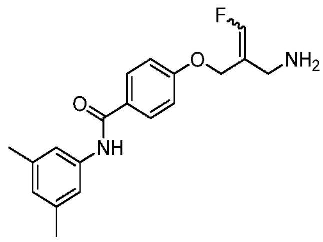

84,

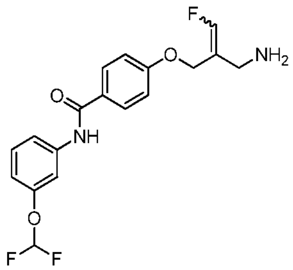

85,

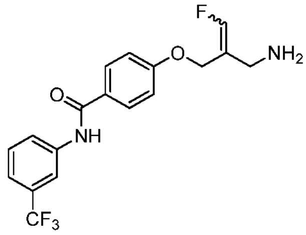

86,

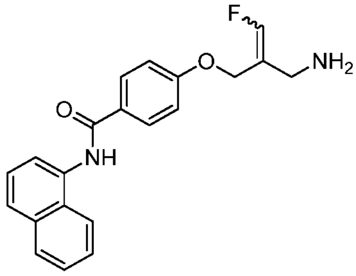

87,

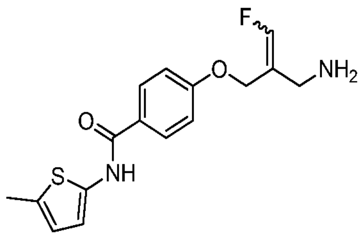

88,

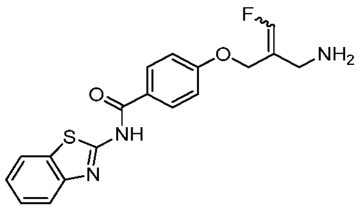

89,

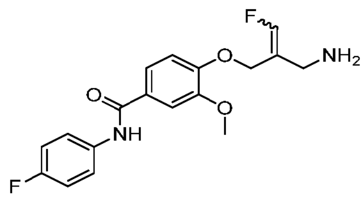

90,

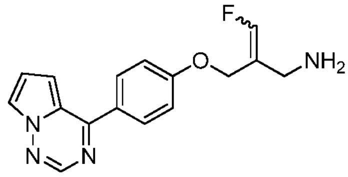

91,

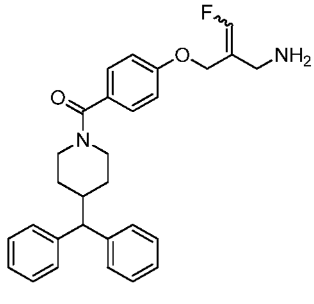

92,

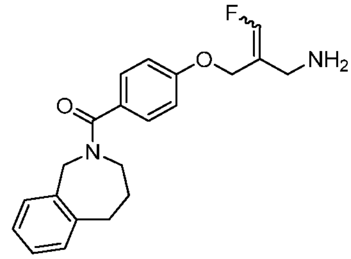

93,

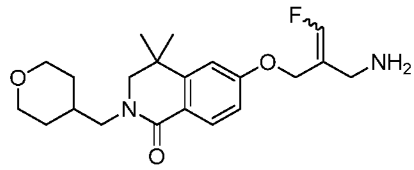
94,

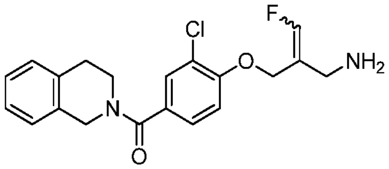
95,

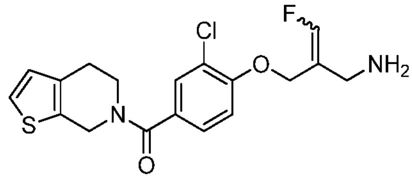
96,

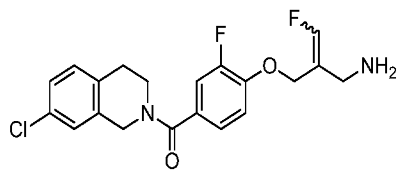
97,

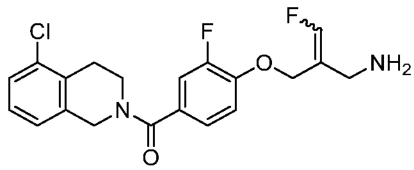
98,

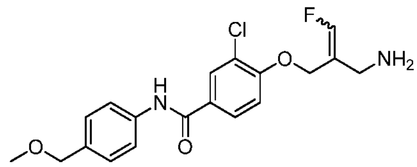
99,

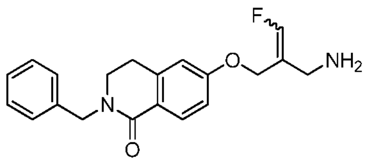
100,

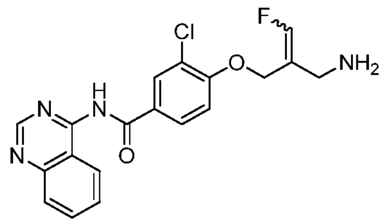
101,

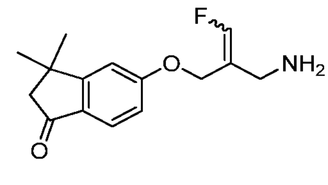
102,

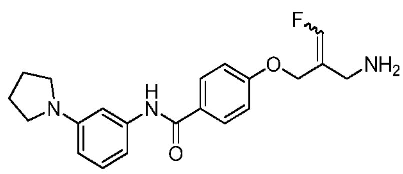
103,

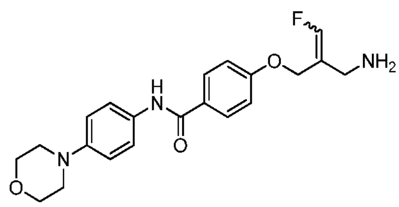
104,

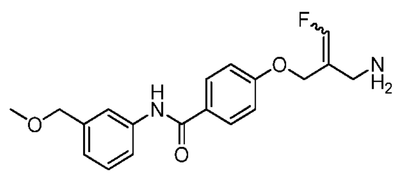
105,

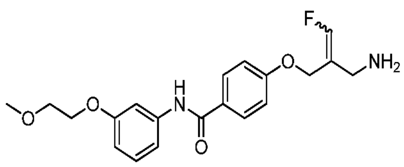
106,

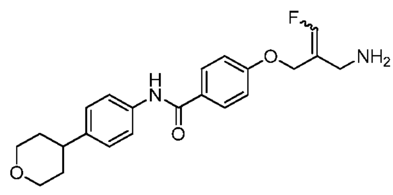
107,

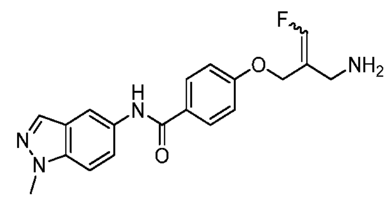
108,

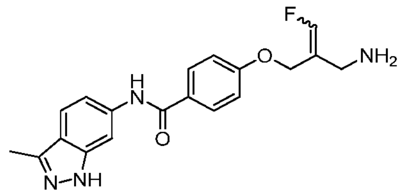

109,

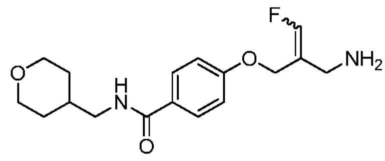

110,

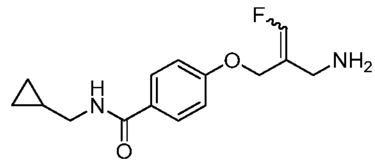

111,

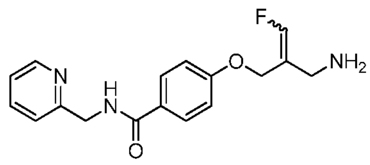

112,

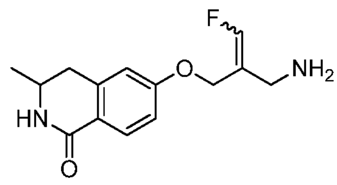

113,

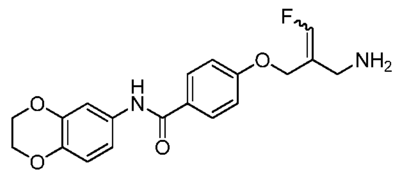

and 114.

8. The compound of any one of claims 1 to 7, or a pharmaceutically acceptable salt, an ester, a solvate, a hydrate, an isomer, or an isotope-labeled compound thereof; any crystal form or racemate thereof; a metabolite thereof; or a mixture thereof, wherein the pharmaceutically acceptable salt is a hydrochloride or a trifluoroacetate, and preferably, the pharmaceutically acceptable salt is a hydrochloride.

9. The compound of any one of claims 1 to 8, or a pharmaceutically acceptable salt, an ester, a solvate, a hydrate, an isomer, or an isotope-labeled compound thereof; any crystal form or racemate thereof; a metabolite thereof; or a mixture thereof, wherein the pharmaceutically acceptable salt thereof is:

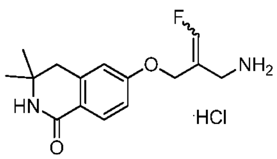

HC38

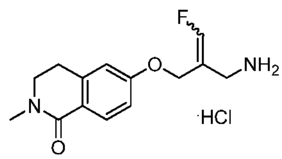

or HC66.

10. The compound of any one of claims 1 to 9, or a pharmaceutically acceptable salt, an ester, a solvate, a hydrate, an isomer, or an isotope-labeled compound thereof; any crystal form or racemate thereof; a metabolite thereof; or a mixture thereof, wherein the pharmaceutically acceptable salt thereof is:

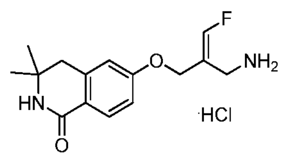

HC38-E

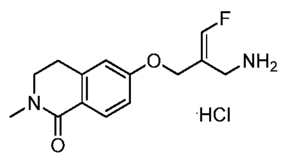

or HC66-E.

11. A pharmaceutical composition, comprising a compound of any one of claims 1 to 10, a pharmaceutically acceptable salt, an ester, a solvate, a hydrate, an isomer, or an isotope-labeled compound thereof; any crystal form or racemate thereof; a metabolite thereof; or a mixture thereof, and one or more pharmaceutical excipients.

12. A process of preparing the compound of any one of claims 1 to 7, wherein $R_6$ in Formula I is hydrogen, the process consists in a route selected from:

Route I:

Subjecting compound SM-1 and compound INT-1 to a nucleophilic substitution reaction to afford compound INT-2; removing the protection of compound INT-2 to afford a target product;

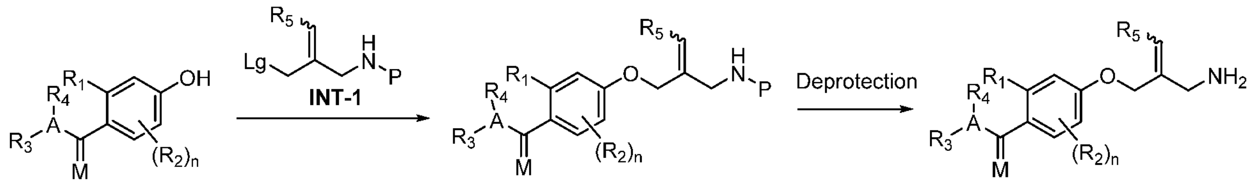


SM-1 INT-2

Route II:

Subjecting compound SM-1 and compound INT-3 to a Mitsunobu reaction to afford compound INT-2; removing the protection of compound INT-2 to afford a target product;

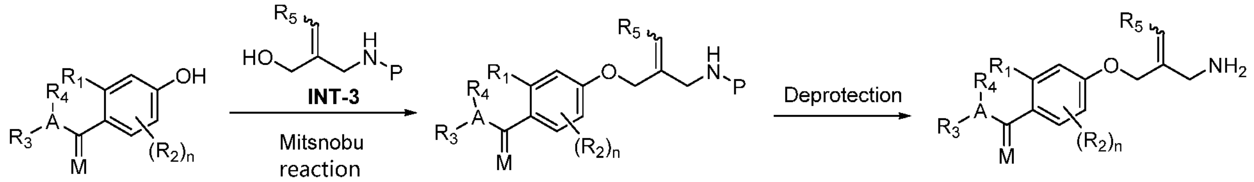


SM-1 INT-2

Route III:

Subjecting compound SM-2 and compound INT-1 to a nucleophilic substitution reaction to afford compound INT-4; subjecting compound INT-4 to a coupling reaction to afford compound INT-2; removing the protection of compound INT-2 to afford a target product;

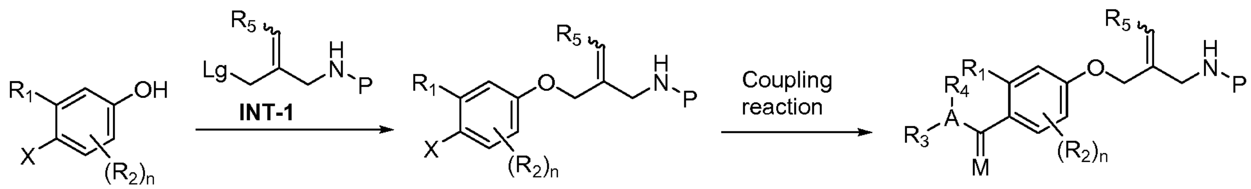


SM-2 INT-4 INT-2

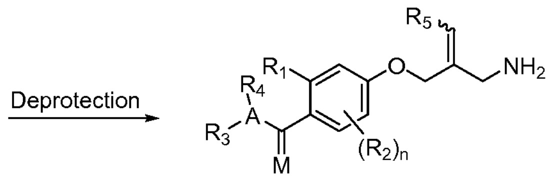


Route IV:

Subjecting compound SM-2 and compound INT-3 to a Mitsunobu reaction to afford compound INT-4; subjecting compound INT-4 to a coupling reaction to afford compound INT-2; removing the protection of compound INT-2 to afford a target compound;

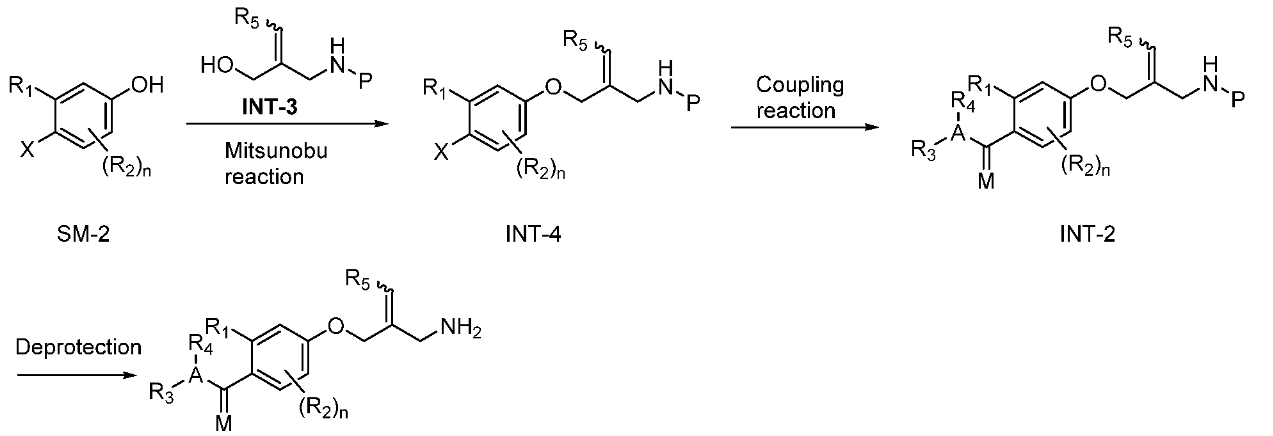


SM-2 INT-4 INT-2

Route V:
Subjecting compound SM-3 and compound INT-1 to a nucleophilic substitution reaction to afford compound INT-5; removing the protection of compound INT-5 to afford a target product;

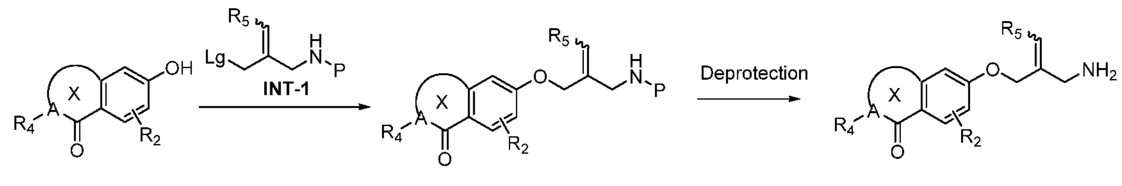


SM-3 INT-5

Scheme VI:

Subjecting compound SM-3 and compound INT-3 to a Mitsunobu reaction to afford compound INT-5; removing the protection of compound INT-2 to afford a target product;

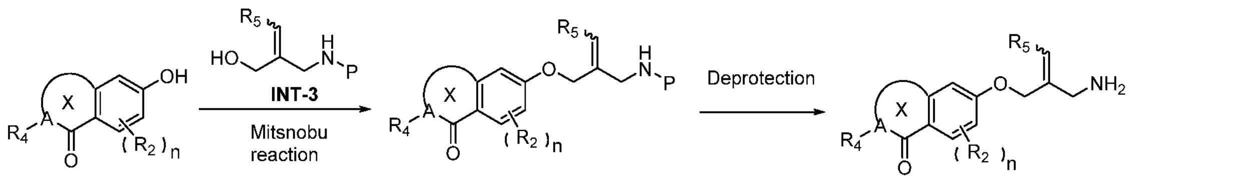


SM-3 INT-5

wherein, Lg represents a leaving group, such as halogen, -OTs, etc.; P represents an amino protective group, such as Boc, Cbz, Fmoc, benzyl, etc.; the other atoms and groups are as defined in any one of claims 1-7.

**13.** Use of the compound of any one of claims 1 to 10, or a pharmaceutically acceptable salt, an ester, a solvate, a hydrate, an isomer, or an isotope-labeled compound thereof; any crystal form or racemate thereof; a metabolite thereof; or a mixture thereof, or the pharmaceutical composition of claim 11, in the manufacture of a medicament for the treatment of a disease or disorder associated with the overactivity of VAP-1/SSAO.

**14.** The use of claim 13, wherein the disease or disorder associated with the overactivity of VAP-1/SSAO is selected from the group consisting of an inflammatory disease (for example a liver-related inflammatory disease, such as hepatitis, hepatomegaly, hepatic fibrosis, cirrhosis or ascites; a respiratory tract-related inflammatory disease, such as tracheitis, pneumonia, pulmonary fibrosis, asthma, acute lung injury, acute respiratory distress syndrome, bronchitis or chronic obstructive pulmonary disease; an eye-related inflammatory disease, for example Le uveitis; other inflammation, for example synovitis or peritonitis), organ and/or tissue transplantation rejection, an autoimmune

disease (for example rheumatoid arthritis or multiple sclerosis (for example chronic multiple sclerosis)), a skin disease (for example eczema or psoriasis), a diabetes mellitu and stroke.

**15.** A method for treating a disease or disorder associated with the overactivity of VAP-1/SSAO, comprising a step of administrating a patient in need of such treatment with an effective amount of the compound of any one of claims 1 to 10, or a pharmaceutically acceptable salt, an ester, a hydrate, a solvate, an isomer, or an isotope-labeled compound thereof; any crystal form or racemate thereof; a metabolite thereof; or a mixture thereof, or the pharmaceutical composition of claim 11.

# INTERNATIONAL SEARCH REPORT

International application No.
PCT/CN2018/083792

**A. CLASSIFICATION OF SUBJECT MATTER**

C07C 233/11 (2006.01) i; C07C 311/16 (2006.01) i; A61K 31/18 (2006.01) i; A61K 31/166 (2006.01) i; A61P 29/00 (2006.01) i

According to International Patent Classification (IPC) or to both national classification and IPC

B. FIELDS SEARCHED

Minimum documentation searched (classification system followed by classification symbols)

C07C 233/-; C07C 311/-; A61K 31/-; A61P/-

Documentation searched other than minimum documentation to the extent that such documents are included in the fields searched

Electronic data base consulted during the international search (name of data base and, where practicable, search terms used)

CNABS; DWPI, CNKI, VEN, STN (REG, CAPLUS): 科伦博泰生物医药, 氟代烯丙胺, 血管粘附蛋白, 氨基脲敏感性胺氧化酶, vascular adhesion protein-1, VAP-1, semicarbazide-sensitive amine oxidase, SSAO

C. DOCUMENTS CONSIDERED TO BE RELEVANT

| Category* | Citation of document, with indication, where appropriate, of the relevant passages | Relevant to claim No. |
|---|---|---|
| X | WO 2013163675 A1 (PHARMAXIS LTD.) 07 November 2013 (07.11.2013), claims 1-20, embodiments 1-28, compound of table 1, and description, paragraphs [0096], [0100] and [0101] | 1-14 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 01 June 2018 | 02 July 2018 |

| Name and mailing address of the ISA | Authorized officer |
|---|---|
| State Intellectual Property Office of the P. R. China<br>No. 6, Xitucheng Road, Jimenqiao<br>Haidian District, Beijing 100088, China<br>Facsimile No. (86-10) 62019451 | WANG, Ying<br>Telephone No. (86-10) 62086304 |

Form PCT/ISA /210 (second sheet) (July 2009)

# INTERNATIONAL SEARCH REPORT

International application No.
PCT/CN2018/083792

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 15
because they relate to subject matter not required to be searched by this Authority, namely:
[1] The subject matter of claim 15 relates to a method of treatment of the human or animal body by therapy, and therefore the subject matter of claim 15 does not warrant an international search by the International Searching Authority (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA /210 (continuation of first sheet (2)) (July 2009)

**INTERNATIONAL SEARCH REPORT**
Information on patent family members

International application No.
PCT/CN2018/083792

| Patent Documents referred in the Report | Publication Date | Patent Family | Publication Date |
|---|---|---|---|
| WO 2013163675 A1 | 07 November 2013 | LT 2844637 T | 10 May 2018 |
| | | EP 2844637 A4 | 09 December 2015 |
| | | BR 112014027406 A2 | 27 June 2017 |
| | | JP 2017132767 A | 03 August 2017 |
| | | JP 2015521167 A | 27 July 2015 |
| | | US 9302986 B2 | 05 April 2016 |
| | | CA 2871793 A1 | 07 November 2013 |
| | | AU 2013203655 A1 | 21 November 2013 |
| | | PT 2844637 T | 17 April 2018 |
| | | EP 2844637 B1 | 28 March 2018 |
| | | KR 20150014926 A | 09 February 2015 |
| | | SG 11201406948P A | 27 November 2014 |
| | | HK 1203477 A1 | 30 October 2015 |
| | | ES 2668300 T3 | 17 May 2018 |
| | | US 2018086698 A1 | 29 March 2018 |
| | | US 2015158813 A1 | 11 June 2015 |
| | | MX 2014013294 A | 08 May 2015 |
| | | CN 104520268 A | 15 April 2015 |
| | | AU 2013255103 A1 | 06 November 2014 |
| | | AU 2013203655 B2 | 02 February 2017 |
| | | CN 104520268 B | 24 May 2017 |
| | | CN 107266332 A | 20 October 2017 |
| | | DK 2844637 T3 | 23 April 2018 |
| | | US 9815782 B2 | 14 November 2017 |
| | | US 2016244406 A1 | 25 August 2016 |
| | | EP 2844637 A1 | 11 March 2015 |
| | | JP 6182595 B2 | 16 August 2017 |
| | | AU 2013255103 B2 | 29 September 2016 |

Form PCT/ISA /210 (patent family annex) (July 2009)

**INTERNATIONAL SEARCH REPORT**
Information on patent family members

International application No.
PCT/CN2018/083792

| Patent Documents referred in the Report | Publication Date | Patent Family | Publication Date |
|---|---|---|---|
| | | SG 10201707020T A | 30 October 2017 |

Form PCT/ISA /210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **FONTANA, E.** *Biochem. J.,* 2001, vol. 356, 769
- **MOLDES, M.** *J. Biol. Chem.,* 1999, vol. 274, 9515
- **DUNKEL, P.** *Curr. Med. Chem.,* 2008, vol. 15, 1827
- *Tetra. Letters,* 1977, vol. 36, 3155
- **O'CONNELL, K. M.** *Biochemistry,* 2004, vol. 43, 10965